# EUROPEAN PATENT SPECIFICATION

(11) **EP 1 824 965 B1**
(45) Date of publication and mention of the grant of the patent: **05.10.2011**
(21) Application number: 04822334.1
(22) Date of filing: 27.10.2004
(51) Int. Cl.: C12N 5/071

(54) **Hepatic differentiation of stem cells**
Hepatische Differenzierung von Stammzellen
Différenciation hépatique de cellules souches

(43) Date of publication of application: 29.08.2007
(73) Proprietor: VRIJE UNIVERSITEIT BRUSSEL, 1050 Brussel (BE)
(72) Inventor: ROGIERS, Vera, B-2820 Bonheiden (BE); VANHAECKE, Tamara, B-1502 Lembeek (BE); SNYKERS, Sarah, B-9340 Lede (BE); PAPELEU, Peggy, B-1541 St.-Pieters-Kapelle (BE); VINKEN, Matthieu, B-8400 Oostende (BE); HENKENS, Tom, B-9052 Zwijnaarde (BE); ELAUT, Greetje, B-1050 Brussel (BE)
(74) Representative: Michalík, Andrej
(86) International application number: PCT/EP2004/012134
(87) International publication number: WO 2006/045331

(56) References cited:
- WO-A-00/23567
- WO-A-01/81549
- WO-A-02/08388
- WO-A-2004/046312
- WO-A2-02/064748
- SCHWARTZ R E ET AL: "Multipotent adult progenitor cells from bone marrow differentiate into functional hepatocyte-like cells" JOURNAL OF CLINICAL INVESTIGATION, vol. 109, no. 10, May 2002 (2002-05), pages 1291-1302, XP002313082 ISSN: 0021-9738 cited in the application
- ELAUT G ET AL: "Major phase I biotransformation pathways of Trichostatin A in rat hepatocytes and in rat and human liver microsomes." DRUG METABOLISM AND DISPOSITION, vol. 30, no. 12, December 2002 (2002-12), pages 1320-1328, XP002343455 ISSN: 0090-9556 cited in the application
- ELAUT G ET AL: "Rat hepatocyte suspensions as a suitable in vitro model for studying the biotransformation of histone deacetylase inhibitors" ATLA ALTERNATIVES TO LABORATORY ANIMALS, vol. 32, no. Suppl. 1A, June 2004 (2004-06), pages 105-112, XP008051496 ISSN: 0261-1929 cited in the application
- VANHAECKE T ET AL: "Effect of the histone deacetylase inhibitor trichostatin A on spontaneous apoptosis in various types of adult rat hepatocyte cultures" BIOCHEMICAL PHARMACOLOGY, vol. 68, no. 4, 15 August 2004 (2004-08-15), pages 753-760, XP002343456 ISSN: 0006-2952
- NIKI T ET AL: "A histone deacetylase inhibitor, trichostatin A, suppresses myofibroblastic differentiation of rat hepatic stellate cells in primary culture" HEPATOLOGY, vol. 29, no. 3, March 1999 (1999-03), pages 858-867, XP002967260 ISSN: 0270-9139

## Description

### Field of the Invention

In one aspect, the present invention relates generally to methods for inducing differentiation of stem cells, to differentiated cells obtained by the methods, and to uses for those cells.

In another aspect the present invention relates generally to methods for stabilizing the phenotype of isolated primary cells *in vitro,* and to uses for those cells.

### Background to the Invention

In one aspect, the present invention provides a method for differentiating stem cells. Specific cell types obtained by *in vitro* differentiation of stem cells may find use in pharmacotoxicological research and regulatory testing, in clinical applications, including tissue engineering, transplantation, and gene therapy, in studies of cell and organ development, and in other areas.

For example, although several disorders, such as cardiovascular disease, liver failure, or kidney failure, may be successfully treated by transplantation of organs from human donors, this approach is severely limited by the existing shortage of donor organs. In contrast, stem cells may be grown in culture to sufficient quantities and subsequently induced to differentiate into select cell types. Differentiated cells so obtained may be transplanted as such, or as part of an *in vitro* engineered tissue or organ, to the body of a patient in need of such treatment.

Another problem associated with transplantation of organs from human donors is the potential incompatibility of the transplanted tissue with the immune system of the recipient. Because the donated organ or tissue is recognized by the host immune system as foreign, antirejection medications must be provided to the patient at a significant cost, both financially and physically. On the contrary, stem cells may be genetically modified to comprise either altered or no major histocompatibility complex component, thus minimizing the risk of rejection. Alternatively, adult stem cells may be obtained from the patient himself, propagated *ex vivo,* induced to differentiate into a desired cell type and re-introduced into th e patient's body. Another potential area for the use of stem cells is gene therapy. For example, adult stem cells may be obtained from the patient, their genetic material may be modified and cells with the desired modification may be propagated *ex vivo,* induced to differentiate into a given cell type and re-introduced into the patient's body.

Yet another area for the use of differentiated cells is *in vitro* testing of toxicity or carcinogenicity of chemical compounds or candidate pharmaceuticals. While animal model testing is a mainstay in this area, it cannot always predict the effects that chemical compounds or candidate pharmaceuticals may have on human cells. Moreover, there exists a trend to minimize the number of animals used in such tests. In one particular example, stem cells induced to differentiate into hepatocytes may be used to evaluate toxicity, pharmacokinetics and detoxification of chemical compounds and candidate therapeutics. Very useful examples of chemical compounds would be those comprised or potentially to be comprised in, for example, compositions for plant protection, cosmetics, food or food additives, or pharmaceuticals.

Given these and other promising applications for stem cells differentiated into specific cell types *in vitro,* there is a strong need for efficient methods to differentiate stem cells into such cell types. These methods typically use growth factors or cytokines, which are administered either single or as mixtures of two or more growth factors or cytokines. For example, Jiang et al. (Nature 418: 41-49, 2002) differentiated multipotent adult progenitor cells (MAPC) into endothelial cells by addition of vascular endothelial growth factor (VEGF); into neuroectodermal cells (astrocytes, glial cells and neurons) by addition of basic fibroblast growth factor (b-FGF); and into hepatocyte-like cells by addition of fibroblast growth factor type 4 (FGF-4) and hepatic growth factor (HGF).

Other protocols are known e.g. from WO 01/81549 (ES) or WO 02/64748 (MASC).

However, such treatments often result in differentiation of only a fraction of cells. For example, only 60% of MAPC cells induced to differentiate into hepatocytes by addition of FGF-4 and HGF in the above example acquired epithelioid morphology. Moreover, such treatments may also produce mixed population of cells having various phenotypes. For instance, when MAPC cells were differentiated into neuro-ectodermal cells in the above example, 15±4% of the cells acquired morphological and phenotypical features of astrocytes, 12±3% of oligodendrocytes, and 68±9% of neurons.

Therefore, there exists a need for alternative, more efficient methods to differentiate stem cells *in vitro,* especially to obtain more homogeneous populations of cells and/or more advanced differentiated phenotype in such cells. The present invention aims to provide such alternative, novel method to differentiate stem cells.

In another aspect, the present disclosure relates to methods for stabilizing the phenotype of isolated primary cells *in vitro.* Primary cells, i.e., differentiated cells isolated directly from a particular tissue or organ of an organism and subsequently cultured *in vitro,* provide a powerful tool for the study of biological processes, as it is believed that biology of primary cells closely resembles the situation *in vivo.* A useful, but non-limiting, example of the possible applications of primary cells is that of using primary hepatocytes to assess the potential hepatotoxicity and biotransformation of, for example, chemical compounds or candidate therapeutics. However, while such primary hepatocyte cultures provide an *in vitro* system capable of integrated biotransformation, the cells gradually dedifferentiate and loose their liver-specific properties in culture, which may limit their usefulness. Therefore, to facilitate long-term assays in such primary hepatocyte cultures, methods for culturing these cells that would stabilize their differentiated phenotype are required. By analogy, this requirement also applies to other types of primary cells.

The inventors previously reported that the histone deacetylase inhibitor trichostatin A delays the occurrence of spontaneous apoptosis and the loss of one liver-specific function (albumin secretion) when added to cultured primary hepatocytes (Vanhaecke et al. in HUG meeting 2003, 28-29 March 2003, Aberdeen, UK). The present invention extends these findings in order to establish a method for stabilizing the phenotypic properties of isolated primary cells cultured *in vitro.*

### Summary of the Invention

In one aspect, the present invention provides a novel, efficient method to differentiate stem cells *in vitro*. According to the present invention, it was surprisingly discovered that when differentiation agents are administered to stem cells in a specific order, this results in more advanced differentiation than if such differentiation agents were administered to stem cells together as a mixture. Further, it was also surprisingly discovered that exposing of so treated stem cells to a histone deacetylase inhibitor further promotes the differentiation. Accordingly, the present invention provides a method for producing differentiated cells from stem cells *in vitro*, wherein at least one stem cell is sequentially exposed to differentiation agents and to at least one histone deacetylase inhibitor as described in the claims. Additional features and advantages of this aspect of the invention will be set forth in the Detailed description below.

In another aspect, the present disclosure provides a novel method for stabilization of phenotypic properties of isolated primary cells cultured *in vitro.* In the present invention, the inventors surprisingly discovered that when added to cultured primary hepatocytes, trichostatin A or another histone deacetylase inhibitor stabilizes numerous phenotypic attributes of primary hepatocytes, such as albumin secretion, expression of enzymes required for phase I and phase II biotransformation, expression of liver-enriched transcription factors (LETF) or expression of proteins responsible for maintaining cell-cell junctions. Even more surprisingly, the inventors further discovered that administration of trichostatin A or another histone deacetylase inhibitor already during isolation of the primary hepatocytes by perfusing the liver, stabilizes even further the phenotype of these cells. This effect can be further improved by addition of other components into the composition used for the perfusion. By analogy, these findings can be extended to other primary cells. Accordingly, the present invention provides a method for stabilization of phenotypic properties of isolated primary cells cultured *in vitro,* wherein the primary cells are isolated and cultured in the presence of at least one histone deacetylase inhibitor. Additional features and advantages of this aspect of the invention will be set forth in the Detailed description which follows.

### Brief description of the Figures

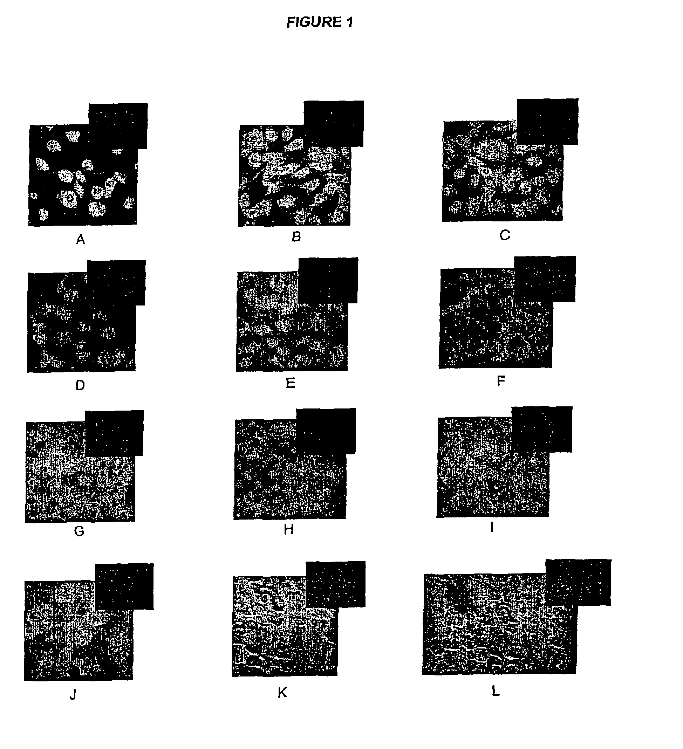

**Figure 1**. Characterization of rat BMSC differentiation into hepatocyte-like cells at the protein level. The rat BMSC were cultivated sequentially with LSP cytokines on 1 mg/ml collagen type I at 100% confluence. Immunofluorescense for (A-C) AFP-FITC respectively at days 3, 4 and 9 compared with goat IgG as negative controls; (D,E) HNF-1-cy-3 respectively at days 4 and 9 compared with rabbit IgG as negative controls; (F,G) CK18-FITC respectively at days 9 and 17 compared with mouse IgG as negative controls; (H,I) HNF-3β-cy-3 respectively at days 4 and 10 compared with goat IgG as negative controls; (J-L) ALB-FITC respectively at days 6 and 17 compared with goat IgG as negative controls. 1-5, 8-12: 20 x 10 magnification; 6,7: 10 x 10 magnification. Representative example for more than 5 experiments.

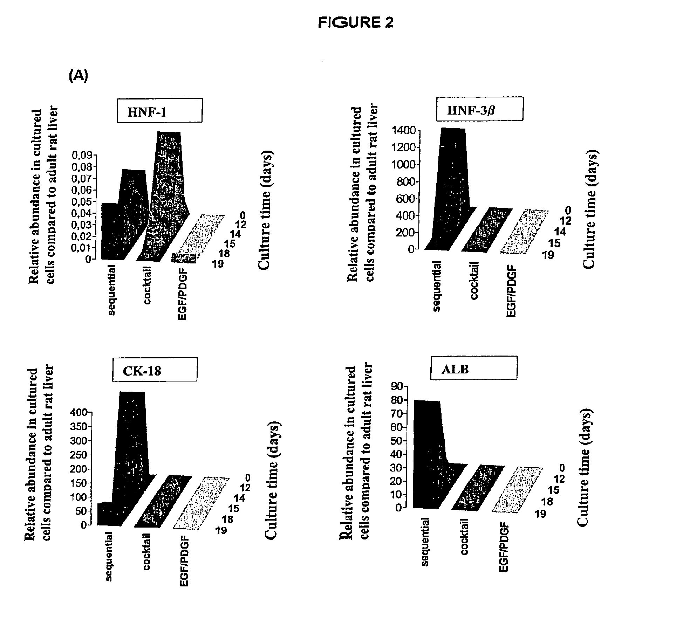

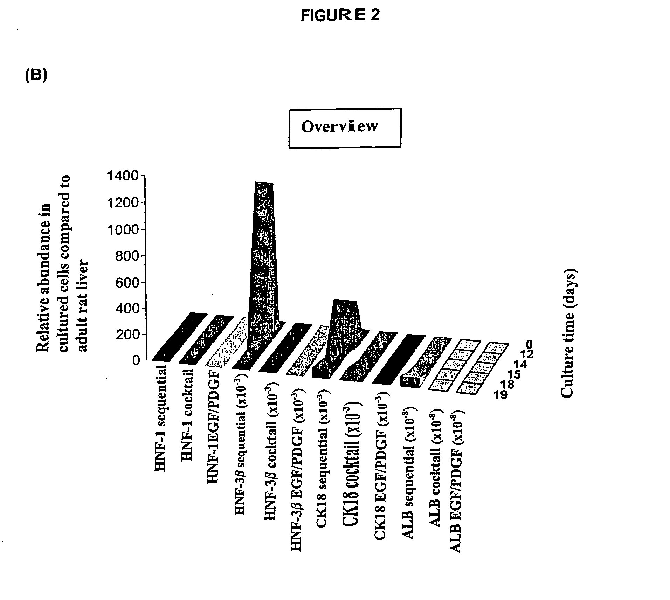

**Figure 2** **A,B.** Characterization of rat BMSC differentiation into hepatocyte-like cells at the mRNA level (relative abundance in cultivated cells compared with rat adult liver). The R8 clone of rat BMSC was cultivated on 1mg/ml collagen at 100% confluence in the presence of hepatocyte-specific cytokines, added either as a cocktail (FGF+HGF+ITS+Dex) or sequentially (FGF/HGFIHGF,ITS,Dex). mRNA levels were normalized using 18S and compared with mRNA levels in rat adult liver (positive control) and undifferentiated BMSC (negative control) cultivated in expansion medium (EGF, PDGF). Values rep resent means of three independent experiments. (A) Expression of individual markers. (B) Overview of marker expression.

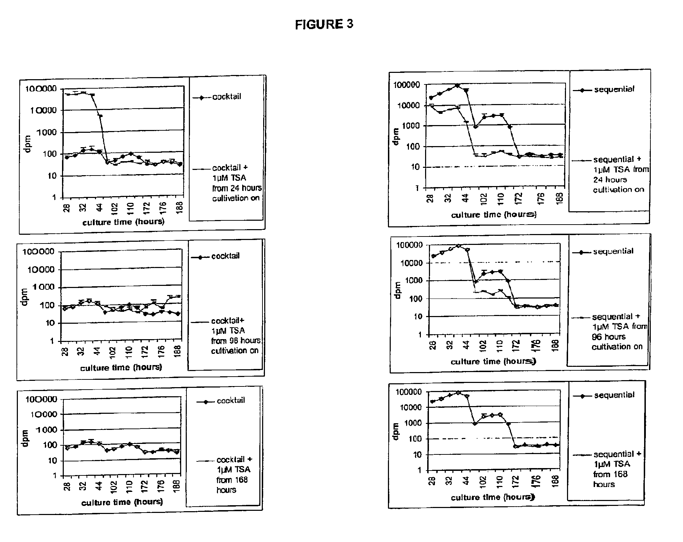

**Figure 3****.** Measurement of DNA-synthesis by [methyl-³H] thymidine incorporation. Rat BMSC are cultivated on 10ng/ml FN at 2.5 10³ cells/cm² in the presence of hepatocyte-specific cytokines, added either as a cocktail (FGF+HGF+ITS+Dex) or sequentially (FGF/HGF/HGF,ITS,Dex). 1µM TSA was added 24, 96 and 168h after splitting, respectively. The graphs represent three independent experiments.

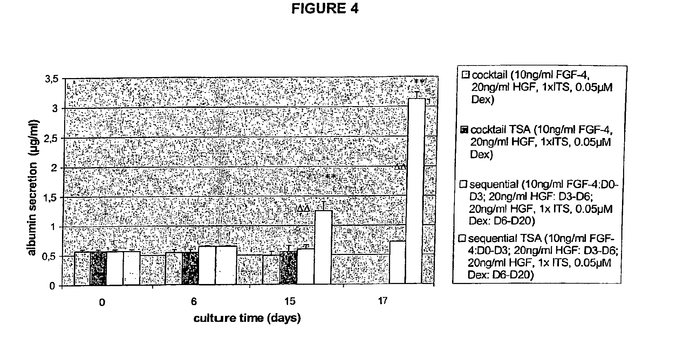

**Figure 4****.** ALB secretion into the medium was measured by ELISA. Rat BMSC were cultivated on collagen type I at 100% confluence in the presence of hepatocyte-specific cytokines, added either as a cocktail (FGF+HGF+ITS+Dex) or sequentially (FGF/HGF/HGF,ITS,Dex). 1µM TSA was added from day 6 on. The graph is representative for 3 experiments.

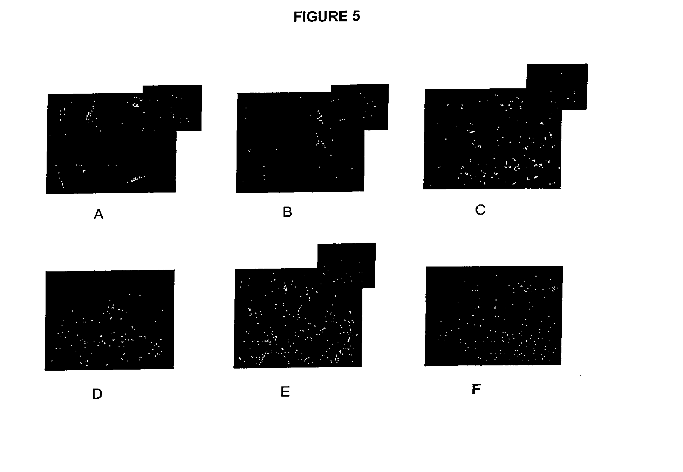

**Figure 5****.** Characterization of rat BMSC differentiation into neuroectodermal-like cells at the protein level. The rat BMSC were cultivated in the presence of neuronal-specifec cytokines (bFGF and FGF-8), added either as a cocktail (co) or separately (sep) on 10ng/ml FN at 30% confluence. Immunofluorescense for (A,C) NF- 200-cy-3 at days 9 and 17, respectively compared with mouse IgG as negative controls (sep); (B,E) NF-200-cy-3 at days 9 and 17, respectively compared with mouse IgG as negative controls (co); (D,F) DAPI-staining to visualize nuclei of respectively neuroectodermal-like-derived- rat BMSC at day 17, cultivated sequentially and in the presence of a cocktail of cytokines. 10 x 10 magnification. Representative example for more than 5 experiments.

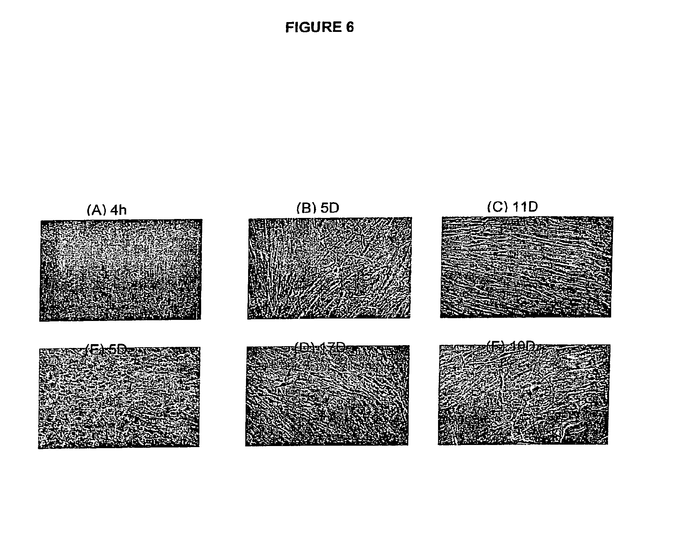

**Figure 6****.** Adipogenic differentiation was shown by Sudan III (A,B,C,D) and ORO (E,F) staining from day 5 on. hMSC were cultivated for 4h, 5, 10, 11 and 17 days at 21.5 10³ cells/cm² on 1mg/ml collagen type I in the presence of 10ng/ml FGF-4, 20ng/ml HGF, 1x ITS and 0.05µM Dex, respectively.

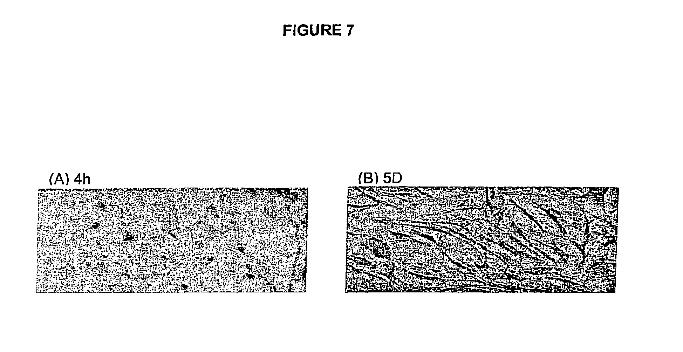

**Figure 7****.** Osteogenic differentiation was shown by Von Kossa (A, B) staining from day 5 on. hMSC were cultivated for 4h and 5 days at 21.5 10³ cells/cm² on 1mg/ml collagen type I in the presence of 10ng/ml FGF-4, 20ng/ml HGF, 1x ITS and 0.05µM Dex, respectively.

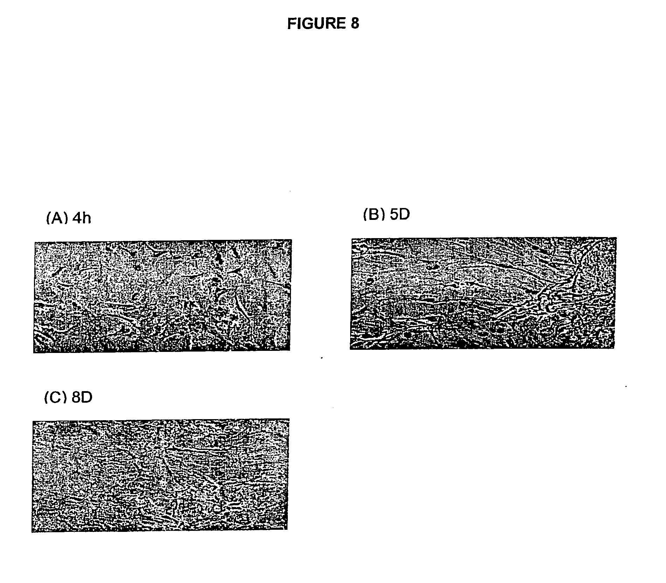

**Figure 8****.** Neurogenic differentation was shown by Cresyl Fast (A, B) and Bodian (C) staining from day 5 on. hMSC were culivated for 4h, 5 and 8 days at 21.5 10³ cells/cm² on 1mg/ml collagen type I in the presence of 10ng/ml FGF-4, 20ng/ml HGF, 1x ITS and 0.05µM Dex, respectively.

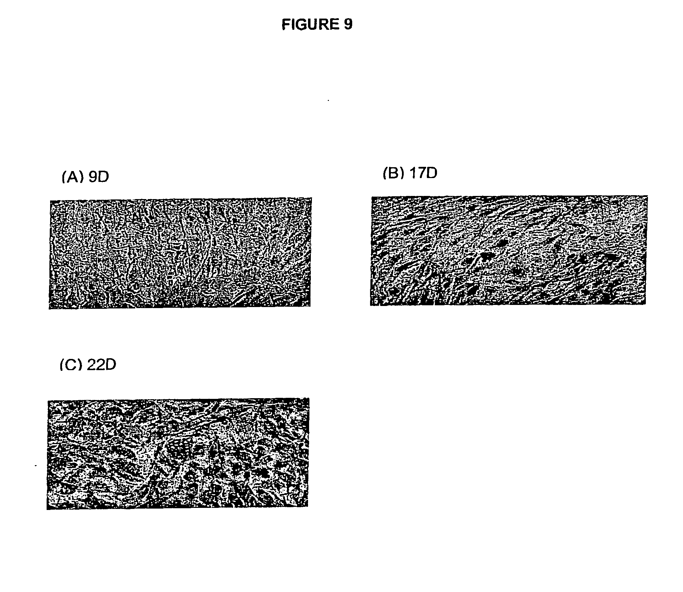

**Figure 9****.** Glycogen storage was shown by PAS staining from day 9 on. hMSC were cultivated for 9, 17 and 22 days (A, B, C) at 21.5 10³ cells/cm² on 1mg/ml collagen type I and sequentially treated with 10ng/ml FGF-4/ 20ng/ml HGF/ 20ng/ml HGF, 1x ITS and 0.05µM Dex, respectively.

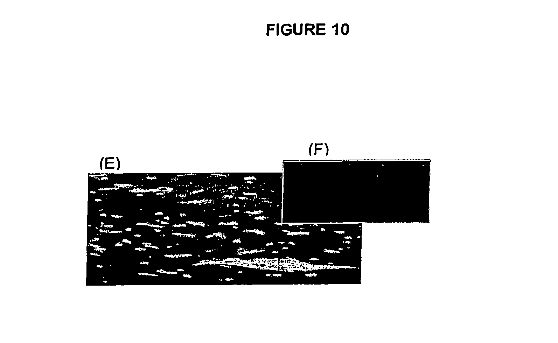

**Figure 10****.** Immunofluorescence for CK18-FITC at day 7 (E) compared with mouse IgG (F) as a negative control. hMSC were cultivated for 7 days at 21.5 10³ cells/cm² on 1mg/ml collagen type I and sequentially treated with 10ng/ml FGF-4/ 20ng/ml HGF/ 20ng/ml HGF, 1x ITS and 0.05µM Dex, respectively.

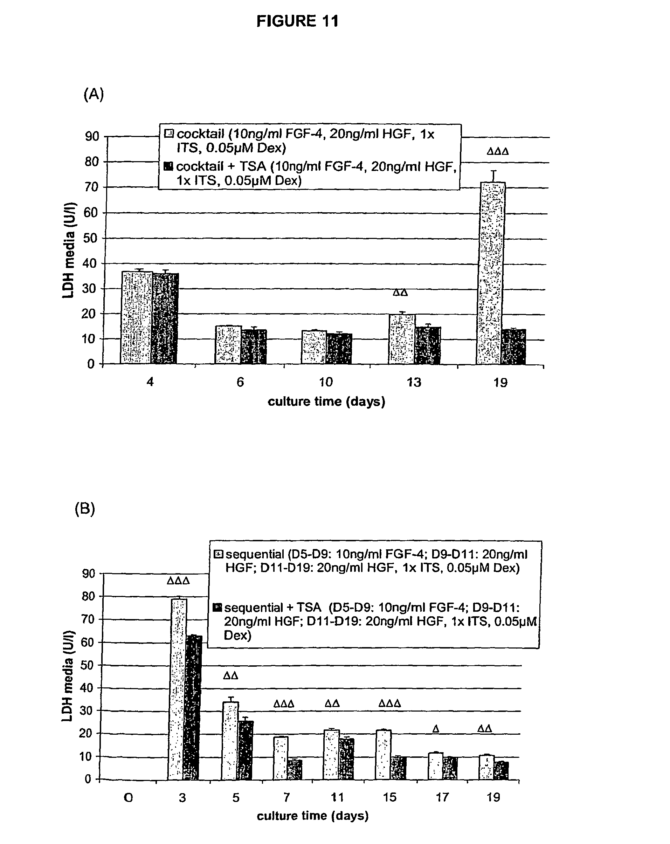

**Figure 11****.** Measurement of LDH-leakage into the media as an indicator of cytotoxicity of 1µM TSA. hMSC were cultivated on collagen type I at 21.5 10³ cells/cm² in the presence of LSP cytokines, added either as a cocktail (figure 11A) or separately (figure 11B). 1µM TSA was added from day 0 until addition of HGF. The graph is representative for more than 5 separate experiments.
   Δ, ΔΔ, ΔΔΔ: The LDH-activity of control cultures is significant higher than those of TSA cultures with p<0.05, p<0.01 and p<0.001, respectively (Oneway Anova + t-test).

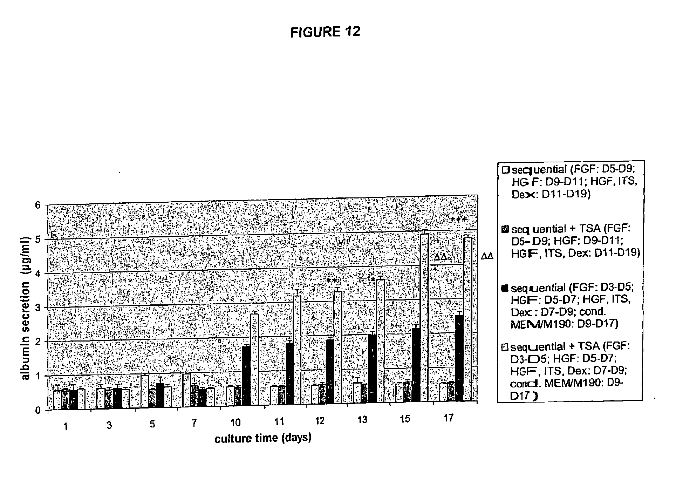

**Figure 12****.** ALB secretion into the media, measured by ELISA. hMSC were cultivated on collagen type I at 21.5 10³cells/cm² in the presence of LSP cytokines, added sequentially. 1µM TSA was added from day 0 until addition of HGF. Conditioned MEM/M190 is medium first used for cultivation of monolayer cultures of primary hepatocytes. The graph is representative for 3 separate experiments
   *: ALB-secretion of hMSC cultivated in conditioned sequential +- TSA was at days 12-13 significant higher than at days 1-7 with p<0.05 (Oneway Anova).
   **: ALB-secretion of hMSC cultivated in conditioned sequential + TSA was at day 11 significant higher than at days 1-7 with p<0.01 (Oneway Anova ).
   ***: ALB-secretion of hMSC cultivated in conditioned sequential + TSA was at days 15-17 significant higher than at days 1-7 with p<0.001 (Oneway Anova).
   ΔΔ: ALB-secretion of hMSC cultivated in conditioned sequential + TSA is significant higher than those of control cultures (conditioned sequential - TSA) with p<0.01 (t-test).

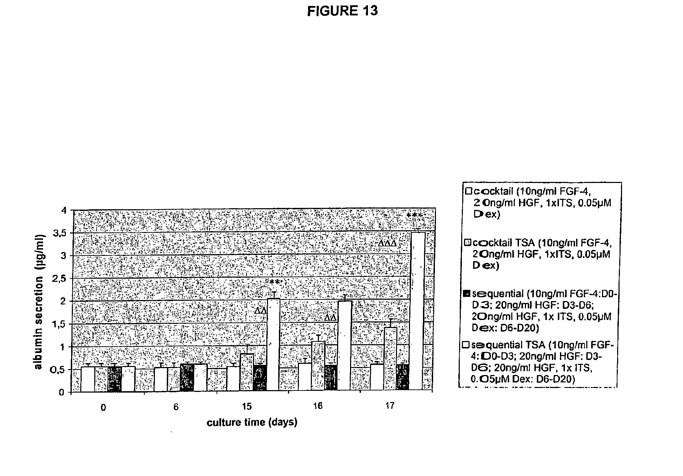

**Figure 13****.** ALB secretion into the media, measured by ELISA. hMSC were cultivated on collagen type I at 21.5 10³cells/cm² in the presence of LSP cytokines, added either as a cocktail or sequentially. 1µM TSA was added from day 6 on. The graph is representative for more than 4 separate experiments.
   **: ALB-secretion of hMSC cultivated in sequential + TSA is at day 15 significant higher than at day 6 with p<0.01 (Oneway Anova).
   ***: ALB-secretion of hMSC cultivated in sequential + TSA is at day 17 significant higher than at days 6-16 with p<0.001 (Oneway Anova).
   ΔΔ: ALB-secretion of hMSC cultivated in sequential + TSA is significant higher than control cultures (sequential) with p<0.01 (Oneway Anova + t-test).
   ΔΔΔ: ALB-secretion of hMSC cultivated in sequential + TSA is significant higher than control cultures (sequential) and hMSC cultivated in cocktail + TSA with p<0.001 (Oneway Anova + t-test), respectively.

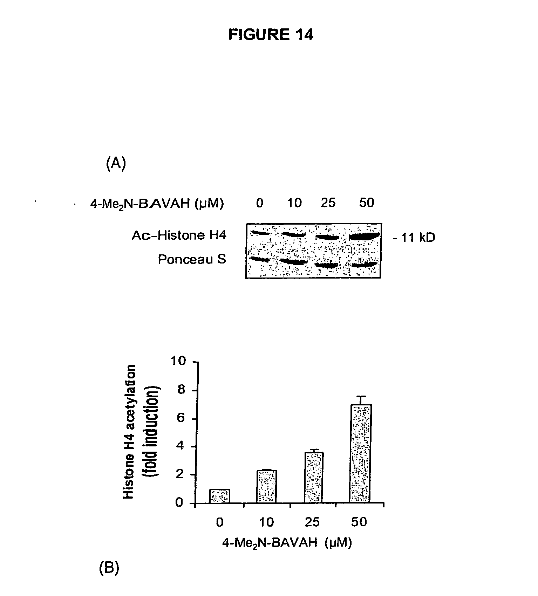

**Figure 14****.** *In vitro* HDAC inhibitory potential of 4-Me₂N-BAVAH. Hepatocytes were cultured during 48 hours (from time of plating on) in EGF-containing medium (50 ng/ml) in the absence (0 µM) or presence of 10, 25 or 50 µM 4-Me₂N-BAVAH. Histones were subsequently extracted and evaluated by Western blotting using a polyclonal antibody that is specifically directed against hyperacetylated histone H4 species (A). The blot shown is representative for three independent experiments. Quantification of Western blots by densitometry (B) is from 3 independent experiments.

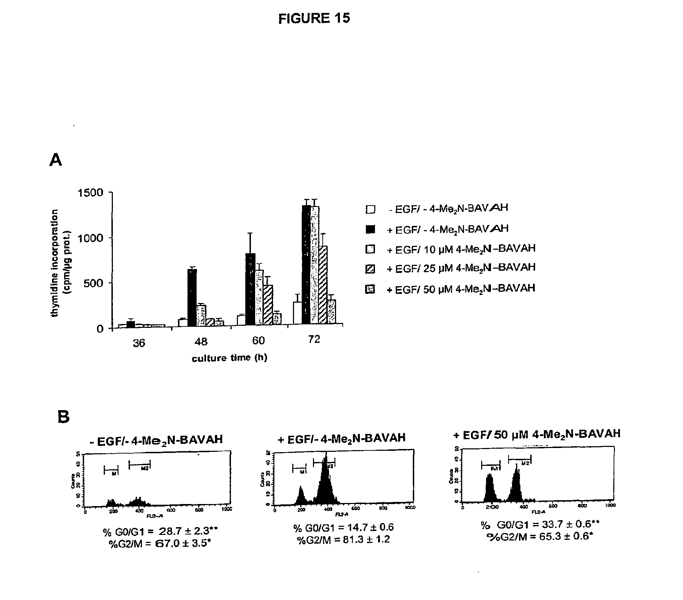

**Figure 15** (A) Dose-dependent inhibition of DNA replication by 4-Me₂N-BAVAH. Hepatocytes (0.4 x 10⁵ cells/cm²) were cultured in triplicate and ³H-thymidine incorporation was measured at the indicated time points. Results shown are the means±SD from 5 independent experiments. (B) FACS analyses of cell cycle distribution of primary rat hepatocytes at 72 hours of culture. Hepatocytes were either cultured in the absence or in the presence of EGF, alone or in combination with EGF and 4-Me₂N-BAVAH (50 µM). The histograms shown are derived from one particular experiment; the percentages±SD of cells in G0/G1 or G2/M are results from 3 independent experiments. The relative high percentage of G2/M hepatocytes in the non-proliferating conditions (-EGF/-4-Me₂N-BAVAH and +EGF/+ 50 µM 4-Me₂N-BAVAH) can be explained by the fact that mononuclear tetraploid hepatocytes (1 x 4n G2/M) cannot be discriminated from binuclear diploid hepatocytes (2 x 2n G0/G1). *p<0.05 or **p<0.001 for significant difference from EGF-stimulated untreated control cultures according to a Student t-test.
**Figure 16** (A) Dose-dependent inhibition of EGF-induced cyclin D1 protein expression analyzed by Western blotting. Hepatocytes (0.4 x 10⁵ cells/cm²) were stimulated with EGF (50 ng/ml) 4 hours after plating and treated or not with 10, 25 or 50 µM 4-Me₂N-BAVAH. Drug treatment started from time of plating and was maintained until harvesting at 72 hours of culture. (B) Effect of 4-Me₂N-BAVAH on c-jun protein expression- Cells (0.4 x 10⁵ cells/cm²) were plated in the absence of EGF, treated or not with 4-Me₂N-BAVAH (50 µM) and harvested 0.5, 1 and 3 hours after plating. (C) Effect of 4-Me₂N-BAVAH on downstream cell cycle proteins. Hepatocytes were cultured as described under (A) and harvested at 72 hours for Western blot analysis. Twenty-five µg of total cellular proteins were separated on a 10% (c-jun, cyclin D1, cdk4, cyclin E, cdk2 and cdk1), 15% (p21) or 7% (Rb Ser780) SDS-PAGE and blotted on nitrocellulose membranes. Equal sample loading was verified by Ponceau S staining. Blots shown are representative for at least three independent experiments.

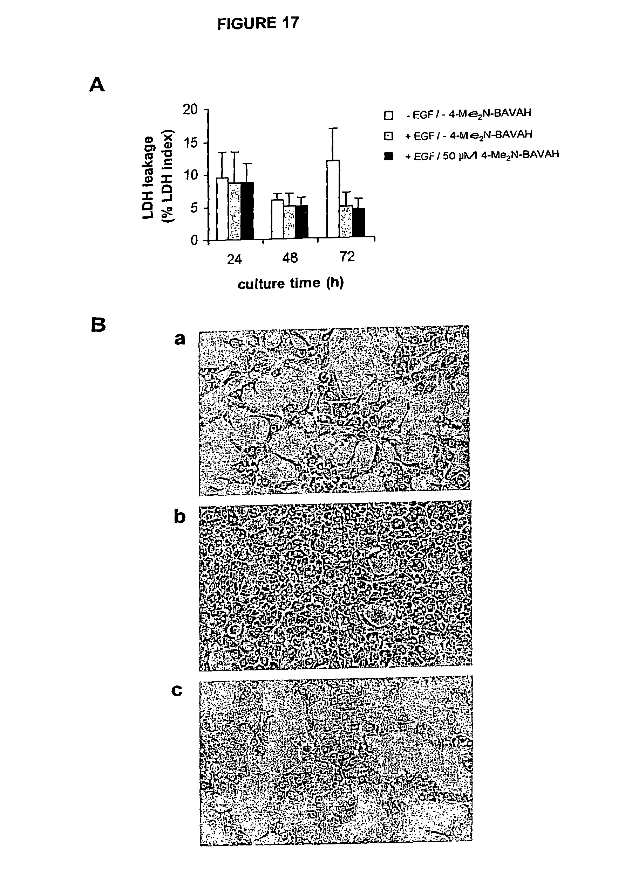

**Figure 17****.** (A) Effect of 4-Me₂N-BAVAH (50 µM) on the LD H leakage index from rat hepatocytes. The results are expressed as means±SD of five independent experiments. (B) Morphological appearance of primary rat hepatocytes at 72 hours of culture; (a) in the absence of both EGF and 4-Me₂N-BAVAH, (b) in the presence of EGF alone and (c) in the presence of both EGF and 4-Me₂N-BAVAH (50 µM). Light microscopy (x 100).
**Figure 18****.** Effect of 4-Me₂N-BAVAH on EGF-induced cyclin D1 and p21 protein expression. (A) Hepatocytes were cultured under the conditions indicated and harvested at 24, 48 and 72 hours of culture. (B) Hepatocytes were cultured during 24 hours in the presence of EGF alone or in the presence of both EGF and 4-Me₂N-BAVAH (50 µM). Thereafter, cultures were provided with fresh medium enriched with EGF alone or with EGF and 4-Me₂N-BAVAH. Cells were harvested 0.5, 1, 6 and 24 hours post medium renewal. (C) Quantification of 4-Me_{2N}-BAVAH and the acid and amide metabolite by HPLC combined with ESI-MS/UV detection. (D) Hepatocytes were cultured in the absence or presence of EGF. TSA treatment (1 µM) started during perfusion of the liver and was maintained throughout the culture period. Cells were harvested at 72 hours of culture and analyzed for their cyclin D1 and p21 protein expression. Proteins (25 µg) were separated on a 10% (cyclin D1) or 15% (p21) SDS-PAGE and blotted on nitrocellulose membranes. Blots shown are representative for at least three different experiments.

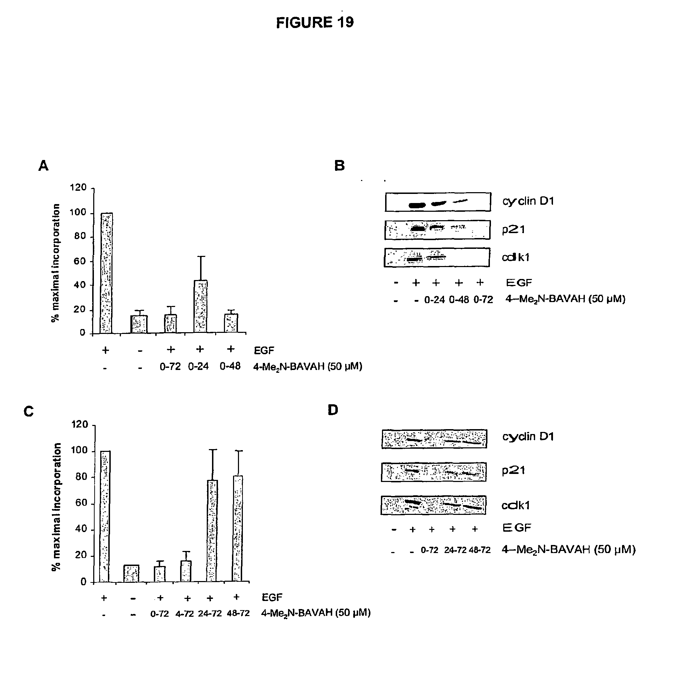

**Figure 19****.** Restoration of DNA replication and cell cycle protein expression depend on the length of 4-Me₂N-BAVAH exposure in G1 and on time of onset of treatment. Freshly isolated hepatocytes were cultured in the absence of EGF or stimulated with EGF 4 hours after plating. 4-Me₂N-BAVAH (50 µM) was added from time of plating (Oh) and was maintained until 24, 48 or 72 hours of culture. (A) ³H-Thymidine incorporation into DNA was measured at 72 hours of culture. Results are expressed as % of maximal incorporation observed in hepatocytes cultured in the presence of EGF alone. Means±SD from at least three independent experiments are shown. (B) Western blot analysis of cyclin D1, p21 and cdk1 protein expression at 72 hours of culture. Cells were cultured under the conditions described in (A). Blots shown are representative for three different experiments. (C) Freshly isolated hepatocytes were cultured in the absence of EGF or were stimulated with EGF, 4 hours after plating. 4-Me₂N-BAVAH (50 µM) was added at the indicated time points. ³H-Thymidine incorporation into DNA measured at 72 hours of culture. Results are expressed as % of maximal incorporation observed in hepatocytes cultured in the presence of EGF alone and are the means±SD from at least three independent experiments. (D) Western blots of cyclin D1, p21 and cdk1 protein expression at 72 hours of culture are shown. These are representative for three different experiments.

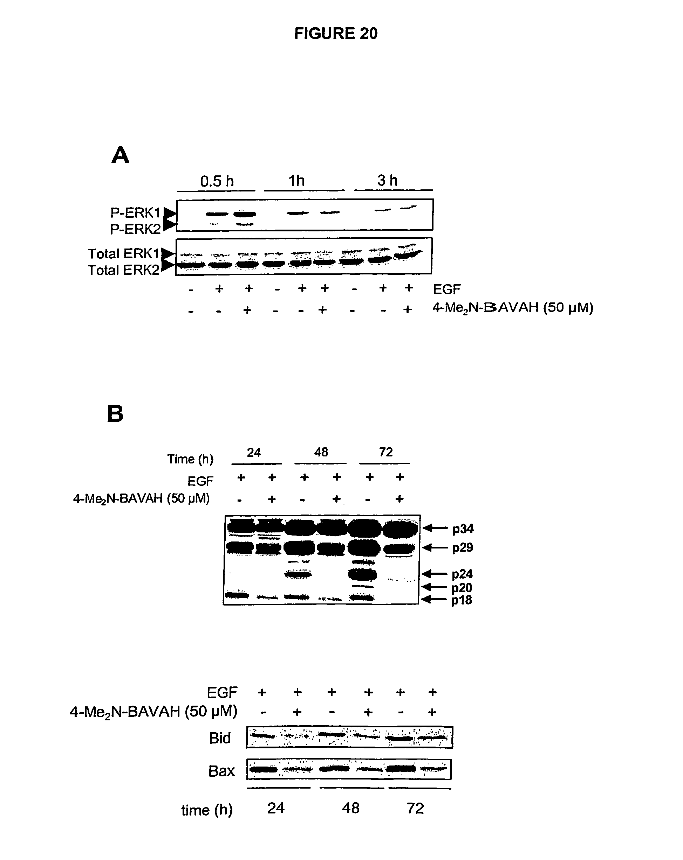

**Figure 20****.** (A) Effect of 4-Me₂N-BAVAH on ERK1/2 phosphorylation. Synchronized hepatocytes were stimulated or not with EGF (50 ng/ml) in the presence or absence of 4-Me₂N-BAVAH (50 µM) and analyzed 0.5, 1 and 3 hours after stimulation. Blots shown are representative for three independent experiments. (B) Freshly isolated hepatocytes were cultured under the conditions indicated and were analyzed for caspase-3 activation and Bid and Bax expression by Western blotting at 24, 48 and 72 hours of culture (n=3).

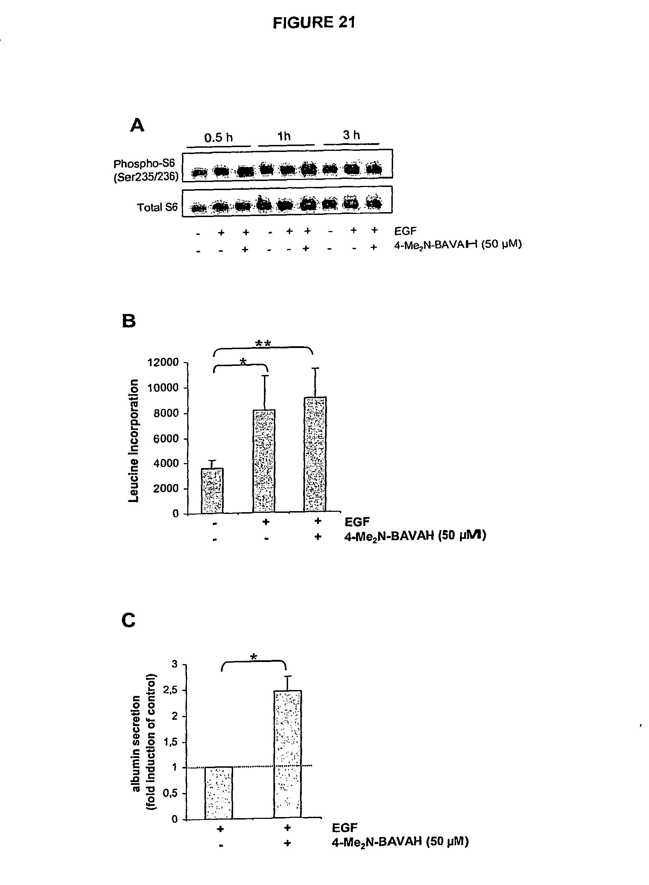

**Figure 21****.** (A) Effect of 4-Me₂N-BAVAH on S6 phosphorylation. Synchronized hepatocytes were stimulated or not with EGF (50 ng/ml) in the presence or absence of 4-Me₂N-BAVAH (50 µM) and analyzed 0.5, 1 and 3 hours after stimulation. (n=3) (B) De *novo* protein synthesis was measured by means of ³H-leucine incorporation as outlined in Materials and Methods. Results are expressed as cpm per culture dish (35 mm) (n=3). *p<0.001 and **p<0.0001 for statistically significant difference according to a Student t-test test. (C) Albumin secretion into the culture medium of rat hepatocytes, treated or not with 50 µM 4-Me₂N-BAVAH (n=4). Results are expressed as fold induction of control (i.e. untreated EGF-stimulated hepatocytes). *p<0.05 for statistically significant difference according to a Student t-test.

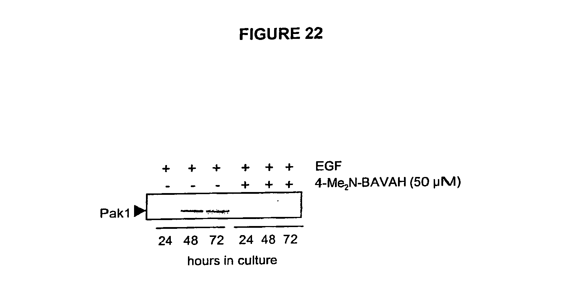

**Figure 22****.** Effect of 4-Me₂N-BAVAH on Pak1 expression. Hepatocytes were cultured in the presence of EGF (50 ng/ml) alone and either treated or not with 4-Me₂N-BAVAH (50 µM). Pak 1 expression was analyzed at the time points indicated. (n=3).

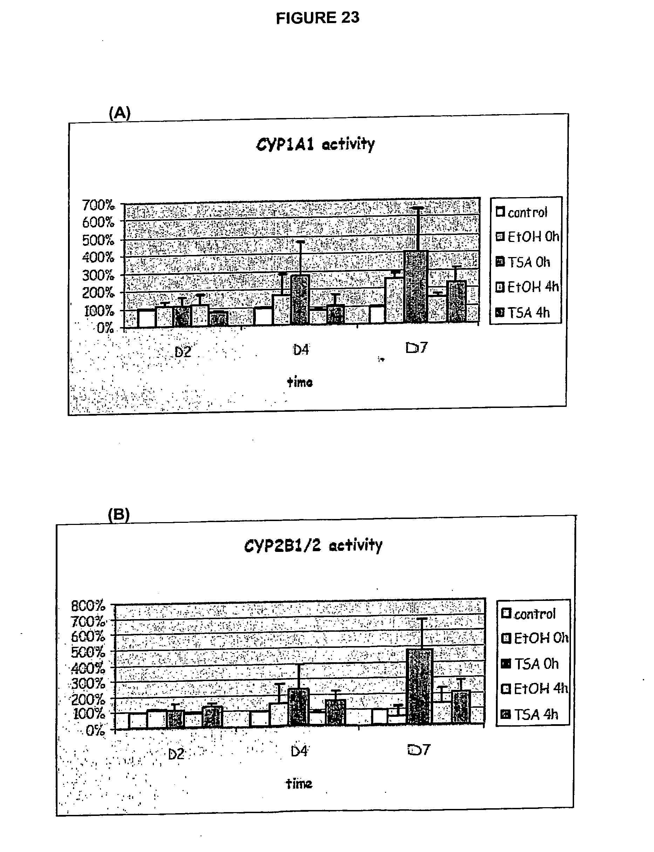

**Figure 23****.** The activity at day 4 and 7 of CYP 1A1 (A) and CYP 2B1/2 (B) in primary hepatocytes treated with TSA or its solvent ethanol starting at 0h or at 4h (negative control = untreated). Results are the mean ±SD of 2 independent isolations, expressed as % of the negative control (= 100%) of the related day.

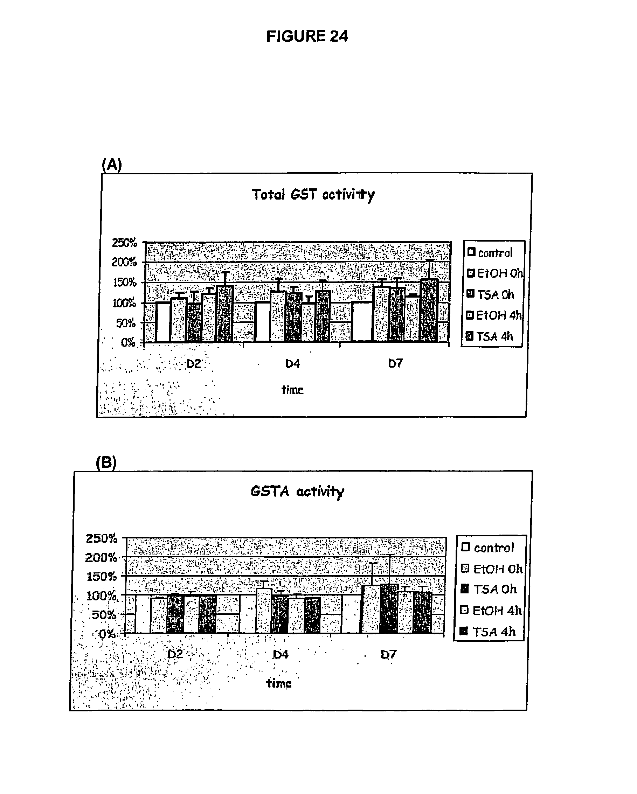

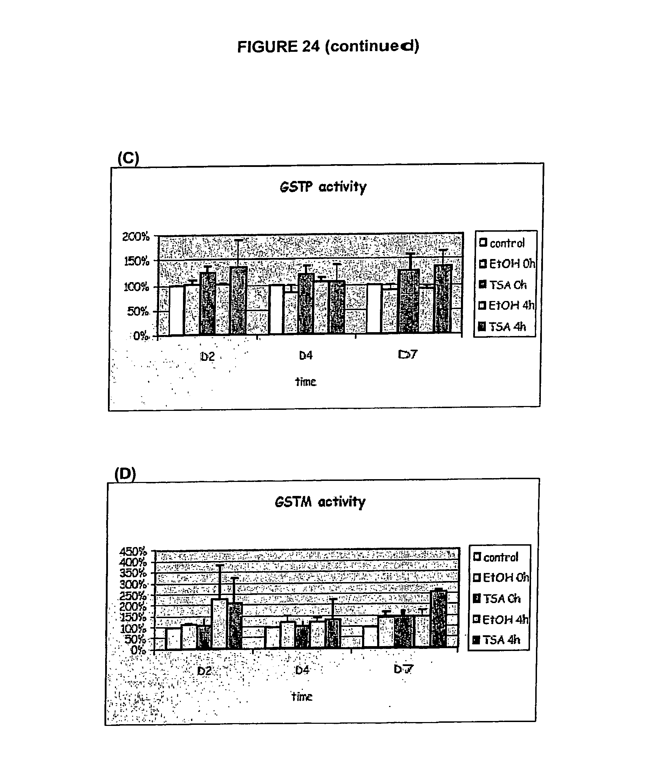

**Figure 24****.** The activity at day 4 and 7 of GST (total activity) (A), GSTA iso-enzyme (B), GSTP iso-enzyme (C), and GSTM iso-enzyme (D) in primary hepatocytes treated with TSA or its solvent ethanol starting at 0h or at 4h (negative control = untreated). Results are the mean ±SD of 2 independent isolations, expressed as % of the negative control (= 100%) of the related day.

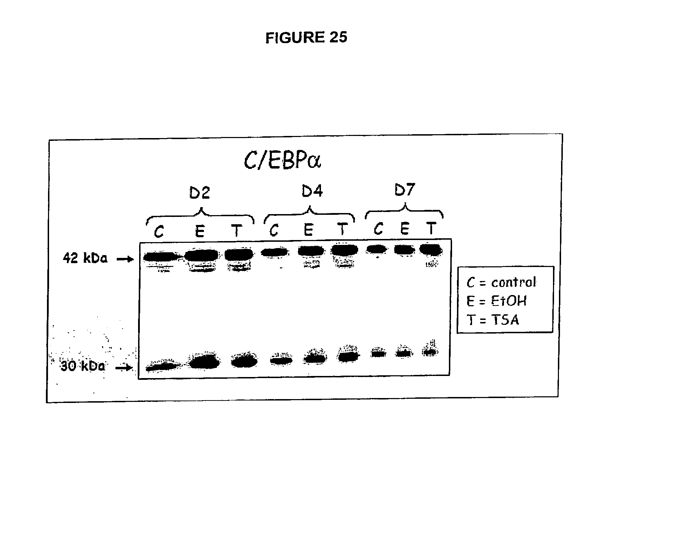

**Figure 25****.** Expression of the liver-enriched transcription factor C/EBPalpha at day 4 and 7 in primary hepatocytes treated with TSA or its solvent ethanol starting at 0h (negative control = untreated).

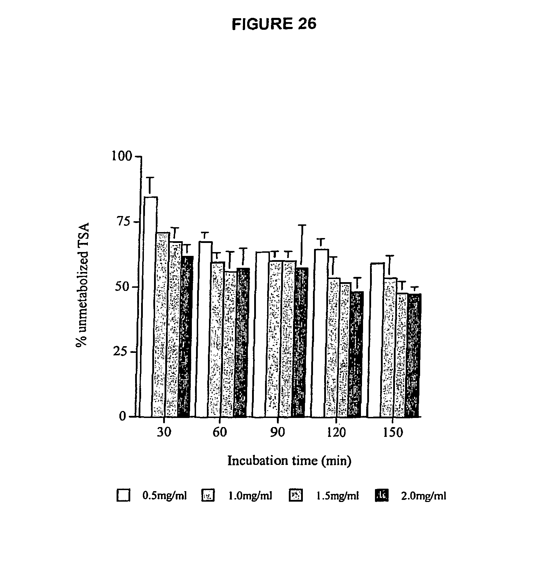

**Figure 26****.** TSA biotransformation by rat liver microsomes. The initial concentration of TSA was 140 µM. Results are expressed as % unmetabolized TSA as a function of the incubation time. Each point represents the mean (SD) of three experiments.

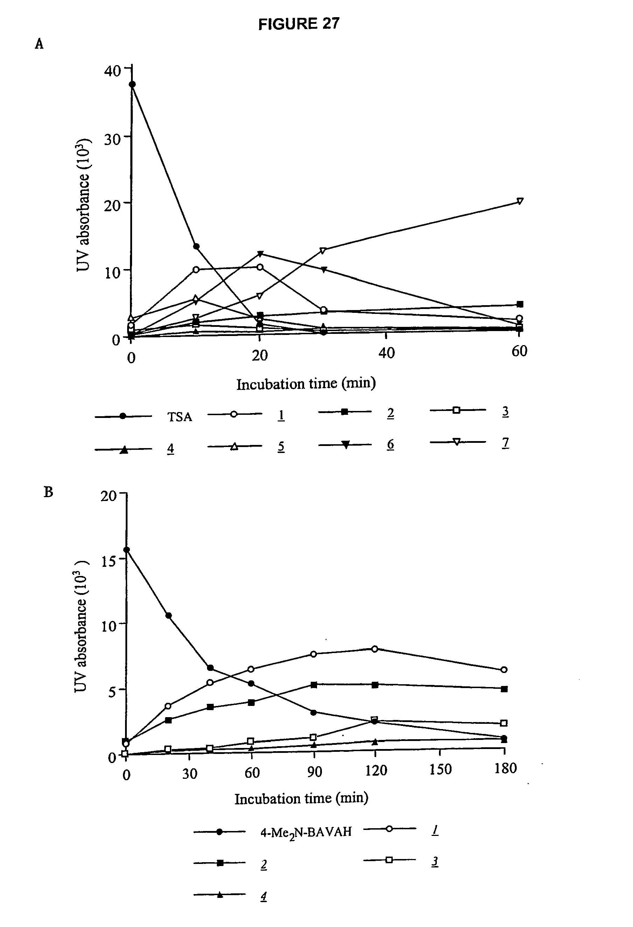

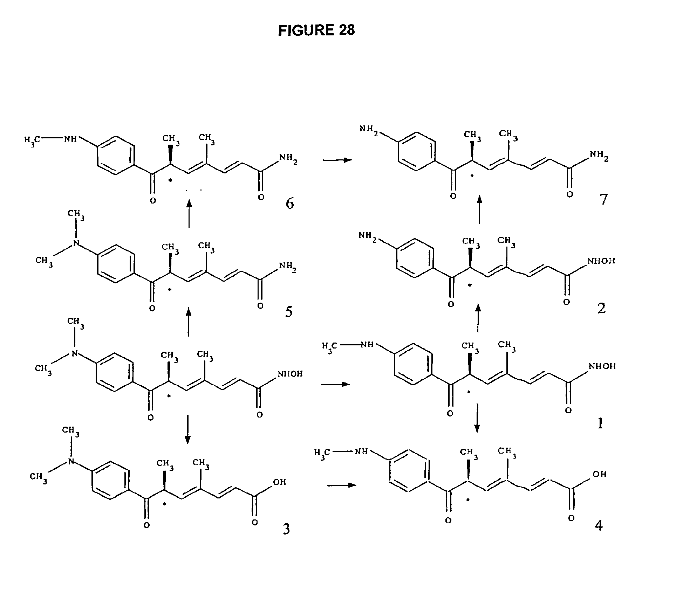

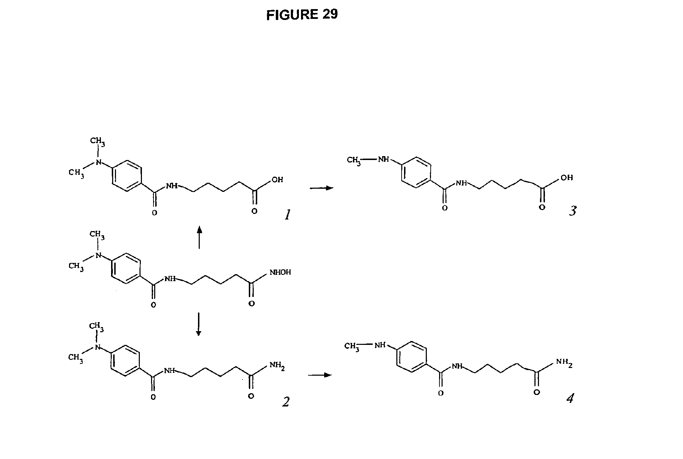

**Figure 27****.** Kinetics of TSA (A) and 4-Me₂N-BAVAH (B) consumption and metabolite production in suspensions of freshly isolated rat hepatocytes. 2×10⁶ rat hepatocytes/ml were exposed to 50µM TSA and 4-Me₂N-BAVAH during 1h and 3h, respectively. The peak areas detected at 266nm (TSA) and 255nm (4-Me₂N-BAVAH) as a function of the incubation time are shown. Each point represents the mean of three experiments. Standard deviations were omitted for clarity reasons. Metabolite structures are depicted in figures 28 and 29, respectively.
**Figure 28****.** Chemical structures of the phase I metabolites of TSA identified in suspensions of rat and human liver microsomes and in freshly isolated rat hepatocytes. The metabolites were identified by ESI CID MS/MS as N-monodemethylated TSA (1), N-didemethylated TSA (2), Trichostatic acid (3), *N*-monodemethylated Trichostatic acid (4), TSA amide (5), *N-*monodemethylated TSA amide (6) and *N*-didemethylated TSA amide (7).
**Figure 29****.** Chemical structures of the phase I metabolites of 4-Me₂N-BAVAH identified in suspensions of freshly isolated rat hepatocytes. The metabolites were identified by ESI CID MS/MS as the corresponding acid (*1*), amide (*2*) and the *N*-demethylated products of *1* (3) and *2* (*4*).

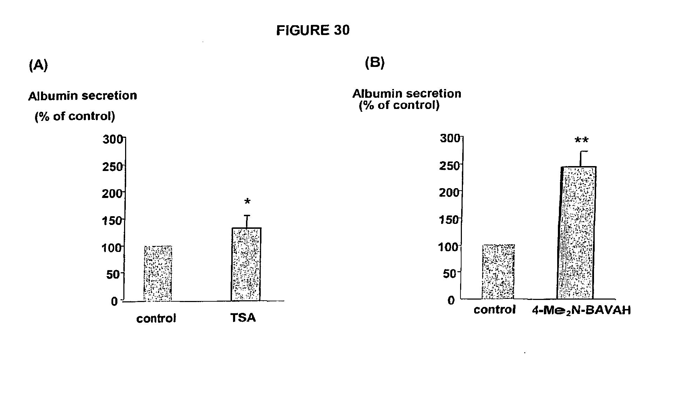

**Figure 30****.** Albumin secretion into the culture medium of rat hepatocytes, treated with 1 µM TSA (A) or with 50 µM 4-Me₂N-BAVAH. Results reflect 4 independent cultures and are expressed as fold induction of control (i.e. untreated EGF-stimulated hepatocytes). *p<0.05 and **<0.01 for statistically significant difference according to a Student t-test.

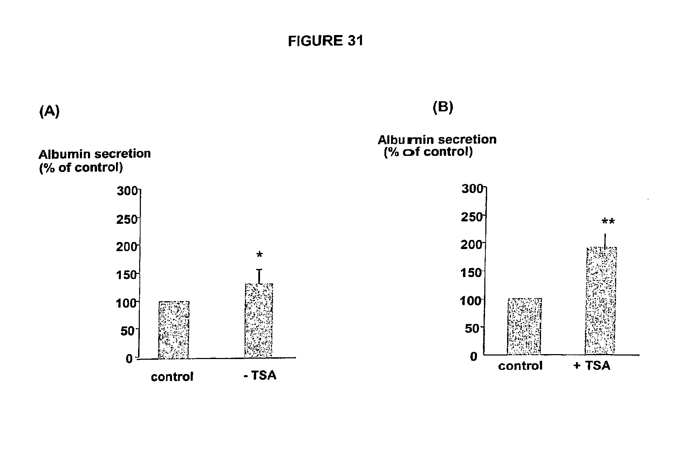

**Figure 31****.** Albumin secretion into the culture medium of rat hepatocytes, isolated in absence (A) or in presence (B) of 1µ M TSA in the collagenase perfusion solution.

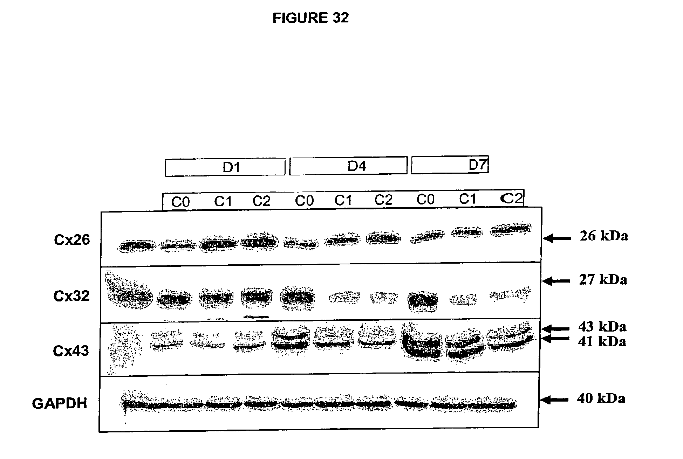

**Figure 32****.** Analysis of expression of Cx 26, Cx 32 and Cx 43 by Western blotting. Conditions: C0 - cells cultured in absence of TSA; C1 - cells cultured in presence of 1 µM TSA; C2 - isolated liver perfused with 1 µM TSA, and drug treatment continued during subsequent cultivation of the cells. D1, D4, D7 - samples taken at day 1, 4 or 7 of the cultivation time, respectively.

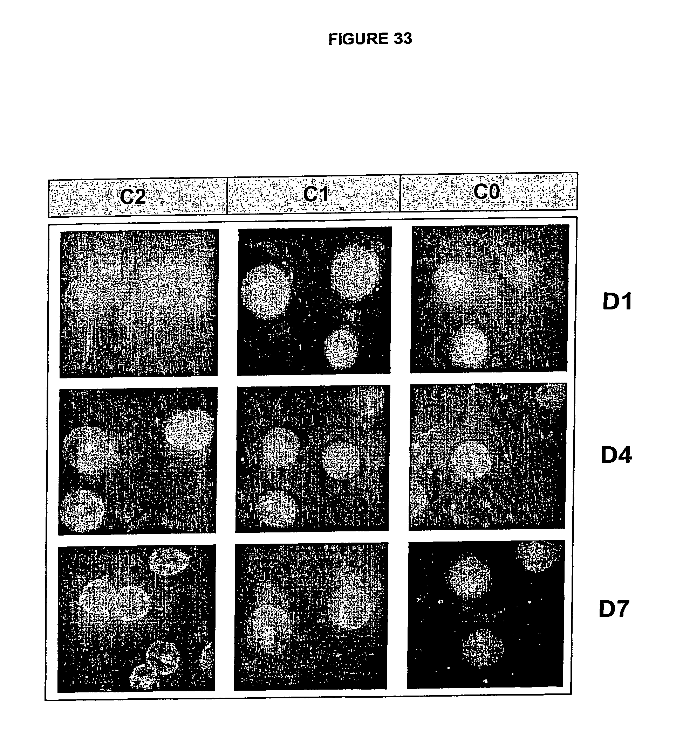

**Figure 33****.** Subcellular localization of Cx 26 analyzed by immunocytochemistry. Conditions: C0 - cells cultured in absence of TSA; C1 - cells cultured in presence of 1 µM TSA; C2 - isolated liver perfused with 1 µM TSA, and drug treatment continued during subsequent cultivation of the cells. D1, D4, D7 - samples taken at day 1, 4 or 7 of the cultivation time, respectively. Nuclei counterstained with DAPI.

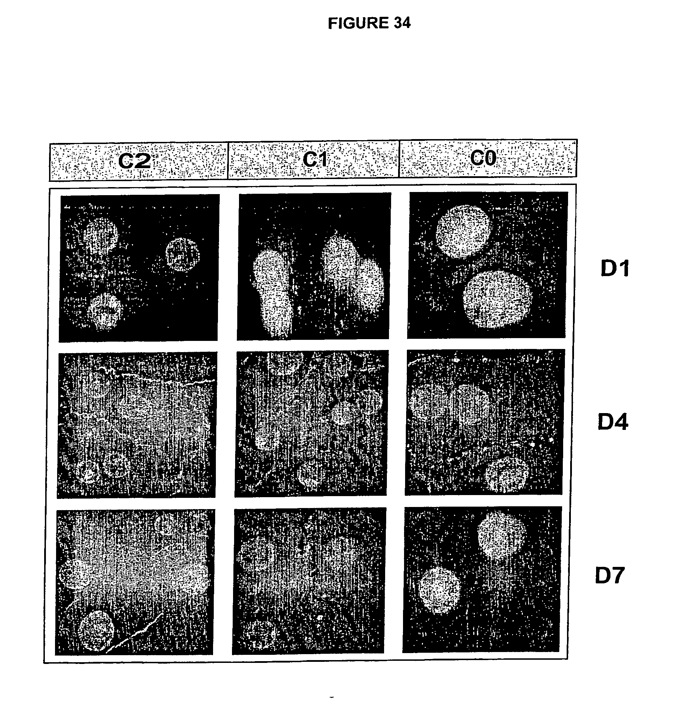

**Figure 34****.** Subcellular localization of Cx 32 analyzed by immunocytochemistry. Conditions: C0 - cells cultured in absence of TSA; C1 - cells cultured in presen ce of 1 µM TSA; C2 - isolated liver perfused with 1 µM TSA, and drug treatment continued during subsequent cultivation of the cells. D1, D4, D7 - samples taken at day 1, 4 or 7 of the cultivation time, respectively. Nuclei counterstained with DAPI.

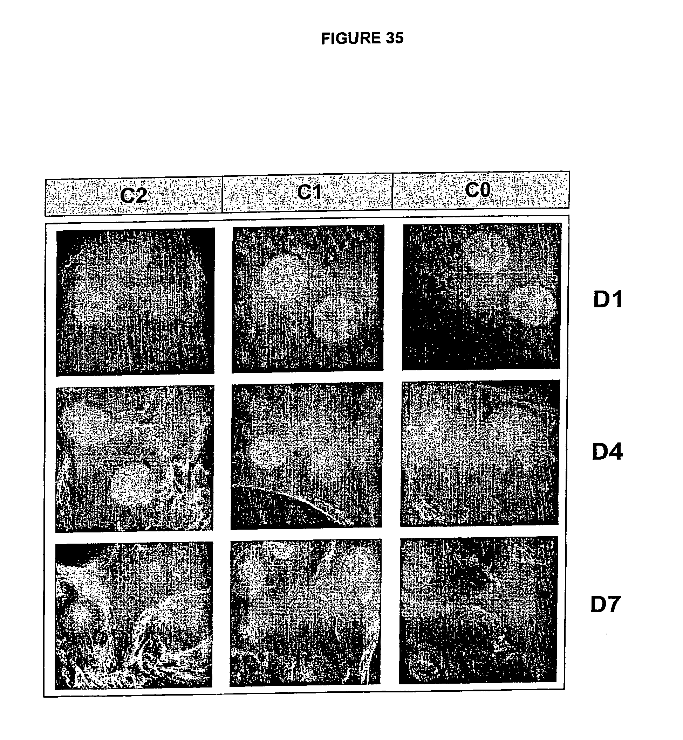

**Figure 35****.** Subcellular localization of Cx 43 analyzed by immunocytochemistry. Conditions: C0 - cells cultured in absence of TSA; C1 - cells cultured in presence of 1 µM TSA; C2 - isolated liver perfused with 1 µM TSA, and drug treatment continued during subsequent cultivation of the cells. D1, D4, D7 - samples taken at day 1, 4 or 7 of the cultivation time, respectively. Nuclei counterstained with DAPI.

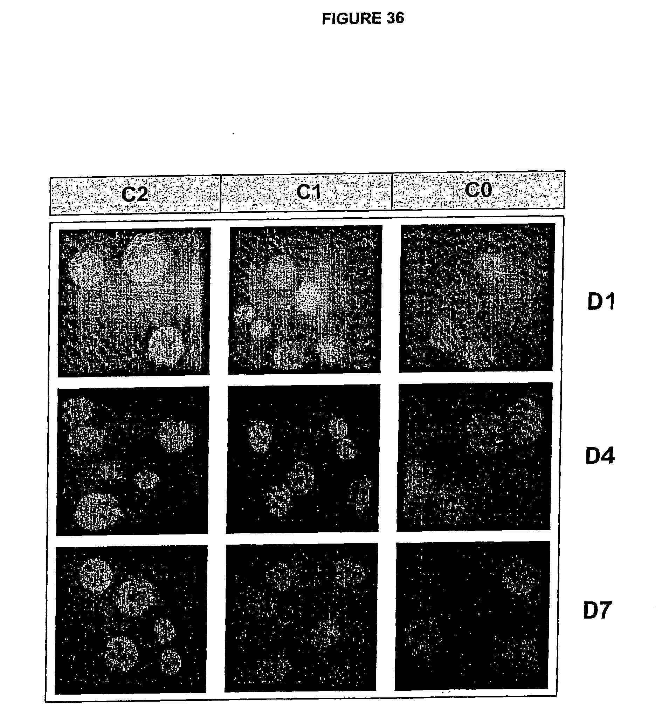

**Figure 36****.** Expression of acetylated histone H4 analyzed by immunocytochemistry. Conditions: C0 - cells cultured in absence of TSA; C1 - cells cultured in presence of 1 µM TSA; C2 - isolated liver perfused with 1 µM TSA, and drug treatment continued during subsequent cultivation of the cells. D1, D4, D7 - samples taken at day 1, 4 or 7 of the cultivation time, respectively.

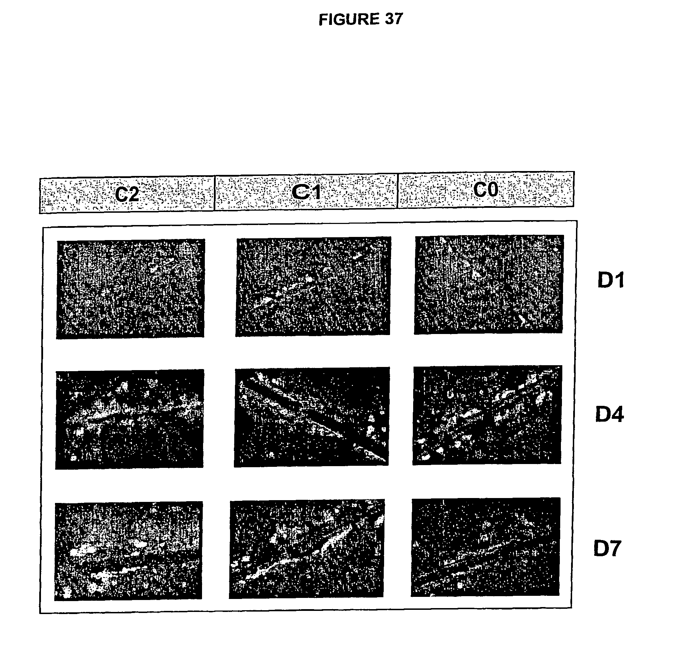

**Figure 37****.** Scrape loading/dye transfer assay. Conditions: C0 - cells cultured in absence of TSA; C1 - cells cultured in presence of 1 µM TSA; C2 - isolated liver perfused with 1 µM TSA, and drug treatment continued during subsequent cultivation of the cells. D1, D4, D7 - samples taken at day 1, 4 or 7 of the cultivation time, respectively.

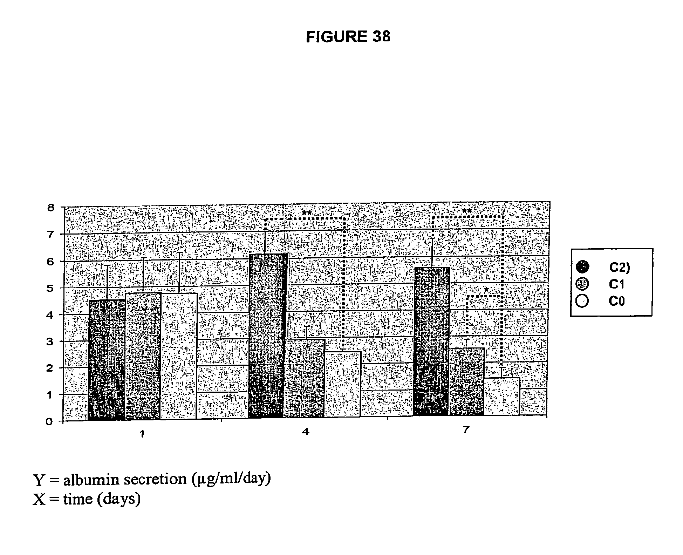

**Figure 38****.** Analysis of albumin secretion. Conditions: C0 -cells cultured in absence of TSA; C1 - cells cultured in presence of 1 µM TSA; C2 - isolated liver perfused with 1 µM TSA, and drug treatment continued during subsequent cultivation of the cells. D1, D4, D7 - samples taken at day 1, 4 or 7 of the cultivation time, respectively.

### Detailed Description of the Invention

In one aspect, the present invention provides a method for producing differentiated cells from stem cells *in vitro,* wherein at least one stem cell is sequentially exposed to two or more differentiation agents and to at least one histone deacetylase inhibitor.

In an embodiment, the present invention provides the method for producing differentiated cells from stem cells *in vitro,* wherein the method comprises the steps of:
(i.) providing at least one stem cell;
(ii) exposing said at least one adult stem cell to a first differentiation agent or a combination of differentiation agents chosen from fibroblast growth factor 4 (FGF-4), hepatic growth factor (HGF), ITS (insulin, transferrin, selenious acid), dexamethasone, oncostatin M (OSM), bone morphogenetic protein 2 (BMP-2), BMP-4 and BMP-7;
(iii) subsequently exposing said at least one adult stem cell to one or more another differentiation agent or another combination of differentiation agents chosen from those defined in step (ii);
(iv) repeating step (iii), whereby said at least one adult stem cell is sequentially exposed to a series of differentiation agents or combinations of differentiation agents chosen from those defined in step (ii); and
(v) exposing said at least one adult stem cell to at least one inhibitor of histone deacetylases during and/or after exposing said at least one adult stem cell to the differentiation agents according to steps (ii) to (iv).

Within the present specification, the terms "cell" or "cells" refer not only to the particular subject cell or cells but as well to the progeny or potential progeny of such a cell or cells. Because certain modifications may occur in succeeding generations due to either mutation or environmental influences, such progeny may not, in fact, be identical to the parent cell, but are still included within the scope of the terms "cell" or "cells" as used in the present specification.

Within the present specification, the term "stem cell" denotes any cell that, if exposed to appropriate conditions, is capable of giving rise to two or more different cell types. Such a stem cell may be capable of extensive, or perhaps indefinite, proliferation *in vivo* and, under specific conditions, also *in vitro,* wherein the progeny of such a stem cell may retain the phenotypic features and the proliferative capacity of the mother cell, or else may, if exposed to appropriate conditions, give rise to more specialized, i.e. more differentiated, cell(s). A stem cell is said to "give rise" to another, more differentiated, cell when, for example, the stem cell differentiates to become the other cell without previously undergoing cell division, or if the other cell is produced after one or more rounds of cell division and/or differentiation of the stem cell.

Within the present specification, the term "differentiation" denotes the process by which an unspecialized or less specialized cell, such as, for example, a stem cell or the progeny or potential progeny of such a stem cell, becomes more specialized. In the context of cell ontogeny, the adjective "differentiated" is usually a relative term. Hence, a "differentiated cell" is a cell that has progressed further down a certain developmental pathway than the cell it is being compared with. The differentiated cell may, for example, be a "terminally differentiated cell", which takes up specialized functions in various tissues or organs of an organism, or may also be a "precursor cell" or "progenitor cell" within a particular differentiation lineage, which can further proliferate and/or differentiate.

Based on its ability to give rise to diverse cell types, a stem cell may be usually described as totipotent, pluripotent, or multipotent. A single "totipotent stem cell" may be capable of growing, i.e. developing, into an entire organism. A "pluripotent stem cell" is believed not to be able of growing into an entire organism, but it is capable of giving rise to cell types originating from all three germ layers, i.e. mesoderm, endoderm, and ectoderm, according to standard art-accepted tests, such as the ability of a pluripotent stern cell to form teratomas in a suitable host. A "multipotent stem cell" is capable of giving rise to at least one cell type from each of two or more different organs or tissues of an organism, wherein said cell types may originate from the same or from different germ layers. Within the context of the present invention, the at least one stem cell may thus be a totipotent, a pluripotent, or a multipotent stem cell.

Pluripotent stem cells may be exemplified for example by embryonic stem (ES) cells of various types, comprising among others human embryonic stem cells, e.g., as described by Thomson et al. (Science 282:1145-1147, 1998); embryonic stem cells from other primates, such as Rhesus embryonic stem cells, e.g., as described by Thomson et al. (PNAS 92:7844-7848, 1995), or marmoset embryonic stem cells, e.g., as described by Thomson et al. (Biol Reprod 55: 254-259, 1996); or embryonic stem cells from mice, e.g., as described by Evans and Kaufman (Nature 292:154-156, 1981). As detailed in these and other publications, an embryonic stem cell is usually derived from the blastocyst stage of the embryo. A blastocyst is normally sphere-shaped and comprises an outer layer of cells (the trophectoderm), a fluid-filled cavity (the blastocoel), and a cluster of cells in the interior (the inner cell mass). The ES cells are typically derived from the inner cell mass of the blastocyst.

Pluripotent stem cells may also be exemplified by embryonic germ (EG) cells, comprising among others human embryonic germ cells, e.g., as described by Shamblott et al. (PNAS 95:13726-13731, 1998). Such cells may be derived from gonadal ridges and mesenteries containing primordial germ cells from fetuses. In humans, such fetuses may be typically 5-9 weeks postfertilization.

By the term "adult stem cell" is usually understood a stem cell found in fetal or in adult tissues or organs of an organism. An adult stem cell is usually capable of developing into all cell types of the tissue or organ in which it resides. Typically, an adult stem cell will generate one or more intermediate cell types, i.e., precursor or progenitor cell types, before achieving the fully differentiated state of a mature cell type. An adult stem cell is also characterized by its self-renewal capacity, i.e., by its ability to proliferate without differentiation for prolonged periods of time. *In vivo,* an adult stem cell is predicted to maintain this self-renewal capacity for the entire life-span of the organism. *In vitro,* the self-renewal capacity of an adult stem cell is typically more limited than that of an embryonic stem cell or an embryonic germ cell. To date, adult stem cells have been identified in many animal and human tissues or organs, such as, for example, in bone marrow, peripheral blood, brain, spinal cord, dental pulp, blood vessels, skeletal muscle, epithelia of the skin and digestive system, cornea, retina, liver, or pancreas. It is generally expected that adult stem cells are present in all tissues or organs of an organism.

Typically, adult stem cells have been considered to be capable of producing only the cell types of the tissue or organ, in which they reside. However, recently numerous studies demonstrated the *in vivo* and *in vitro* capacity of adult stem cells to generate specialized cell types of other tissues or organs than those in which they normally reside. Such other tissues or organs may be derived from the same or from a different embryonic germ layer than the tissue of origin of the adult stem cell. Hence, adult stem cells may have the characteristics of multipotent, or even pluripotent, stem cells. As a non-limiting example, bone marrow stem cells, which are derived from the mesoderm, may be able to generate both skeletal muscle, also derived from the mesoderm (Ferrari et al. Science 279: 528-530,1998; Gussoni et al. Nature 401: 390-394, 1999), neurons, which are derived from the ectoderm (Mezey et al. Science 290:1779-1782, 2000), and hepatic cells, which are derived from the endoderm (Petersen et al. Science 284: 1168-1170, 1999). In another non-limiting example, Clarke et al. (Science 288: 1660-1663, 2000) reported that neural stem cells injected into mouse blastocysts can contribute to all tissues of the chimeric mouse. This capacity of adult stem cells to differentiate to cells of other tissues may be interchangeably referred to as "plasticity", e.g., as in Brazelton et al. (Science 290: 1775-1779, 2000) and Krause et al. (Cell 105: 369-377, 2001), "unorthodox differentiation", e.g., as in Bianco et al. (Exp Cell Res 251: 257-263, 1999), or "transdifferentiation", e.g., as in Anderson et al. (Nat Med 7: 393-395, 2001) and Lagasse et al. (Nat Med 6: 1229-1234, 2000)

In view of the different possible origins and types of stem cells, it should be understood that the present invention may use any type of stem cells, either existing or to be derived in the future. Accordingly, in the present invention, the at least one stem cell is an adult stem cell.

In add ition, it should be appreciated that the stem cells used in the present invention may be derived from tissues or organs of either human origin or animal origin.

In accordance, in an embodiment of the present invention, the at least on e stem cell is derived from a human. Under "a human" is understood a human organism at any stage of its development after fertilization. Such stages may for example include the pre-embryonic stage, embryonic stage, fetal stage, as well as all stages following the birth, up to and including the adulthood and post-mortem stages.

In another embodiment of the present invention, the at least one stem cell is derived from an animal. Under "an animal" is understood an animal organism at any stage of its development after fertilization. Such stages may for example include the pre-embryonic stage, embryonic stage, fetal stage, as well as all stages following the birth, up to and including the adulthood and post-mortem stages.

It should be appreciated that stem cells from different, and likely all, animal species may be used in the context of the present invention. For example, in an embodiment of the present invention, said animal is chosen from the group comprising mouse, rat, guinea pig, hamster, rabbit, chicken, cat, dog, a non-human primate, pig, sheep, cow, horse, fish, reptile, and amphibian.

The choice of the stem cells for use in the method of the present invention will likely be governed by the foreseen application for the differentiated cells obtainable by the method, as well as by distinct advantages and/or disadvantages of particular stem cells.

In another non-limiting example, the use of human stem cells may be preferred to the use of animal stem cells, when differentiated cells obtainable by the method are to be administered, e.g., transplanted, into the body of a human subject.

In another non-limiting example, the use of human adult stem cells, as obtainable from a particular human subject, is preferred to the use of human embryonic stem cells or embryonic germ cells, when the differentiated cells obtainable by the method of the invention are to be introduced, e.g., transplanted, into the body of said human subject, because the differentiated cells obtainable from adult stem cells of said human subject are largely genetically identical to the other cells of the subject, thus minimizing the likelihood of the immunological rejection of the introduced cells. On the contrary, differentiated cells obtainable from human embryonic stem cells or embryonic germ cells may be genetically more distant from the other cells of the subject, thus possibly provoking the immunological rejection of the introduced cells. It will be appreciated that this non-limiting example may also be extended to the use of animal adult stem cells for a similar purpose in animals.

In yet another non-limiting example, the use of human ad ult stem cells is preferred to the use of human embryonic stem cells or embryonic germ cells, due to the complexity of the ethical issues relating to the isolation and use of human embryonic stem cells. Hence, in the present invention, the at least one stem cell is an adult stem cell.

As mentioned above, adult stem cells have been identified in many animal and human tissues or organs, such as, for example, in bone marrow, peripheral blood, brain, spinal cord, dental pu Ip, blood vessels, skeletal muscle, epithelia of the skin and digestive system, cornea, retina, liver, or pancreas, and it is generally expected that adult stem cells are present in all tissues or organs of an organism. Therefore, in an embodiment of the present invention, the at least one adult stem cell may be derived from any tissue or organ of a human or animal organism.

Among the so-far identified adult stem cells, the properties of some types of adult stem cells are experimentally characterized to a greater extent than the properties of other types. The use of the better-characterized types of adult stem cells may be particularly useful in the context of the present invention. Hence, in an embodiment of the present invention, the at least one adult stem cell is chosen from the group comprising a bone marrow stem cell (hematopoietic stem cell and mesenchymal stem cell), a bone marrow stromal stem cell (mesenchymal stem cell and multipotent adult progenitor cell), a neural stem cell and all stem cells from the central nervous system (adult, foetal), endothelial progenitor stem cells, skeletal muscle stem cells, epidermal and epithelial stem cells in skin and digestive system and stem cells of pancreas and liver.

A "hematopoietic stem cell" (HSC) is a mesoderm-derived cell that can be isolated from, e.g., bone marrow, blood, umbilical cord blood, fetal liver and yolk sac, and that is capable of generating, e.g., blood cells and multiple hematopoietic lineages. For example, when introduced into lethally irradiated animals or humans, hematopoietic stem cells can repopulate the erythroid, neutrophil-macrophage, megakaryocyte and lymphoid hemopoietic cell pools. *In vitro,* hematopoietic stem cells can be induced to undergo at least self-renewing cell divisions and can be induced to differentiate into at least the same lineages as is seen *in vivo.* The plasticity of hematopoietic stem cells was demonstrated for example by Lagasse et al. (Nat Med 6:1229-1234, 2000), who showed that purified HSC can differentiate into hepatocytes *in vivo,* or by Gussoni et al. (Nature 401: 390-394, 1999), who showed that bone marrow Sp cells, which are highly enriched for HSC, can differentiate into muscle cells. Accordingly, in an embodiment of the present invention, the at least one stem cell is a hematopoietic stem cell.

A "mesenchymal stem cell" (MSC) is a mesoderm-derived cell that can be isolated from, e.g., bone marrow, blood, umbilical cord, placenta, fetal yolk sac, fetal and adolescent skin (dermis), and periosteum, and that is capable of generating cells of, e.g., multiple mesenchymal lineages, such as osteocytic (bone), chondrocytic (cartilage), myocytic (muscle), tendonocytic (tendon), fibroblastic (connective tissue), adipocytic (fat), and stromogenic (marrow stroma) lineage. Human mesenchymal stem cells, their isolation and in *vitro* expansion, have been described in, e.g., US Pat. No. 5,486,359; US Pat. No. 5,811,094; or US Pat. No. 5,736,396. Description of mesenchymal stem cells from other organisms can be found in, e.g., Cassiede et al. (J Bone Miner Res 11: 1264-1273, 1996). *In vitro,* MSC have shown to be capable of being differentiated into, for example, osteocytes, chondrocytes, marrow stromal cells (US Pat. No. 5,736,396), myocytes (WO 9,639,035 A1), or adipocytes (US Pat. No. 6,709,864). Plasticity of MSC is further demonstrated also by WO2004/016779, which discloses a method for transdifferentiating mesenchymal stem cells into neuronal cells. Accordingly, in an embodiment of the present invention, the at least one stem cell is a mesenchymal stem cell.

A "multipotent adult progenitor cell" (MAPC) is a specific type of cell that co-purifies with mesenchymal stem cells when isolated from bone marrow; proliferates *in vitro* without obvious senescence or loss of differentiation potential; expresses markers commonly only found in ES or EG cells, such as oct-4 and Rex-1; and can be, at a single cell level, induced to differentiate *in vitro* not only into mesenchymal cells, but also into cells having characteristics of visceral mesoderm (endothelium), neuroectoderm (astrocytes, oligodendrocytes, neurons), and endoderm (hepatocytes) (Jiang et al. Nature 418:41-49, 2002). Moreover, when injected into mouse blastocysts, MAPC contribute to most or all tissues *in vivo,* thus further demonstrating their pluripotency (Jiang et al. Nature 418:41-49, 2002). Accordingly, in an embodiment of the present invention, the at least one stem cell is a multipotent adult progenitor cell.

The term "bone marrow stem cell" (BMSC) refers to any stem cell isolated from the bone marrow, which may be obtained, e.g., from iliac crest, femora, tibiae; spine, rib or other medullary spaces. Such BMSC may among others be a hematopoietic stem cell, a mesenchymal stem cell, or a multipotent adult progenitor cell. Accordingly, in an embodiment of the present invention, the at least one stem cell is a bone marrow stem cell.

"Neural stem cells" (NSC) are ectoderm-derived cells that were initially isolated from the subventricular zone and the olfactory bulb of fetal brain, but were later also identified in adult brains of rodents, non-human primates and humans (Gage et al. Science 287: 1433-1438, 2000; Svendsen et al. Brain Path 9: 499-513,1999). NSC can proliferate *in vivo* and continuously regenerate at least some neuronal cells *in vivo.* When cultured *in vitro,* neural stem cells can be induced to proliferate, as well as to differentiate into different types of neurons and glial cells (e.g., astrocytes, oligodendrocytes). When transplanted into the brain, NSC can engraft and generate neural cells and glial cells. Accordingly, in an embodiment of the present invention, the at least one stem cell is a neural stem cell.

"Gastrointestinal stem cells" are described in, e.g., Potten et al. (Philos Trans R Soc Lond B Biol Sci 353:821-830, 1998). Accordingly, in an embodiment of the present invention, the at least one stem cell is a gastrointestinal stem cell.

"Epidermal stem cells" are described in, e.g., Watt et al. (Philos Trans R Soc Lond B Biol Sci 353: 831-837, 1998). In another embodiment of the present invention, the at least one stem cell is an epidermal stem cell.

"Hepatic stem cells", also termed oval cells, are described in, e.g., Alison et al. (J Hepatol 29: 676-682, 1998). In yet another embodiment, the at least one stem cell is a hepatic stem cell.

In the present invention, stem cells are differentiated *in vitro* by being exposed to a series of differentiation agents, which are administered to the stem cells in a specific sequence, as opposed to being administered as a mixture. The term "differentiation agent" relates to any agent exerting a desirable effect on the differentiation of said stem cells. In one embodiment, such differentiation agent is chosen from the group comprising a growth factor, a cytokine, an interleukin, a hormone, a protein, an enzyme, a vitamin, a metabolite, a nutrient, a small molecule, a chemical compound, a solvent, or a chemical element.

A useful example of differentiation agents are growth factors and cytokines. These are essentially biological molecules, such as, for example, proteins or peptides, which provide signals for cell proliferation and/or differentiation during the ontogenic development of an organism. Growth factors and cytokines for use in the method of the present invention may, for example, be isolated from an organism, or may be produced using recombinant DNA techniques in cellular expression systems.

In the method of the present invention, the differentiation agents may be added to the media used to culture the stem cells. Such media will be optimized for each particular type of stem cell and will be known to a person skilled in the art. As illustrative, but non-limiting examples, the media optimized to culture rat multipotent adult progenitor cells and human mesenchymal stem cells are described in Examples section of this specification. Additives preventing spontaneous, undesirable differentiation of the cultured stem cells may also be added to the media. If needed, the stem cells may be cultured using feeder cell layers, matrix components, such as, e.g., fibronectin, collagen, laminin, or matrigel, or with other types of cells to form co-culture.

At the time of addition of the first differentiation agent or the first combination of differentiation agents, the adult stem cells may be 50-100% confluent, but may preferably be 80-100% confluent, and more preferably 90-100% confluent. Embryonic stem cells or embryonic germ cells may have other confluence. The particular order, identity, and time of exposure to stem cells of the differentiation agents used to differentiate stem cells using the method of the present invention will need to be optimized for each given stem cell type and differentiated phenotype to be obtained. Based on knowledge of ontogenic development of a particular organism, a person skilled in the art may be able to make educated guesses about the choice of the differentiation agents, which will provide a basis for further experimentation to optimize the exact condition of treatment. As non-limiting examples, conditions to achieve differentiation of rat multipotent adult progenitor cells and human mesenchymal stem cells into hepatocytes, and rat multipotent adult progenitor cells to neuro-ectodermal cells are detailed in the Examples section of this specification.

In the present invention, stem cells are differentiated *in vitro* by exposing to a series of differentiation agents administered in a specific sequence, and by exposing to at least one histone deacetylase inhibitor.

"Histone deacetylases" (hereinafter also referred to as HDAC) represent a class of enzymes that catalyze removal of an acetyl group from the epsilon-amino group of lysine side chains in histones (for example, histones H2A, H2B, H3 or H4), thereby reconstituting a positive charge on the lysine side chain (Gray et al. Exp Cell Res 262:75-83, 2001). Without being bound by the theory, hyperacetylated histones are in general associated with transcriptionally active chromatin regions, whereas transcriptionally inactive chromatin is enriched in hypoacetylated histones. Thereby, HDAC play an essential role in regulating gene expression. As cell proliferation and differentiation are controlled to a large extent at the transcriptional level (Kouzarides et al. Curr Opin Genet Dev 9:40-48, 1999), HDAC play an important role in regulating these processes.

Based on sequence similarity, mammalian HDAC can be classified into three distinct families: Class I, including but not limited to HDAC1, HDAC2, HDAC3, HDAC8, and HDAC11; Class II, including but not limited to HDAC4, HDAC5, HDAC6, HDAC7, HDAC9, and HDAC11; and Class III.

The term "histone deacetylase inhibitor" (hereinafter also referred to as HDAC inhibitor) denotes any agent capable of inhibiting the activity of at least one type of histone deacetylase by, for example, 10%, 20%, 30%, 40%, 50%, 60%, 70%, 80%, 95% or more. HDAC inhibitors may preferably inhibit both Class I and Class II HDAC. Such a gents may take the form of a chemical compound, a pharmaceutical agent or drug, a therapeutically effective oligonucleotide, a specific binding agent, or a fragment or variant of histone deacetylase. Within the present invention, the HDAC inhibitor may preferably be a specific HDAC inhibitor, i.e., an agent that, while inhibiting the activity of at least one HDAC, does not substantially effect other enzyme systems, and wherein the effect(s) on downstream cellular components and functions are a direct consequence of that agent.

Several structural classes of histone deacetylase inhibitors have been identified, comprising naturally occurring as well as and synthetic compounds. These include, by means of example and not limitation, benzamides (e.g., MS-27-275, Cl-994), sulfonamide-based anilides, straight chain anilides, heterocyclic ketones (e.g., apicidin and its derivatives, FR235222), allyl sulfur compounds (e.g., diallyl sulfide), psammaplins (e.g., psammaplin A), heterocyclic thiols (e.g., depsipeptide, spiruchostatin A), sulfur-containing cyclic peptides, bromoacetamide-based straight chain inhibitors, short-chain fatty acids (e.g., sodium butyrate), pivaloyloxymethyl butyrate, tributyrin, phenylalkanoic acids, valproic acid, phenylbutyrate, N-acetylcystein sulforaphane, cystein sulforaphane, straight chain trifluoromethylketones, alpha-ketoamides, alpha-ketoesters, heterocyclic ketones, cyclic trifluoromethylketones, cyclic pentafluoromethylketones, heterocyclic epoxides (e.g., trapoxin A), depudecin, short-chain fatty hydroxamic acids, trichostatins (e.g., trichostatin A), hybrid polar compound (e.g., suberoyl anilide hydroxamic acid, pyroxamide), benzamide-based hydroxamic acids (e.g., 5-(4-dimethylaminobenzoyl)aminocaproic acid hydroxamide, 5-(4-dimethylaminobenzoyl)-aminovaleric acid hydroxamid), arylketones, amino acid-containing benzamide based hydroxamic acids, indole amide-based hydroxamic acids, aryl-heterocyclic-based hydroxamic acids, aryl-pentadienoic hydroxamic acids, sulfonamide-based hydroxamic acids (e.g., oxamflatin, PXD101), (bi-)aryl-(heterocyclic)-based hydroxamic acids (e.g., SK-7041), NVP-LAQ824, A-161906, aroyl-pyrrole-hydroxyamides, cyclic hydroxam ic acid-containing peptides, cyclic hexapeptides, succinimide hydroxamic acids, cystein-based hydroxamic acids, 1,3-dioxanes (e.g., tubacin), phosphorus-based straight chain inhibitors, cyclostellettamines (e.g., cyclic 3-alkyl pyridinium dimers), straight-chain retrohydroxamates, cyclic retrohydroxamates, semicarbazide-based straight chain inhibitors, and thiol-based straight chain inhibitors. In an embodiment of the present invention, the at least one histone deacetylase inhibitor will be chosen from HDAC inhibitors falling into one of the above structural classes. However, it will be appreciated that all presently known HDAC inhibitors, whether falling into one of the above categories or not, may be used in the method according to the present invention. Also, novel HDAC inhibitors identified in the future, will be ca pable of being used in the method according to the present invention. In addition, if any HDAC inhibitor needs to be used as a derivative, e.g., as a salt, in order to extert the maximal effect in solution, in cells, or in an organism, such derivatives may be used in the method according to the present invention.

Trichostatin A (hereinafter also referred to as TSA), a fungistatic antibiotic purified from Streptomyces *platensis,* is a potent specific inhibitor of Class I and Class II HDAC both *in vitro* and *in vivo* (Yoshida et al. J Biol Chem 265:17174-17179, 1990). TSA shows a potent antitumor activity both *in vitro* and *in vivo.* Without being bound to theory, this effect of TSA is due to it inducing differentiation, cell cycle arrest, and/or apoptosis in transformed cells. Trichostatin A may be brought in the form of derivates, such as salts. As such derivates may be used so long as they do not adversely affect the desired effects of the compound. Accordingly, in an embodiment of the present invention, the at least one histone deacetylase inhibitor will be trichostatin A or a derivate thereof.

The production of trichostatin A is costly, lengthy and inefficient. Semi-synthetic or synthetic TSA analogues may be more easily available. A TSA analogue may be brought in the form of derivatives, such as salts. As such derivative may be used so long as they do not adversely affect the desired effects of the compound. Accordingly, in an embodiment of the present invention, the at least one histone deacetylase inhibitor will be a semi-synthetic or a synthetic analogue of trichostatin A or a derivative of such analogue.

For example, Jung et al. (J Med Chem 42: 4669-4679, 1999) described the production and properties of several amide based analogues of trichostatin A, including, e.g., 5-(4-dimethylaminobenzoyl)-aminocaproic acid hydroxamid. For example, Van Ommeslaeghe et al. (Bioorg Med Chem Lett 13:1861-1864, 2003) described the production of several am ide based analogues of TSA, among others 5-(4-dimethylaminobenzoyl)-aminovaleric acid hydroxamid. An amide based analogue of trichostatin A may be brought in the form of derivatives, such as salts. As such derivatives may be used so long as they do not adversely affect the desired effects of the compound. Accordingly, in an embodiment of the present invention, the at least one histone deacetylase inhibitor will be an amide based analogue of trichostatin A or a derivative thereof.

5-(4-dimethylaminobenzoyl)-aminovaleric acid hydroxamid is a potent HDAC inhibitor and is metabolically more stable than TSA, when metabolized in freshly isolated rat hepatocytes (Elaut et al. Tox Lett 144(S1): 78, 2003). 5-(4-dimethylaminobenzoyl)-aminovaleric acid hydroxamid may be brought in the form of derivatives, such as salts. As such derivatives may be used so long as they do not adversely affect the desired effects of the compound. Accordingly, in an embodiment of the present invention, the at least one histone deacetylase inhibitor is 5-(4-dimethylaminobenzoyl)-aminovaleric acid hydroxamid or a derivative thereof.

The histone deacetylase inhibitor(s) may be added to the media used to culture the stem cells. The histone deacetylase inhibitor(s) may be administered to the stem cells during and/or after treating of the cells with the differentiation agents. As a non-limiting example, it may be administered to the stem cells together with the last differentiation agent or combination of differentiation agents. In another example, it may be administered when the stem cells already show partially differentiated phenotype. The point and duration of administration of the histone deacetylase inhibitor(s) will need to be optimized for each differentiation protocol and each stem cell type, but non-limiting, illustrative examples, can be found in the Examples section of this specification.

In a further aspect, the present disclosure provides a differentiated cell or cells, obtainable using the method of the invention. Such cells may be in culture, such as, e.g., in suspension culture or in adherent culture. Such culture may be a short-term culture or a long-term culture. Such cells may also be frozen in an appropriate solvent, such as, for example, one comprising DMSO or glycerol, or may be processed, such as, for example, fixed and stained for use in microscopy or immunocytochemistry.

Such differentiated cell(s) may show one or more phenotypic features of any differentiated cell type of an organism, such as, for example, of a terminally differentiated, mature cell type of said organism. The differentiated cell(s) obtainable by the method of the invention may show one phenotypic feature of a given differentiated cell type of an organism, or may show more than one such phenotypic feature, for example two or three such features, or a plurality of such features. The term "phenotypic feature of a cell" refers to all the observable characteristics of a cell, such as, for example, its morphology, its interactions with other cells and with the extracellular matrix, the nature of proteins expressed on its surface (i.e. surface markers) or within the cell, or secreted by the cell, or the cell's behavior, e.g., secretion, contraction, synaptic transmission, etc. Such phenotypic features of various differentiated cell types of an organism are well-known to those skilled in the art. All cells, and thus all cell types, of an organism originate from one of the three embryonic germ layers, namely the endoderm, ectoderm and mesoderm. Accordingly, in an embodiment the present invention provides a differentiated cell or cells obtainable using the method of the invention, wherein said differentiated cell(s) show at least one phenotypic feature of a cell originating from the endoderm, ectoderm, or mesoderm.

Examples of tissues and organs derived from the endoderm comprise among others the thymus, thyroid, parathyroid glands, larynx, trachea, lung, urinary bladder, vagina, urethra gastrointestinal organs, such as liver and pancreas; lining of the gastrointestinal tract, or lining of the respiratory tract. Accordingly, examples of cell types derived from the en doderm comprise among others an epithelial cell, a hepatocyte, a gastrointestinal tract epithelial cell, a lung epithelial cell, or a kidney epithelial cell.

Examples of tissues and organs derived from the ectoderm comprise among others the skin, nervous system, including the brain, spinal cord and the peripheral nervous system, adrenal medulla, pituitary gland, connective tissue of the head and face, eyes, or ears. Accordingly, examples of cell types derived from the ectoderm comprise among others a neuronal cell, a glial cell, an astrocyte, an oligodendrocyte, a Schwan cell, or a keratinocyte.

Examples of tissues and organs derived from the mesoderm comprise among others the bone marrow, blood, adrenal cortex, lymphatic tissue, skeletal muscle, smooth muscle, and cardiac muscle, connective tissues, such as bone and cartilage, fat tissue, urogenital system, or the cariovascular system, including the heart and blood vessels. Accordingly, examples of cell types derived from the mesoderm comprise among others an osteoblast, a chondroblast, a chondrocyte, a fibroblast, an adipocyte, a skeletal muscle myoblast, a smooth muscle myoblast, a cardiac muscle myoblast, a hematopoietic cell and cells derived therefrom, a stromal cell, or an endothelial cell.

It is to be understood that the above examples of tissues, organs, and cell types derived from the three germ layers, i.e. endoderm, ectoderm, and mesoderm, are only mentioned to illustrate the present invention, and are in no way to be construed as limiting the scope of the invention, as any cell type from any tissue or organ of an organism may be produced by the method of the present invention.

It is another aspect of the present disclosure to provide differentiated cell(s) showing one or more phenotypic features of a hepatocyte. Hepatocytes are the most significant type of parenchymal cell in the liver, and hepatocytes perform most of the liver functions. The liver participates in many important physiological processes, including protein, lipid and carbohydrate metabolism, bile secretion, fibrinogen production and detoxification of a wide variety of foreign compounds and endogenous metabolites, including many therapeutic agents. By virtue of a portal circulatory system, the liver is the initial processing point for most materials absorbed through the gastrointestinal tract, and in this manner the liver protects the body from many harmful compounds. In drug development, great significance is attached to the nature of the interaction between a candidate therapeutic and the cells of the liver. Many candidate therapeutics are significantly hepatotoxic. In addition, the pharmacokinetics of a candidate therapeutic are heavily influenced by the metabolic activities of hepatocytes. Hence, *in vitro* assays for predicting the *in vivo* hepatotoxicity and pharmacokinetics of a candidate compound are an important part of the drug development process. The liver is vulnerable to many disorders. A variety of compounds can cause temporary or permanent liver failure as well as liver cell death. In addition, a variety of diseases, such as hepatitis, may result in liver damage. While these conditions are usually correctable with a liver transplant, the chronic shortage of donor organs places this method of treatment out of reach for many patients. For these and other reasons, cells showing phenotypical features of hepatocyte s produced by an *in vitro* differentiation method, such as the method of the present invention, would be invaluable. Accordingly, in an embodiment the present invention provides a differentiated cell or cells obtainable using the method of the invention, wherein said differentiated cell(s) show at least one phenotypic feature of a hepatocyte.

Such phenotypic features of a hepatocyte are well-known to a person skilled in the art, and may comprise among others the ability to use pyruvate as a sole carbon source; butyrate resistance at concentrations of at least about 1-20 mM and preferably at least about 5 mM sodium butyric acid; the ability to take up vinblastine; cytochrome P450 activity (e.g., dibenzylfluorescein metabolism, dextromethorphan oxidation, coumarin glucuronidation or sulfation); cytochrome P450 expression (detected, for example, by RT-PCR or antibody staining); or expression of other genes and/or proteins that are indicative of hepatocytes, such as albumin, alphafoetoprotein , gamma-glutyryltransferase, hepatocyte nuclear factor (HNF) 1alpha, HNF 1beta, HNF 3alpha, HNF 3beta, HNF 4, anti-trypsin, transthyretin, cytokeratin type 18 (CK-18) and CFTR. Said differentiated cell may show one phenotypic feature of a hepatocyte, or may show more than one such phenotypic feature, for example two or three such features, or a plurality of such features.

Differentiated cell or cells obtainable using the method of the invention showing at least one phenotypic feature of a hepatocyte may be used in several applications. Accordingly, in one embodiment the present disclosure provides differentiated cell or cells showing at least one phenotypic feature of a hepatocyte for use in artificial liver devices. Such devices, for example the Extracorporeal Liver Assist Device (ELAD) will be well-known to the person skilled in the art. Other uses for such cells are detailed elsewhere in this specification.

In a further embodiment, the present disclosure provides a differentiated cell or cells obtainable from mesenchymal stem cell(s) using the method of the invention, wherein said differentiated cell(s) show at least one phenotypic feature of a hepatocyte. Importantly, the discovery of the present invention that mesenchymal stem cells, which are of mesodermal origin, can be differentiated *in vitro* to hepatocytes or cells with phenotypic features of hepatocytes, which are of endodermal origin, is surprising and novel, and contradicts the currently prevailing opinion about the differentiation potential of MSC.

In another embodiment, the present disclosure provides a differentiated cell or cells obtainable from multipotent adult progenitor cell(s) using the method of the invention, wherein said differentiated cell(s) show at least one phenotypic feature of a hepatocyte. Although the differentiation of MAPC into hepatocytes has been achieved previously (Schwartz et al. J Clin Invest 109:1291-1302, 2002), the present invention provides a significant improvement over this prior art, as the differentiation of MAPC to hepatocytes accomplished by the method of the present invention is considerably more profound. This point is further illustrated in the accompanying Examples.

It is an aspect of the present disclosure to provide differentiated cells for use in trans plantation. Due to the growing demand for life-saving organ replacements and the recognized shortage of donor organs, tissue and organ generation from stem cells and their subsequent transplantation may provide promising treatments for a number of pathologies, such as, for example, Alzheimer's disease, Parkinson's disease, Huntington's disease, liver diseases, diabetes, cardiovascular diseases, and autoimmune disorders.

Several instances of tissue engineering have been described previously. For example, Oberpenning et al. (Nat Biotechnol 17: 149-155, 1999) reported the formation of a working bladder by culturing muscle cells from the exterior canine bladder and lining cells from the interior of the canine bladder, preparing sheets of tissue from these cultures, and coating a small polymer sphere with muscle cells on the outside and lining cells on the inside. The sphere was then inserted into a dog's urinary system, where it began to function as a bladder. For example, Nicklason et al (Science 284: 489-493, 1999) reported the production of lengths of vascular graft material from cultured smooth muscle and endothelial cells. Other examples of tissue engineering using cultured cells will be known to those of skill in the art. The above and other tissue engineering methods may be particularly efficient if used in combination with the method of stem cell differentiation provided by the present invention, and in combination with differentiated cells provided by this method.

Depending on the particular therapeutic application, the method of the present invention may be used to induce stem cell(s), by means of example but not limitation, to generate skin epithelial cells for skin grafting and plastic surgery; to generate myoblasts or myocytes for enhancement of muscle bulk; to generate hematopoietic cells or blood cells to produce blood *ex vivo;* to generate cardiomyocytes for use in the treatment of cardiac diseases, such as, e.g., myocarditis, cardiomyopathy, heart failure, damage caused by heart attacks, hypertension, atherosclorosis, or heart valve dysfunction; to generate neuronal cells for the treatment of CNS disorders, such as, e.g., neurodegenerative disorders, including among others Alzheimer's disease, Parkinson's disease and Huntington's disease, or CNS dam age, such as, e.g., resulting from stroke or spinal cord injury; to generate glial cell, including Schwann cells, for the treatment of demyelinating conditions of CNS and PNS; to generate chondrocytes to treat diseases of the joints or cartilage, such as, e.g., cartilage tears, cartilage thinning, or osteoarthritis; to generate osteoblasts to treat bone disorders, such as, e.g., bone fractures, non-healing fractures, or osteoarthritis. In a very useful example, the stem cell(s) may be stimulated to generate hepatocytes for the treatment of liver disease or liver damage, such as, e.g., hepatitis, or liver cirrhosis.

Differentiated cells for use in transplantation may be preferably generated from a patient's own stem cells. Alternatively, such cells may also be generated from heterologous stem cells, such as from ES cells or EG cells. In the latter case, such heterologous stem cells may be genetically altered to minimize the risk of immunological rejection of the transplanted cells by the patient's body, or alternatively, transplant rejection may be prevented by administration of, e.g., chemotherapeutic immune suppressors to the patient.

Regardless of the origin of stem cells used for the production of transplantable differentiated cells, and tissues or organs comprising such differentiated cells, the method of stem cell differentiation provided by the present invention may offer a source of such differentiated cells. Accordingly, in an embodiment, the present invention provides a differentiated cell or cells for use in transplantation.

It is another aspect of the present disclosure to provide differentiated cells for use in gene therapy. The general principles, methods and potential applications of gene therapy will be well-known to a person skilled in the art. The method of the present invention may be particularly useful in *ex vivo* therapy. Here, stem cell(s), either heterologous stem cells or stem cells obtained from a patient in need of the gene therapy, will be maintained *in vitro* and their genetic material will be altered by one of the methods available in the art for this purpose. The required nature of the genetic alteration will correspond to the desired therapeutic effect, and will be obvious to the skilled person. By means of example and not limitation, such genetic alteration may introduce a functional copy of an otherwise defective gene, or may cause down-regulation of expression of a particular gene, e.g., by RNA interference, or may lead to expression of a protein normally not present in the cell, such as, e.g., for the purposes of vaccination. The stem cell(s) carrying the desired genetic alteration will be selected, optionally propagated, and differentiated *in vitro* into a desired cell type, and subsequently introduced, e.g., transplanted or injected, into the patient's body. Accordingly, in an embodiment, the present invention provides a differentiated cell or cells for use in gene therapy.

It is another aspect of the present disclosure to provide differentiated cells for use in assays of toxicity. The use of differentiated cells may be preferred in certain assays of toxicity, as such cells more closely resemble the cell types present in the tissues and organs of an organism. These differentiated cells will be very useful in assays of toxicity performed *in vitro,* i.e., using cultured cells or suspensions of cells. Such *in vitro* assays examine the toxicity to cultured cells or suspended cells of compounds or compositions, e.g., chemical, pharmaceutical or biological compounds or compositions, or biological agents. In this context, a particular compound or composition may be considered toxic or likely toxic, if it shows a detrimental effect on the viability of cells or on one or more aspect of cellular metabolism or function. Typically, the viability of cells *in vitro* may be measured using colorimetric assays, such as, e.g., the MTT (or MTT derivative) assays or LDH leakage assays, or using fluorescence-based assays, such as, e.g., the Live/Dead assay, CyQuant cell proliferation assay, or assays of apoptosis. Other assays may measure particular aspects of cellular metabolism or function. While the above are non-limiting examples, a person skilled in the art will be able to make an appropriate choice of assay of toxicity to use in combination with the differentiated cells provided by the present invention, and will be knowledgeable of the technical requirements to perform such assay. Accordingly, in an embodiment, the present invention provides a differentiated cell or cells for use in assays of toxicity. In another embodiment, the present invention provides a differentiated cell or cells of human or animal origin for use in assays of toxicity. The use of human cells in assays of toxicity will provide a relevant reference for the potential toxicity of chemical compounds, compositions, or agents on the cell types present in human or animal tissues or organs, respectively. Moreover, because such chemical compounds or compositions may also be comprised in a sample obtainable from the environment, in one embodiment the present invention also provides differentiated cells for use in assays of ecological toxicity.

It is another aspect of the present disclosure to provide differentiated cells for use in assays of carcinogenicity. The use of differentiated cells may be preferred in certain assays of carcinogenicity, as such cells more closely resemble the cell types present in the tissues and organs of an organism. These differentiated cells will be very useful in assays of both genotoxic and non-genotoxic (i.e., epigenetic) carcinogenicity. These differentiated cells will be very useful in assays of carcinogenicity performed *in vitro,* i.e., using cultured cells or suspensions of cells. Such *in vitro* assays examine the carcinogenicity to cultured cells or suspensions of cells of compounds or compositions, e.g., chemical, pharmaceutical or biological compounds or compositions, or biological agents. In this context, a particular compound or composition may be considered carcinogenic or likely carcinogenic, if it induces neoplastic transformation of the cells, or induces phenotypic changes in the cells that may be predictive of such neoplastic transformation, or induces genetic or metabolic changes that may potentially cause such neoplastic transformation. Such phenotypic changes in the cells may, by means of example but not limitation, comprise morphological transformation , increased proliferation, dedifferentiation, independence of attachment, removal of contact inhibition of cells grown in monolayers, or expression of specific marker proteins. Such genetic changes in the cells may, by means of example but not limitation, comprise DNA damage, chromosomal aberrations, such as chromosomal rearrangements, alterations in chromosome number (aneuploidy), or karyotype aberrations, gene mutations, such as point mutations, deletions or insertions. Compounds or compositions that cause this kind of genetic changes are often referred to as mutagenic or mutagens. Accordingly, the cells provided by the present invention will be very useful in assays of mutagens, i.e., in assays of mutagenicity. For the purposes of mutagenicity testing, the cells of the present invention may but need not be genetically altered. For example; the cells may comprise a transgene, the product of which increases their sensitivity to a particular proliferation-inhibiting agent. Consequently, genetic alterations in some cells removing the expression of such transgene would release these cells from this inhibition. Mutagenicity may then be assessed by methods of scoring such cells. A person skilled in the art will be able to make an appropriate choice of assay of carcinogenicity to use in combination with the differentiated cells provided by the present invention, and will be knowledgeable of the technical requirements to perform such assay. Accordingly, in an embodiment, the present invention provides a differentiated cell or cells for use in assays of carcinogenicity. In another embodiment, the present invention provides a differentiated cell or cells of human or animal origin for use in assays of carcinogenicity. The use of human cells in assays of carcinogenicity will provide a relevant reference for the potential carcinogenicity of chemical compounds, compositions, or agents on the cell types present in human tissues or organs. Moreover, because such chemical compounds or compositions may also be comprised in a sample obtainable from the environment, in one embodiment the present invention also provides differentiated cells for use in assays of ecological carcinogenicity and ecological mutagenicity.

It is another aspect of of the present disclosure to provide differentiated cells having the phenotypical features of hepatocytes for use in assays of toxicity. As detailed elsewhere in this specification, many candidate therapeutics are significantly hepatotoxic. Therefore, in drug development, great significance is attached to the potential toxicity a candidate therapeutic may have to hepatocytes. Hence, cells showing phenotypical features of hepatocytes produced by the *in vitro* differentiation method of the present invention, will be invaluable in assays of toxicity, as the outcomes of such assays may prove highly predictive of the toxicity of various compounds or compositions, e.g., chemical, pharmaceutical or biological compounds or compositions, or biological agents to hepatocytes *in vivo*. It is detailed elsewhere in this specification what is meant by "a cell showing at least one phenotypical feature of a hepatocyte". The differentiated cells of the present invention will be very useful in assays of toxicity performed *in vitro,* i.e., using cultured cells or suspensions of cells. The details of *in vitro* assays of toxicity are provided elsewhere in this specification. A particular compound or composition may be considered toxic or likely toxic to hepatocytes, if it shows a detrimental effect on the viability of cells provided by the present invention having phenotypic features of hepatocytes or on one or more aspect of cellular metabolism, or function of such cells. Accordingly, in an embodiment, the present invention provides a differentiated cell or cells showing at least one phenotypic attribute of a hepatocyte for use in assays of toxicity. In another embodiment, the present invention provides a differentiated cell or cells of human or animal origin showing at least one phenotypic attribute of a hepatocyte for use in assays of toxicity. The use of human cells in assays of toxicity will provide a relevant reference for the potential toxicity of chemical compounds, compositions, or agents on the cell types present in human tissues or organs.

It is another aspect of the present disclosure to provide differentiated cells having the phenotypic features of hepatocytes for use in assays of biotransformation. The pharmacokinetics of a candidate therapeutic belongs to factors determining its usefulness. For example, low metabolic stability may result in poor availability and high clearance of such candidate therapeutic. Moreover, while in most cases biotransformation of drugs results in bio-inactivation, it is also possible that pharmacologically active, reactive, or toxic intermediates and metabolites are produced. Therefore, the biotransformation of a new chemical entity, such as a drug or a candidate therapeutic, needs to be investigated as early in the drug discovery process as possible. As detailed elsewhere in this specification, the liver represents the major site of xenobiotic biotransformation and/or detoxification of a wide variety of foreign compounds, including many therapeutic agents, and the pharmacokinetics of a candidate therapeutic are heavily influenced by their biotransformation in the liver. Hepatocyte cultures represent one *in vitro* system capable of integrated biotransformation. Freshly isolated hepatocytes exhibit biotransformation capacities close to those observed *in vivo,* but can only be used in *in vitro* assays of biotransformation for about 5-6 hours. Monolayer cultures of primary hepatocytes may be used in such assays for about 2-4 days. Cells showing phenotypical features of hepatocytes produced by the *in vitro* differentiation method of the present invention, may provide an alternative assay system to study the biotransformation of various compounds or compositions, e.g., chemical, pharmaceutical or biological compounds or compositions, as the outcomes of such assays may prove highly predictive of the biotransformation of the respective various compounds or compositions by primary hepatocytes *in vivo* and/or *in vitro.* It is detailed elsewhere in this specification what is meant by "a cell showing at least one phenotypical feature of a hepatocyte". Within relation to biotransformation, such differentiated cell will ideally have a biotransformation capacity, i.e., either phase I or phase II, or both phase I and phase II biotransformation capacities, sufficiently comparable to the hepatocytes *in vivo* or to freshly isolated primary hepatocytes. The differentiated cells of the present disclosure will be very useful in assays of biotransformation performed *in vitro,* i.e., using cultured cells or suspensions of cells. Accordingly, in an aspect, the present disclosure provides a differentiated cell or cells showing at least one phenotypic attribute of a hepatocyte for use in assays of biotransformation. In another aspect, the present disclosure provides a differentiated cell or cells of human or animal origin showing at least one phenotypic attribute of a hepatocyte for use in assays of biotransformation. The use of human cells in assays of biotransformation will provide a relevant reference for biotransformation of chemical compounds, compositions, or agents by human liver.

In a further aspect, the present disclosure provides a biochemical extract of differentiated cell or cells produced by the method of the present invention. Such extract may primarily contain, by means of example but not limitation, the cytosolic fraction, the nuclear fraction, the cytoplasmic membrane fraction, the nuclear membrane fraction, the microsomal fraction, the mitochondrial fraction, the endoplasmic reticuluum fraction, the Golgi apparatus fraction, the lysosomal fraction, the cytoskeletal fraction, etc. Such extract may comprise cellular proteins, wherein the function of said proteins may or may not be preserved in said extract. Depending on the desired application for the biochemical extract, the choice of the appropriate extracted fraction, as well as the methods suitable to obtain that fraction will be known to the one skilled in the art.

In a further aspect, the present disclosure provides a biochemical extract of differentiated cell or cells showing at least one phenotypic attribute of a hepatocyte produced by the method of the present invention. Such extract may primarily contain, by means of example but not limitation, the cytosolic fraction, the nuclear fraction, the cytoplasmic membrane fraction, the nuclear membrane fraction, the microsomal fraction, the mitochondrial fraction, the endoplasmic reticul uum fraction, the Golgi apparatus fraction, the lysosomal fraction, the cytoskeletal fraction, etc. Such extract may comprise cellular proteins, wherein the function of said proteins may but need not be preserved in said extract. Depending on the desired application for the biochemical extract, the choice of the appropriate extracted fraction, as well as the methods suitable to obtain that fraction will be known to the one skilled in the art. One preferred application for the extract may be its use in in vitro biotransformation assays.

In a further aspect, the present disclosure provides a microsomal extract of differentiated cell or cells showing at least one phenotypic attribute of a hepatocyte produced by the method of the present invention. Microsomal extracts or suspensions will contain the smooth" endoplasmic reticuluum of the differentiated cells and may be used in biotransformation assays to evaluate, for example, phase I oxidation reactions, cytochrome P450 (CYP)-dependent inhibitory drug-drug interactions and the importance of genetic polymorphisms in biotransformation. Accordingly, in a further aspect the present disclosure provides the microsomal extract of differentiated cell or cells showing at least one phenotypic attribute of a hepatocyte for use in assays of biotransformation. Methods to obtain microsomal extracts or suspensions for use in biotransformation assays, and methods of carrying out biotransformation assays using such extracts are well known to the person skilled in the art.

In another aspect the present disclosure provides differentiated cells for use in detection of differentiation-inducing agents. In another aspect the present disclosure provides differentiated cells for use in creation of copy DNA libraries. In another aspect the present disclosure provides differentiated cells for use in production of antibodies. In another aspect the present disclosure provides differentiated cells for use in transfections with exogenous DNA. In another aspect the present disclosure provides differentiated cells for cryopreservation.

In another embodiment, the present invention provides a kit for use in producing differentiated cells from stem cells *in vitro* according to the method of the present invention. In the kit will be included four or more containers, each comprising one or more differentiation agents. These differentiation agents may be present as lyophilized powder or in a solution with an appropriate solvent. Also in the kit will be included at least one container comprising at least one histone deacetylase inhibitor, such as, for example, trichostatin A or 5-(4-dimethylaminobenzoyl)-aminovaleric acid hyd roxamid, or a derivative thereof. This histone deacetylase inhibitor may be present as lyophilized powder or in a solution with an appropriate solvent. Also in the kit may be included, optionally, at least one container comprising a particular type of stem cells. The stem cells may be present in a liquid suspension, but more likely will be present as frozen stock in an appropriate solvent, such as one containing DMSO or glycerol. Also in the kit will be included information for the user with at least one optimized manner of administering the components of said kit to the stem cells to produce differentiated cells. By following this information, the user will be able to produce such differentiated cells.

In another aspect the present disclosure provides a method for stabilization of the phenotype of isolated primary cells *in vitro,* comprising the steps of:
(i) obtaining a tissue or organ from an organism;
(ii) optionally, perfusing the tissue or organ with a first composition comprising at least one histone deacetylase inhibitor;
(iii) isolating at least one primary cell from the tissue or organ; and
(iv) culturing *in vitro* said at least one primary cell in the presence of another composition comprising at least one histone deacetylase inhibitor.

A tissue or organ may be the whole tissue or an organ, or only a portion thereof. Such tissue or organ may be obtained, for example, by surgery, dissection, or by a biopsy.

"Perfusing" a tissue or organ with a composition involves exposing the tissue or organ to the composition upon isolation. The tissue or organ may, for example, be submerged in the composition, or the composition may be introduced into the blood vessels supplying the tissue or organ. Within this context, such composition will likely be a liquid composition, such as a solution of various components in a liquid solvent.

The first and the another composition used in the method for stabilization of the phenotype of isolated primary cells *in vitro* both comprise at least one inhibitor of histone deacetylases. The function and types of histone deacetylases a re detailed elsewhere in this specification, as are the properties and functions of HDAC inhibitors.

The histone deacetylase inhibitor(s) comprised in the first and in the another composition may be identical, or may also be different. The histone deacetylase inhibitor(s) comprised in the first and in the another composition may preferably be specific HDAC inhibitors, i.e., agents that, while inhibiting the activity of at least one HDAC, do not substantially effect other enzyme systems, and wherein the effect(s) on downstream cellular components and functions are a direct consequence of those agents. In an embodiment, one of the at least one histone deacetylase inhibitor comprised in the first and/or in the another composition will be chosen from HDAC inhibitors falling into one of the structural types comprising benzamides (e.g., MS-27-275, CI-994), sulfonamide-based anilides, straight chain anilides, heterocyclic ketones (e.g., apicidin and its derivatives, FR235222), allyl sulfur compounds (e.g., diallyl sulfide), psammaplins (e.g., psammaplin A), heterocyclic thiols (e.g., depsipeptide, spiruchostatin A), sulfur-containing cyclic peptides, bromoacetamide-based straight chain inhibitors, short-chain fatty hydroxamic acids (e.g., sodium butyrate), pivaloyloxymethyl butyrate, tributyrin, phenylalkanoic acids, valproic acid, phenylbutyrate, N-acetylcystein sulforaphane, cystein sulforaphane, straight chain trifluoromethylketones, alpha-ketoamides, alpha-ketoesters, heterocyclic ketones, cyclic trifluoromethylketo nes, cyclic pentafluoromethylketones, heterocyclic epoxides (e.g., trapoxin A), depudecin, short-chain fatty hydroxamic acids, trichostatins (e.g., trichostatin A), hybrid polar compound (e.g., suberoyl anilide hydroxamic acid, pyroxamide), benzamide-based hydroxamic acids (e.g., 5-(4-dimethylaminobenzoyl)aminocaproic acid hydroxamide, 5-(4-dimethylaminobenzoyl)-aminovaleric acid hydroxamid), arylketones, amino acid-containing benzamide based hydroxamic acids, indole amide-based hydroxamic acids, aryl-heterocyclic-based hydroxamic acids, aryl-pentadienoic hydroxamic acids, sulfonamide-based hydroxamic acids (e.g., oxamflatin, PXD101), (bi-)aryl-(heterocyclic)-based hydroxamic acids (e.g., SK-7041), NVP-LAQ824, A-161906, aroyl-pyrrole-hydroxyamides, cyclic hydroxamic acid-containing peptides, cyclic hexapeptides, succinimide hydroxamic acids, cystein-based hydroxamic acids, 1,3-dioxanes (e.g., tubacin), phosphorus-based straight chain inhibitors, cyclostellettami nes (e.g., cyclic 3-alkyl pyridinium dimers), straight-chain retrohydroxamates, cyclic retrohydroxamates, semicarbazide-based straight chain inhibitors, and thiol-based straight chain inhibitors. However, it will be appreciated that all presently known HDAC inhibitors, whether falling into one of the above categories or not, may be used in the method for stabilization of the phenotype of isolated primary cells *in vitro* according to the present invention. Also, novel HDAC inhibitors identified in the future, will be capable of being used in the method according to the present invention. In addition, if any HDAC inhibitor needs to be used as a derivative, e.g., as a salt, in order to exert the maximal effect in solution, in cells, or in an organism, such derivatives may be used in the method.

The properties and uses of the HDAC inhibitor trichostatin A and its derivatives, such as salts, as well as of semi-synthetic and synthetic analogues of trichostatin A and their derivatives, such as salts, as well as of amide based analogues of trichostatin A and their derivatives, such as salts, are detailed elsewhere in this specification. In an embodiment, one of the at least one histone deacetylase inhibitor comprised in the first and/or in the another composition is trichostatin A or a derivative thereof. In another embodiment, one of the at least one histone deacetylase inhibitor comprised in the first and/or in the another composition is a semi-synthetic or a synthetic analogue of trichostatin A or a derivative of such an analogue. In another embodiment, one of the at least one histone deacetylase inhibitor comprised in the first and/or in the another composition is an amide based analogue of trichostatin A or a derivative of such an analogue.

The properties and uses of the HDAC inhibitor 5-(4-dimethylaminobenzoyl)-aminovaleric acid hydroxamid are detailed elsewhere in this specification. In an embodiment, one of the at least one histone deacetylase inhibitor comprised in the first and/or in the another composition is 5-(4-dimethylaminobenzoyl)-aminovaleric acid hydroxamid or a derivative thereof.

The first and/or the another composition used in the method for stabilization of the phenotype of isolated primary cells *in vitro* according to the present disclosure may also comprise other agents that can increase the viability of the isolated primary cells. For example, such agents comprised in the first composition, i.e., in the composition used for perfusion, may, by means of example and not limitation, reduce ischemic stress to the isolated tissue or organ, reduce the formation of reactive oxygen species (ROS) in the isolated tissue or organ; reduce the extent of apoptosis in the isolated tissue or organ; or suppress the tissue generation response (i.e., prevent quiescent from initiating cell division) in the isolated tissue or organ. Accordingly, in an embodiment, the first and/or the another composition for use in the method for stabilization of the phenotype of isolated primary cells *in vitro* comprises at least one agent promoting the viability of the primary cells. In another embodiment, such agent(s) may be chosen from the group comprising inhibitors of macrophage activation, inhibitors of TNF alpha, caspase inhibitors, inhibitors of pro-apoptotic proteins, inhibitors of the opening of mitochondrial transition pore, antioxidants and scavengers of reactive oxygen species, precursors of glutathione, prostaglandins, donors of NO, inhibitors of inducible nitrogen oxide synthase, and inhibitors of DNA methyl transfe rase.

As detailed elsewhere in the present specification, suspensions and monolayers of primary hepatocytes, e.g., primary rat hepatocytes, provide an important tool to study *in vitro* the hepatotoxicity and biotransformation of, e.g., chemical compounds and candidate therapeutics. However, the time frame allowed for such *in vitro* tests is limited, as suspensions of primary hepatocytes may be used for only about 5 to 6 hours and monolayers of primary hepatocytes for about 2 to 4 days, after which the cells gradually loose their liver-specific properties. This precludes the use of su ch primary cultures for, e.g., long-term studies of toxicity or carcinogenecity. Stabilization of the liver-specific phenotype of cultured primary hepatocytes for prolonged time intervals would, however, facilitate such long-term assays in cultured primary hepatocytes. Accordingly, in an aspect, the present disclosure provides a method for preserving the phenotype of isolated primary cells *in vitro,* wherein said cells are primary hepatocytes isolated from the liver.

In a further aspect, the present disclosure provides a method for stabilization of the phenotype of isolated primary cells *in vitro,* wherein the primary cells are isolated from an animal. Such animal may be, for example, a mouse, rat, guinea pig, hamster, rabbit, chicken, cat, dog, a non-human primate, pig, sheep, cow, horse, fish, reptile, or amphibian.

In another aspect, the present disclosure provides a method for stabilization of the phenotype of isolated primary cells *in vitro,* wherein the primary cells are isolated from human.

In one aspect, the present disclosure provides a composition optimized for use in the method for stabilization of the phenotype of isolated primary cells *in vitro* according to the invention as the first composition, i.e., the co mposition used to perfuse the isolated tissue or organ.

In another aspect, the present disclosure provides a composition optimized for use in the method for stabilization of the phenotype of isolated primary cells *in vitro* according to the invention as the another composition, i.e., the composition used in subsequent culturing of the primary cells. Such composition may be added to the culture medium used to culture the primary cells.

In yet another aspect, the present disclosure provides a kit comprising compositions optimized for use in the method for stabilization of the phenotype of isolated primary cells *in vitro*. Such kit may contain one or more different variants of each composition.

In another aspect, the present disclosure further provides primary cell or cells obtainable using the method for stabilization of the phenotype of isolated primary cells *in vitro.* In yet another aspect, the present disclosure provides primary hepatocyte(s) obtainable using the method for stabilization of the phenotype of isolated primary cells *in vitro.*

In an aspect the present disclosure provides primary cell or cells obtainable using the method for stabilization of the phenotype of isolated primary cells *in vitro* for use in assays of toxicity. By virtue of their more stable phenotype, the primary cells provided by the present invention may be particularly suitable for long-term assays of toxicity. The use of primary cells may be preferred in certain assays of toxicity, as such cells more closely resemble the cell types present in the tissues and organs of an organism. These primary cells will be very useful in assays of toxicity performed *in vitro,* i.e., using cultured cells or suspensions of cells. The principles and practical aspects of *in vitro* assays of toxicity have been detailed elsewhere in this specification for differentiated stem cells, and these principles and practical aspects may in essence be adopted to toxicity assays in cultures of primary cells.

In another aspect, the present disclosure provides primary cell or cells obtainable using the method for stabilization of the phenotype of isolated primary cells *in vitro* for use in assays of carcinogenicity. By virtue of their more stable phenotype, the primary cells provided by the present invention may be suitable for long-term assays of carcinogenicity. The use of primary cells may be preferred in certain assays of carcinogenicity, as such cells more closely resemble the cell types present in the tissues and organs of an organism. These primary cells will be very useful in assays of carcinogenicity performed *in vitro,* i.e., using cultured cells or suspensions of cells. The principles and practical aspects of *in vitro* assays of carcinogenicity have been detailed elsewhere in this specification for differentiated stem cells, and these principles and practical aspects may in essence be adopted to carcinogenicity assays in cultures of primary cells.

In another aspect, the present disclosure provides primary hepatocytes obtainable using the method for stabilization of the phenotype of isolated primary cells *in vitro* for use in assays of biotransformation. By virtue of their more stable phenotype, the primary hepatocytes provided by the present invention may be suitable for long-term assays of hepatotoxicity and biotransformation. The use of primary hepatocytes may be preferred in assays of hepatotoxicity and biotransformation, as such hepatocytes more closely resemble the situation in a liver. These primary hepatocytes will be very useful in assays of hepatotoxicity and biotransformation performed *in vitro,* i.e., using cultured cells or suspensions of cells. The importance, principles and practical aspects of *in vitro* assays of hepatotoxicity and biotransformation have been detailed elsewhere in this specification for stem cells differentiated to form hepatocytes or hepatocyte-like cells, and these principles and practical aspects may in essence be adopted to hepatotoxicity and biotransformation assays in primary cultures of hepatocytes.

In another aspect, the present disclosure provides a biochemical extract of primary cells obtainable using the method for stabilization of the phenotype of isolated primary cells *in vitro.* The different types of biochemical extracts and the corresponding cellular fractions contained within these have been detailed elsewhere in this specification for *in vitro* differentiated stem cells and comparable extracts may be obtained from cultured primary cells. Particularly useful extracts may be the cytosolic and microsomal extracts of primary hepatocytes obtained using the method according to the invention. Such extract may be used in assays of biotransformation.

Further aspects of the invention are detailed below in the appended Examples. The Examples serve to illustrate the invention and are in no case to be construed as limiting the scope of the invention. If a term or an abbreviation is included in the Examples that is not further defined within the present specification, or that cannot be interpreted based on its context, then it should be construed to have the same meaning as it is understood by those skilled in the art.

### Examples

### EXAMPLE 1: In vitro differentiation of bone marrow stem cells (BMSC) into hepatocyte-like and neuroectodermal-like cells using sequential exposure to differentiation agents and HDAC inhibitor.

### Materials and Methods

### Isolation and cultivation of undifferentiated rat BMSC

Isolated BMSC from male Sprague Dawley rats (4-6 weeks old) were selected and cultivated essentially as described by Reyes et al. (Blood 98: 2615-2625, 2001).

### Differentiation of rat BMSC

Rat BMSC from 60 population doublings on were used for differentiation into hepatocyte- and neuroectodermal-like cells.

### Hepatocyte differentiation

BMSC were, after splitting, plated at 100% confluence on 10ng/ml FN (Sigma), 1mg/ml collagen type I or 1% matrigel (B&D) matrix in serum-low expansion medium as described by Schwartz et al. (J Clin Invest 109:1291-1302, 2002). After 20-24h, medium was removed and cells were washed 3 times with basal medium containing 60% DMEM-LG / 40% MCDB-201 supplemented with 1mg/ml LA-BSA, 100 IU/ml penicillin, 100µg/ml streptomycin, 0.1mM L-ascorbic acid, 4µg/ml nicotinamide, 27.3mg/l sodium-pyruvate and 1.623mM glutamin (all from Sigma).

Then, cells were cultivated in the presence of liver-specific (LSP) cytokines either as a cocktail [basal medium + 10ng/ml FGF-4 (fibroblast growth factor type 4), 20ng/ml HGF (hepatic growth factor) (all from R&D), 1 x ITS (insulin, transferrin, selenious acid) and 0.05µM Dex (dexamethasone) (all from Sigma)] or separately (until day 3: basal medium + 10ng/ml FGF-4; from days 3 to 6: basal medium + 20ng/ml HGF; from day 6 on: basal medium + 20ng/ml HGF + 1x ITS and 0.05µM Dex). From day 6 on, 1, 5 and 25µM TSA (Sigma) was added. The culture media were changed every three days.

### Neuroectodermal differentiation

BMSC were, after splitting, plated at 30% confluence on 10ng/ml FN matrix in serum-low expansion medium. After 20-24h, medium was removed and cells were washed 3 times with basal medium containing 60% DMEM-LG / 40% MCDB-201 supplemented with 1 mg/ml LA-BSA, 100 IU/ml penicillin, 100µg/ml streptomycin, 0.1mM L-ascorbic acid. Then, cells were cultivated in the presence of neuronal-specific cytokines either as a cocktail [basal medium + 100ng/ml bFGF (basic fibroblast growth factor), 10ng/ml FGF-8 (fibroblast growth factor type 8) (all from R&D)] or separately [until day 3: basal medium + 100ng/ml bFGF; from day 3: basal medium + 10ng/ml FGF-8]. The media were changed every three days.

### Total RNA isolation and quantitative RT-PCR

At different time intervals during culture, RNA was isolated using RNeasy mini kit (Qiagen) according to the manufacturer's recommendation and treated with DNase to remove contaminating DNA (DNA free Ambion). For PCR analysis, 0.5-1ug RNA was transcribed to cDNA with Superscript II reverse transcriptase and random hexamer primers (SuperScript First-Strand Synthesis; Invitrogen). The resulting RT-products were amplified as follows: 40 cycles (95°C for 15s, 60°C for 60s, following 3 extra steps to qualify the amplified product: 95°C for 15s, 60°C for 20s and 95°C for 15s) after an initial denaturation (95°C for 10min). For the PCR-reaction 1µl cDNA, 1 x TaqMan SYBR green Master Mix (Applied Biosystems) and empirical determined concentrations of sense and antisense primers were used. De quantitative analysis was done with ABI PRISM 7700 (Perkin Elmer). The primer pairs used for amplification of specific gene transcripts and the expected amplification products are listed in Table 1. The RNA levels were normalized using the house keeping gene 18-S and compared with RNA levels in rat adult liver (positive control) and undifferentiated BMSC (negative control). A no template cDNA-PCR reaction under the same conditions was also run as a negative control for the primers. The authenticity and size of PCR products were confirmed by melting curve analysis (using software provided by Perkin Elmer) and gel analysis.

**Table 1. Primers pairs used for quantitative RT-PCR and expected size (in base pairs) of the amplification products.**

| **Amplified transcript** | **Product size** | **Primer pair** |
|---|---|---|
| *Hepatocyte differentiation* | | |
| HNF-1 (hepatocyte nuclear factor type 1) | 138 | 5'-AGCTGCTCCTCCATCATCAGA-3' 5'-TGTTCCAAGCATTAAGTTTTCTATTCTAA-3' |
| HNF-3β | 68 | 5'-CCTACTCGTACATCTCGCTCATCA-3' 5'-CGCTCAGCGTCAGCATCTT-3' |
| ALB (albumin) | 141 | 5'-CTGGGAGTGTGCAGATATCAGAGT-3' 5'-GAGAAGGTCACCAAGTGCTGTAGT-3' |
| AFP (alphafoetoprotein) | 145 | 5'-GTCCTTTCTTCCTCCTGGAGAT-3' 5'-CTGTCACTGCTGATTTCTCTGG-3' |
| CK-18 (cytokeratin type 18) | 69 | 5'-GCCCTGGACTCCAGCAACT-3' 5'-ACTTTGCCATCCACGACCTT-3' |

| *Neuro-ectodermal differentiation* | | |
|---|---|---|
| Sox-1 | 51 | 5'-AAGATGCACAACTCGGAGATCAG-3' 5'-TFTAATCCGGGTGTTCCTTCAT-3' |
| Nestin | 113 | 5'-GAGAAGACAGTGAGGCAGATGAGTTA-3' 5'-GCCTCTGTTCTCCAGCTTGCT-3' |
| TH | 100 | 5'-AGTTCTCCCAGGACATTGGACTT-3' 5'-ACACAGCCCAAACTCCACAGT-3' |
| GFAP (glial fibrillary acidic protein) | 165 | 5'-GAGGAGTGGTATCGGTCTAAGTTTG-3' 5'-GCCGCTCTAGGGACTCGTT-3' |
| MBP (myelin basic protein) | 153 | 5'-GTGCAGCTTGTTCGACTCCG-3' 5'ATGCTCTCTGGCTCCTTGGC-3' |

### Immunofluorescence

At different time intervals during cell culture, differentiated BMSC were fixed respectively with MeOH for 1min at -20°C (cytoskeletal proteins) or with 4% paraformaldehyde (PFA, Electron Microscopy Sciences) for 10min at room temperature, respectively, followed by incubation with 100mM glycine to saturate reactive groups (nuclear and cytoplasmic markers). The fixed cells were then permeabilized for 15min with 0.1 % Triton (Electron Microscopy Sciences) and blocked for 30min with 1% BSA/5% donkey serum block buffer in PBS at room temperature. After blocking, cells were incubated overnight at 4°C with a primary antibody (fluorochrome labeled or unlabeled) and washed 3 times with PBS. Unlabeled cells were next incubated for 2h at room temperature with a secondary fluorochrome labeled antibody. After incubation, slides were washed again with 0.1% Triton in PBS and finally mounted with a mix of DAKO Fluorescence mounting medium and Vectashield with DAPI (29/1). DAPI was added to visualize the nuclei. As a negative control, cells were incubated with gamma globulines (IgG) (in the same concentration as the respective primary antibody) and immunostained in the same conditions. Cells were analyzed using a fluorescence microscopy with a Zeiss Axiovert scope. The antibodies used are listed in Table 2.

**Table 2. Overview of primary and secondary antibodies used, and gamma globulins used as negative control.**

| **Primary antibodies (hepatocyte differentiation)** | | **specification** | **type** | **species** | **dilution** | **fixation** |
|---|---|---|---|---|---|---|
| AFP | | Santa Cruz; sc-8108 | polyclonal | goat | 1/200 | PFA |
| HNF-1 | | Santa Cruz; sc-8986 | polyclonal | rabbit | 1/125 | PFA |
| HNF-3β | | Santa Cruz; sc-9187 | polyclonal | goat | 1/100 | PFA |
| CK 18-FITC | | Sigma F4772 | monoclonal | mouse | 1/100 | MeOH |
| ALB-FITC | | Bethyl Laboratories A90-234F | polyclonal | goat | 1/250 | PFA |

| **Primary antibody (neuronal differentiation)** | | | | | | |
|---|---|---|---|---|---|---|
| NF-200 | | Sigma N-5389 | monoclonal | mouse | 1/80 | PFA |
| | | | | | | |

| **Secondary antibodies** | | **specification** | | | **species** | **dilution** |
|---|---|---|---|---|---|---|
| anti-mouse IgG (H+L)-FITC | | Jackson Immunoresearch 715-095-150 | | | donkey | 1/500 |
| anti-mouse IgG (H+L)-Cy-3 | | Jackson Immunoresearch 715-165-150 | | | donkey | 1/1500 |
| anti-goat IgG (H+L)-FITC | | Jackson Immunoresearch 705-096-147 | | | donkey | 1/500 |
| anti-goat IgG (H+L)-Cy-3 | | Jackson Immunoresarch 705-166-147 | | | donkey | 1/1500 |
| anti-rabbit IgG (H+L)-FITC | | Jackson Immunoresearch 711-095-152 | | | donkey | 1/500 |
| anti-rabbit IgG (H+L)-Cy-3 | | Jackson Immunoresearch 711-165-152 | | | donkey | 1/1500 |
| | | | | | | |

| **Negative control** | **specification** | | | **Concentration (mg/ml)** | | |
|---|---|---|---|---|---|---|
| mouse IgG | Jackson Immunoresearch 015-000-02 | | | 11.1 | | |
| goat IgG | Jackson Immunoresearch 005-000-02 | | | 10.6 | | |
| rabbit IgG | Jackson Immunoresearch 011-000-02 | | | 11.4 | | |

### DNA synthesis

[methyl⁻³H] thymidine (25µCi/mmol, 2µCi/ml) incorporation into trichloroacetic acid precipitated-DNA (Amersham Inc) was measured.

BMSC were, 24h after splitting; cultivated in the presence of LSP cytokines, added either as a cocktail or separately on 10ng/ml FN at 2.465 10³ cells/cm². 1µM TSA was added 24, 96 and 168h after splitting. Media were changed every three days. Samples were taken 2, 4, 6, 12 and 18h after splitting or media change. Cells were stimulated for 2h before sampling with 2µCi [methyl-³H] thymidine (Amersham Pharmacia Biotech) / ml medium. [methyl-³H] thymidine incorporation was measured using a scintillation counter (Wallac 1410) with a counting efficiency of 62.4%.

### Albumin ELISA

ALB concentrations secreted into the media were analysed by competitive enzym-linked immunosorbent assay (ELISA) as described (Koebe et al. Int J Artit Organs 17: 95-106, 1994). The ALB levels were normalized with respect to the media used.

### Statistics

Results are expressed as means ± SD. Statistical analyses were performed using Oneway and Multiway Anova and student t-test. The significance level was set at 0.05.

### Results

### Characterization of the differentiation pattern of rat BMSC into hepatocyte-like cells

BMSC were selected and cultivated in expansion medium as described previously (Reyes et al. Blood 98: 2615-2625, 2001; Schwartz et al. J Clin Invest 109:1291-1302, 2002). To induce hepatocyte differentiation, BMSC were cultivated at 100% confluence on 1mg/ml collagen and 1% matrigel in the presence of LSP cytokines, added either as a cocktail or sequentially. In the presence of sequential cytokine stimuli, BMSC became epithelioid from day 9 on. They were at that point, however, still surrounded with spindle shaped cells. From day 14 on, only a few spindle shaped, fibroblastic cells were seen and finally at day 17 binucleated cells were formed. For BMSC, cultivated with a cocktail of LSP specific cytokines, this process was delayed with ± 2 days and only a few binucleated cells were seen from day 19 on.

To characterize the differentiation steps underlying such morphological changes of BMSC, the expression pattern was analyzed at the protein (Figure 1) and mRNA level (Figure 2, Table 3) for early [HNF-1, HNF3β, alphafoetoprotein (AFP)], mid-late (CK18) and late (ALB) LSP markers. During BMSC (clone R8) differentiation, the expression of HNF-1 and HNF-3β increased gradually from day 4 on and reached at day 14 a maximum level. In BMSC (clone R8), treated with all LSP cytokines simultaneously, the level of HNF-1 was at day 14 more than 1.5 times higher than in the presence of sequentially treated BMSC (clone R8). During culture time, a significant difference in expression of HNF-1 was seen for medium, time as well as medium-time (all p <0.001, Multiway Anova). More specific, at every time interval, the expression of HNF-1 was significantly higher in BMSC (clone R8) treated with all LSP cytokines simultaneously than those undergoing sequential induction (p<0.01, Oneway Anova). On the contrary, at day 14 the expression of HNF-3β was significant, and more specific 3270 times, higher in BMSC (clone R8), treated sequentially with LSP cytokines than in the presence of a cocktail of cytokines (p>0.001, Oneway Anova). Even more, no statistical difference (Oneway and Multiway Anova) could be measured between undifferentiated BMSC and BMSC cultivated with all LSP cytokines simultaneously at day 14 (p>0.05, Oneway Anova). After day 14, only low expression of HNF1 and HNF3β were seen.

AFP and ALB are two major proteins characteristic of hepatic differentiation. AFP is an early hepatic marker and its expression decreases as the liver develops into an adult phenotype. On the other hand, expression of ALB, the most abundant protein synthesized by mature hepatocytes, starts in early fetal hepatocytes and reaches maximal levels in adult hepatocytes (Oh et al. Bioch Biophys Res Commun 279: 500-504, 2000). During BMSC differentiation, cells stained positive for AFP at day 3 and the level increased up to day 9. From day 12 on, no AFP expression was seen anymore at the mRNA level. The expression of ALB started at a much later stage during BMSC differentiation. At day 6 only rare cells stained slightly positive for ALB. A significant upregulation was seen at the protein and mRNA level from day 15 on (p<0.001, Oneway Anova) for cells treated sequentially with LSP cytokines (30 ± 5) 10⁻⁶ %. An upregulation was also seen from day 15 on for cells (clone R8) treated simultaneously with all LSP cytokines, although not significant. Cells cultivated in expansion medium did not express ALB while expression of ALB in sequentially treated BMSC (clone R8) was characterized by a 8-fold upregulation from day 15 (10 ± 3) 10⁻⁶ % to day 19 (800±1) 10⁻⁷%. Undifferentiated BMSC cultivated in expansion medium at day 0 did not express AFP nor ALB, and only a very low level of CK18 could be measured. At day 19 low levels of HNF-1, HNF-3β and CK18 were measured in BMSC (clone R8) treated with expansion medium. Still a clear difference was seen in the expression of LSP markers between differentiated and undifferentiated BMSC. In differentiated BMSC (clone R8) a gradual increase of CK18 was seen from days 9 and 12 on, at the protein level and mRNA level, respectively. Under both culture conditions, a maximum level was reached at day 14. For cells (clone R8) treated with cocktail it was, however, significant lower, more specifically 236 times, than cells treated (clone R8) sequentially with LSP growth factors (p<0.001, Oneway Anova). Even more, expression of the mid-late marker CK18 was at every time interval during culture higher in the presence of sequentially treated BMSC (clone R8). In conclusion, BMSC sequentially cultivated with LSP cytokines show significant lower expression of the early LSP marker HNF-1 during culture time (p< 0-001, Oneway Anova). Significant higher levels, however, of mid-late (CK18) and late (ALB) LSP markers were measured as compared to those observed in BMSC cultivated with a cocktail of LSP cytokines at day 14 and from day 15 on, respectively (p<0.001, Oneway Anova). Significance was seen for medium, time as well as medium-time (all p<0.001, Multiway Anova). At day 14, a significant higher level of the transcription factor HNF3β was observed in the presence of sequential cytokine stimuli (p<0.001, Oneway Anova), whereas no statistical difference could be measured between undifferentiated BMSC and BMSC cultivated with all LSP cytokines simultaneously. This observation could be explained by the fact that HNF3β is more than HNF1-involved in the transition phase from immature to more mature hepatocytes. It seems thus that sequential cytokine stimuli induce a higher stage of maturation of BMSC into hepatocyte-like cells than a cocktail of LSP cytokines.

**Table 3. Characterization of rat BMSC differentiation into hepatocyte-like cells at the mRNA level (relative abundance in cultivated cells compared with rat adult liver). 2 different clones of rat BMSC, termed R8 (A) and R20 (B) were cultivated on 1mg/ml collagen at 100% confluence in the presence of hepatocyte-specific cytokines, added either as a cocktail (FGF+HGF+ITS+Dex) or sequentially (FGF/HGF/HGF,ITS,Dex). mRNA levels were normalized using 18S and compared with mRNA levels in rat adult liver (positive control) and undifferentiated BMSC (negative control) cultivated in expansion medium (EGF, PDGF). (NT, not tested). Values represent means of three independent experiments.**

| **(A)** | | | | | | | | | | | |
|---|---|---|---|---|---|---|---|---|---|---|---|
| **Day of culture** | **Medium used** | **µg RNA** | | **AFP** | **HNF1** | | **CK18** | | **HNF3β** | | **ALB** |
| D0 | EGF/ PDGF | 1 | | 0±0 | 0±0 | | (65 ±7) 10⁻⁶ | | 0±0 | | 0±0 |
| D12 | Sequent ial | 1 | | 0±0 | (30±4) 10⁻⁴ | | (70 ±4) 10⁻⁶ | | (41±4) 10⁻⁷ | | (100± 6) 10⁻¹⁰ |
| D12 | Cocktail | 1 | | 0±0 | (2000±7) 10⁻⁵ | | (100±8) 10⁻⁷ | | (706±8) 10⁻⁸ | | 0±0 |
| D14 | Sequent ial | 1 | | 0±0 | (550±71) 10⁻⁴ | | (279±44) 10⁻³ | | (121±21) 10⁻² | | NT |
| D14 | Cocktail | 1 | | 0±0 | (900±1)10⁻⁴ | | (1180±8) 10⁻⁶ | | (37±3)10⁻⁵ | | NT |
| D15 | Sequent ial | 1 | | 0±0 | (106±22) 10⁻⁴ | | (2±1)10⁻³ | | (9±4) 10⁻⁴ | | (10 ± 3) 10⁻⁸ |
| D15 | Cocktail | 1 | | 0±0 | (230±156) 10⁻⁴ | | (16±2) 10⁻⁵ | | (107±8) 10⁻⁷ | | (10±1) 10⁻⁹ |
| D18 | Sequent ial | 0,5 | | NT | (261±15) 10⁻⁴ | | (459±3) 10⁻⁴ | | NT | | (30±5) 10⁻⁸ |
| D19 | Sequent ial | 0,5 | | NT | (491±15) 10⁻⁴ | | (772±5) 10⁻⁴ | | NT | | (800±1) 10⁻⁹ |
| D19 | EGF/ PDGF | 1 | | 0 ± 0 | (81±15) 10⁻⁴ | | (800±5) 10⁻⁷ | | (52±6) 10⁻⁶ | | 0±0 |

| **(B)** | | | | | | | | | | | |
|---|---|---|---|---|---|---|---|---|---|---|---|
| **Day of culture** | **Medium used** | | **µg RNA** | | **AFP** | **CK18** | | **HNF-3β** | | **ALB** | |
| D0 | EGF PDGF | | 1 | | 0 ± 0 | (7±0.7) 10⁻⁵ | | 0±0 | | 0±0 | |
| D14 | Sequential | | 1 | | 0±0 | (30566±50) 10⁻⁵ | | (6426± 270) 10⁻⁵ | | (620±30) 10⁻⁸ | |
| D14 | Cocktail | | 1 | | 0±0 | (413±8) 10⁻⁵ | | (13±2) 10⁻⁵ | | (20±0.4) 10⁻⁸ | |
| D14 | EGF/PDGF | | 1 | | 0±0 | (48±2) 10⁻⁵ | | (1±0.08) 10⁻⁵ | | 0 ± 0 | |

### Optimization of the differentiation process of rat BMSC-derived hepatocyte-like cells

In order to enhance differentiation and maturation of BMSC-derived hepatocyte-like cells, the present culture conditions were optimized. Trichostatin A (TSA), a selective and reversible histone deacetylase-inhibitor, was introduced into the culture medium.

It was important to examine from what point of cultivation, at which concentration and for how long TSA was to be added to the culture media in order to induce cell cycle arrest (necessary for differentiation) but not apoptosis. [methyl-³H] thymidine incorporation was used to examine the DNA synthesis of differentiated BMSC (Figure 3). BMSC treated with 5 and 25 µM TSA showed no incorporation of [methyl ³H] thymidine. Microscopic analysis of the treated BMSC suggested that this result was caused by cytotoxicity of 5 and 25µM TSA. TSA was then added at different time intervals during cell culture but cell death could not be inhibited (data not shown). BMSC were next treated with 1µM TSA. Addition of 1µM TSA, from 24 and 96h of cultivation on, to BMSC cultivated in the presence of a cocktail of LSP cytokines, caused an increase of the DNA synthesis compared with control (cocktail-TSA). 8h after first addition of 1µM TSA from 24h of cultivation on, the DNA synthesis was gradually downregulated. However, at that time interval the level of DNA synthesis was still 42.2 times higher than the values observed in non-treated cells. On the other hand, no [methyl-³H] thymidine was seen after first addition of 1 µM TSA from 168h of cultivation on, as was the case for the control. Microscopic analysis showed that no cell death had occurred in both control- and TSA-treated cells at day 7.

Quite different results were obtained when cells where sequentially treated with LSP cytokines in the presence or absence of 1µM TSA. Addition of TSA from 24, 96 and 168h on caused a decrease of the DNA synthesis as compared with control (sequential without TSA). Microscopic analysis showed, however, that in the presence of TSA added from 24 and 96h on, this observation was not caused by cell cycle arrest but rather by cell death. Less cell death although was seen in the latter culture condition. Cells treated for the first time with 1µM TSA from 168h cultivation on, showed no [methyl-³H] thymidine incorporation. No death cells were observed by microscopic analysis, pointing to cell cycle arrest.

We may conclude that 1µM TSA can only be added late (e.g., in the present experiment on day 6) in cultivation to both types of treated BMSC.

### Albumin secretion

To examine whether cells with morphologic and phenotypic characteristics of hepatocytes also had similar functional hepatocyte characteristics, the ALB secretion into the medium was measured at various time intervals throughout the differentiation process (Figure 4). 1µM TSA was added from 168h (day 6 of differentiation) on. The ALB secretion was significantly upregulated in sequentially treated BMSC in the presence of TSA from day 15 on (p<0,001, Oneway and Multiway). Under these conditions, the levels of ALB secretion were at day 17 (3.12 ± 0.118) µg/ml even similar to those seen in monolayer cultures of primary rat hepatocytes at day 2 (Hamilton et al. In Vitro Cell Dev Biol Anim 37: 656-667, 2001).

In control rat BMSC (sequential without TSA) only a 1.1-fold increase of the ALB secretion was seen from day 15 to 17 compared with a 2.5-fold significant increase in the presence of TSA (p<0,001, Oneway and Multiway Anova)- Rat BMSC cultivated in the presence of a cocktail of LSP cytokines did not secrete ALB. No valuable results were obtained in the presence of TSA since cell death had occurred from day 16 on.

These data demonstrate again that BMSC cultivated in the presence of sequentially added LSP cytokines differentiate to a higher extent into hepatocyte-like cells than BMSC cultivated in the presence of all cytokines simultaneously- Addition of 1µM TSA to sequentially treated BMSC, synergistically and significantly increased the differentiation process into hepatocyte-like cells (p<0.001, t-test).

### Characterization of the differentiation pattern of rat BMSC into neuroectodermal-like cells

The mammalian brain develops as a tube containing ventricular compartments filled with cerebral fluid. During development, the dividing cells are located in the cell layer that lines the lumen of the neural tube. These cells show a trilineage potential, being capable of differentiating into neurons, astrocytes and oligodendrocytes. The existence of these multipotential neural stem cells *in vivo* is now widely accepted, and it seems that growth factors such as EGF and the FGFs are important for their self-renewal. It has already been shown that cells from outside the nervous system, including BMSC, are capable to differentiate, as well *in vivo* as *in vitro,* into cells with morphological, phenotypic and functional characteristics of neuroectodermal cells (Jiang et al. PNAS. 100 Suppl 1: 11854-11860, 2003).

Here, we investigated the difference in neuroectodermal differentiation degree between BMSC cultivated with a cocktail of neuronal-specific growth factors (bFGF, FGF-8) and those sequentially treated with bFGF and FGF-8 (Figure 5, Table 4). Expression of neurofilament 200 (NF-200), an intermediate filament found in nerve cells, was clearly seen during culture time from day 9 on. From day 11 on, a clear difference in morphology was seen between both types of BMSC cultures. BMSC cultivated with a cocktail formed clusters while in the presence of FGF-8, cells with round nuclei and small processes were observed. The phenotypic characteristics underlying these morphological differences were investigated by quantitative RT-PCR analysis (Table). For BMSC cultivated in the presence of the mixture of bFGF and FGF-8 a high expression of the early transcription factor Sox1 (60 ± 0.1) % was observed at day 14. The latter is known to be important during neural crest development. At day 18 a significant, 120-fold downregulation (p<0.001, Oneway Anova) was observed and instead other markers important for further neuroectodermal differentiation were expressed including the neuronal progenitor marker nestin, the neuronal lineage specific noradrenergic marker gene TH, which is important in sympathetic neuronal development and the astrocytic marker GFAP. Since only a low expression of GFAP and a high expression of nestin and TH were observed, these results point to the possibility that BMSC in the presence of a cocktail of neuronal-specific cytokines differentiate into a mixture of immature astrocytes and immature peripheral sympathetic neurons at day 18 of the differentiation process. BMSC cultivated sequentially with bFGF and FGF-8, on the other hand, undergo a significant, 100-fold upregulation (p<0.001, Oneway and Multiway Anova) of MBP, a marker of mature oligodendrocytes. It can thus be concluded that the degree of neuroectodermal differentiation of BMSC as well as the specific cell fate of these BMSC-derived neuroectodermal cells (neurons, astrocytes or oligodendrocytes) strongly depend on exogenic environmental factors. In the presence of sequentially added neuronal-specific cytokines, BMSC obtained specific characteristics of mature oligodendrocytes while a mixture of cells with characteristics of immature astrocytes and immature peripheral sympathetic neurons were formed in the presence of a cocktail.

**Table 4. Characterization of rat BMSC differentiation into neuroectodermal-like cells at the mRNA level (relative abundance in cultivated cells compared with rat adult brain). A clone of BMSC, termed R8 was cultivated on 10ng/ml FN at 30% confluence in the presence of neuronal-specific cytokines, added either as a cocktail (bFGF+FGF-8) or sequentially (bFGF/FGF-8). mRNA levels were normalized using 18S and compared with mRNA levels in rat adult brain (positive control) and undifferentiated BMSC (negative control) cultivated in expansion medium (EGF, PDGF Values represent means of three independent experiments.**

| **Day of culture** | **Medium used** | **µg RNA** | **Sox1** | **Nestin** | **TH** | **GFAP** | **MBP** |
|---|---|---|---|---|---|---|---|
| D0 | EGF/PD GF | 1 | 0±0 | NT | 0±0 | 0±0 | 0 ± 0 |
| D14 | Sequent ial | 1 | NT | NT | NT | NT | (4.0±0.3) 10⁻⁷ |
| D14 | Cocktail | 1 | (60.0±0.1) 10⁻² | 0±0 | 0±0 | 0±0 | 0±0 |
| D14 | EGF/PD GF | 1 | | | | | |
| D18 | Sequent ial | 0.5 | (50.0±0.8) 10⁻⁴ | (1.2±0.3) 10⁻¹ | (2.8±0. 2) 10⁻² | (2.0±0.2) 10⁻⁵ | 0 ± 0 |
| D18 | Cocktail | 0.5 | 0 ± 0 | 0 ± 0 | 0 ± 0 | 0 ± 0 | (4.0±0.2) 10⁻⁵ |

### Conclusions

Our results clearly indicate that BMSC-derived hepatocyte-like cells acquire a much more mature phenotype in the presence of sequentially cytokine stimuli than in the presence of a mixture of all LSP cytokines together. Rat BMSC-derived hepatocyte-like cells showed greater expression of mid-late (CK 18) and late (ALB) markers when FGF-4/ HGF/ HGF, ITS and Dex were sequentially added compared to treatment with all LSP cytokines together. Also less of the early marker HNF-1 was detected. Addition of 1µM TSA to sequentially treated BMSC-derived hepatocyte-like cells synergistically and significantly increased the hepatocyte differentiation process. In the presence of 1µM TSA, ALB secretion into the medium was upregulated at days 15 and 17 to levels similar to those found in monolayer cultures of rat primary hepatocytes at day 2.

Additionally, cells with more mature phenotypic characteristics of neuroectodermal cells were obtained in the presence of sequential neuronal-specific cytokine stimuli rather than with a cocktail of cytokines. Microscopic analysis and quantitative RT-PCR analysis showed that the degree of maturation as well as the specific cell fate (neurons, astrocytes and oligodendrocytes) of BMSC-derived neuroectodermal-like cells strongly depend on the time specific presence of external inducing factors. BMSC cultivated sequentially with bFGF and FGF-8 differentiated from day 14 on into cells with morphological and phenotypic characteristics of mature oligodendrocytes while a mixture of cells with phenotypic characteristics of immature astrocytes and neurons were obtained in the presence of the cocktail.

### EXAMPLE 2: In vitro pluripotency of human marrow mesenchymal stem cells

### Materials and methods

### Isolation and expansion of human mesenchymal stem cells (hMSC)

hMSC were collected from 5-30 ml aspirates from the sternum of hematological healthy donors and expanded *in vitro* at low density for 4 passages in fetal-calf serum contai ning medium.

### Multilineage differentiation of hMSC

Low-density hMSC (CD45⁻, Thy⁺) were cultivated at 21.5 10³ cells / cm² on 1mg/ml collagen gel type I matrix in the presence of liver-specific (LSP) cytokines, added either as a cocktail [basal medium supplemented with 10ng/ml FGF-4 (fibroblast growth factor type 4), 20ng/ml HGF (hepatic growth factor) (all from R&D), 1 x ITS (insulin, transferrin, selenious acid) and 0.05µM Dex (dexamethasone) (all from Sigma)] or sequentially [basal medium supplemented with 10ng/ml FGF-4, following basal media supplemented with 20ng/ml HGF (R&D) and finally basal medium supplemented with 20ng/ml HGF, 1x ITS and 0.05µM Dex]. Basal medium consisted of 60% (v/v) DMEM-LG/ 40% (v/v) MCDB-201 supplemented with 1mg/ml LA-BSA, 100 IU/ml penicillin, 100µg/ml streptomycin, 0.1mM L-ascorbic acid, 4µg/ml nicotinamide, 27.3mg/l sodium-pyruvate and 1 .623mM glutamin (all from Sigma). The media were changed every three days. Different concentrations of trichostatin A (TSA) (1, 5, 25µM) (Sigma) were added at different time intervals during cell culture as further indicated.

### Cytological staining

Cells were fixed with 10% formalin for 10min at room temperature (neurogenic, adipogenic, hepatocyte differentiation) or with MeOH for 2min at -20°C (osteogenic differentiation). After fixation, neurons and nerve vessels were identified by Cresyl Fast (Dickinson et al. Brain Res 635: 169-178, 1994; Fang et al. Cryobiology 29: 267-273, 1992) and Bodian staining (Shimada et al. J Vet Med Sci 54: 29-36, 1992; Wullimann et al. Anat Embryol 199: 329-348, 1999), respectively. Adipocytes were identified as red colored lipid droplets after staining with Sudan III or Oil-Red-O (Reyes et al. Blood 98: 2615-2625, 2001). Mineralized nodules were stained black with the von Kossa technique whereas unmineralized nodules were stained yellow. Periodic-acid-Schiff staining (PAS-staining) was used to determine glycogen storage, a functional parameter of endogenic differentiation. As control, PAS staining was done in the presence of diastase.

### Immunofluorescence

### Hepatocyte differentiation

Differentiated and undifferentiated hMSC were fixed for 1 min with MeOH at -20°C, blocked for 45min with 5% BSA in PBS and immunostained for 2h in the dark with mouse CK 18-FITC (1/100; Sigma) at room temperature. Befween each step, slides were washed 3 times with PBS. Finally, cells were mounted with DAKO Fluorescence mounting medium and analyzed using fluorescence microscopy with a Zeiss Axiovert scope. As a negative control, cells were incubated with mouse gamma globulin (IgG mouse) in the same concentration as used for the primary antibody.

### LDH-leakage

The time dependence of the cytotoxicity of TSA (1, 5, 25µM) was evaluated by means of measurement of lactate dehydrogenase leakage into the media. The assay was done with an LDH-kit (Merck 1 14869-0001; Ecoline 1S) according to the manufacturer's recommendations.

### Albumin (ALB) ELISA

ALB concentrations secreted into the media were analysed by a competitive enzyme-linked immunosorbent assay (ELISA) as described (Koebe et at. Int J Artif Organs 17: 95-106, 1994). The ALB levels were normalized with respect to the media used.

### Statistics

Results are expressed as mea ns ± SD. Statistical analyses were performed using Oneway Anova and student t-test. The significance level was set at 0.05.

### Results

Low-density hMSC cultures in the presence of FGF-4, HGF, ITS and Dex differentiated to mesenchymal cell types, neuroectodermal-like and hepatocyte-like cells

hMSC were cultivated at 21.5 10³ cells/cm² on 1mg/ml collagen type I in the presence of 10ng/ml FGF-4, 20ng/ml HGF, 1 x ITS and 0.05µM Dex.

### Adipogenic differentiation

Adipogenic differentiation was seen from day 5 on. After 17 days of cultivation more than 80% of cells, differentiated into lipid-laden cells, stained with Sudan III (Figure 6). Although such markers are also expressed in hepatic epithelium, the differentiated cells did not have an epithelial-like morphology and did not stain for other hepatocyte-specific markers such as alphafoetoprotein, ALB and CK18. Neither did they took up glycogen.

In the presence of all LSP cytokines also cells with morphological characteristics of neuron-like cells and osteoblasts were observed. In order to obtain more homogeneous hMSC-derived cell cultures, hMSC were separately treated with each of the cytokines FGF-4, HGF, ITS and Dex. It appeared that in the presence of ITS and Dex only adipocytes were formed.

### Osteogenic differentiation

The presence of calcium phosphate, characteristic for osteoblasts, was shown by von Kossa staining from day 5 on (Figure 7). hMSC at day 0 did not stain positive. In the presence of all LSP cytokines also adipocytes were observed. A more homogenous cell culture of osteoblast and neuron-like cells could be obtained in the presence of FGF-4 alone.

### Neurogenic differentiation

Neuron-like cells were stained purple at day 5 with Cresyl Fast violet while hMSC at day 0 did not (Figure 8). Bodian staining, on the other hand, clearly showed the presence of nerve fibers (black staining) at day 8. In the presence of all LSP cytokines also adipocytes were observed. A more homogenous cell culture of neuron-like cells and osteoblasts could be obtained in the presence of FGF-4 alone.

### Endogenic differentiation

Since no cells with typical morphological characteristics were seen in the presence of a cocktail of LSP cytokines, MSC were treated sequentially with 10ng/ml FGF-4, 20ng/ml HG F, 1x ITS and 0.05µM Dex.

Glycogen uptake (Figure 9) was seen under these culture conditions from day 9 on and was clearly upregulated throughout culture time. Moreover, cells did also stain positive for CK18, a cytoskeletal filament present in hepatocytes, while control mouse IgG did not (Figure 10). On the other hand, no cells with the same morphological characteristics as hepatocytes were seen.

### Optimization of the differentiation process of hMSC into hepatocyte-like cells

The culture conditions described above were optimised in order to obtain further endogenic differentiation. TSA, a selective and reversible HDAC-I was added to the culture media. It is known that TSA, a drug candidate for hyperproliferative disorders and a selective and reversible potent HDAC-inhibitor, plays a regulating role in cellular proliferation and differentiation (Xu et al. Biochem Pharmacol 53: 951-957, 1997; Gray et al. Biochem Biophys Res Commun 245: 423-427, 1998; Sowa et al. Biochem Biophys Res Commun 241: 142-150, 1997; Yoshida et al. A Exp Cell Res 177: 122-131, 1988; Wang et al. Mol Cell 8: 817-828, 2001; Yoshida et al. J Biol Chem 265: 17174-17179, 1990) depending on the cell type involved, the concentration and the time of exposure. It has for instance been shown that nanomolar concentrations of TSA cause a reversible cell cycle arrest, and induce hepatocyte differentiation and apoptosis in highly proliferating hepatoma cells and cell lines (depending upon the cell type) (e.g., Xu et al. Biochem Pharmacol 53: 951-957, 1997).

The non-cytotoxic concentration of TSA was investigated by determination of the lactate dehydrogenase leakage into the media. hMSC were cultivated on 1mg/ml collagen type I at 21.5 10³ cells/cm² in the presence of LSP cytokines (FGF-4, HGF, ITS and Dex), added either as a cocktail or separately. 1, 5 and 25µM TSA was added from day O until addition of HGF. During hMSC cultivation, exposure to 5 and 25µM TSA caused immediate cell death. Changing the exposure time or the time point of addition of TSA could not prevent cell death. Addition of 1µM TSA to the culture media did not cause immediate cell death. Even better, TSA significantly downregulated cell lethality in the presence of all and sequentially added cytokine stimuli from day 13 and 3 on, respectively (p<0.05, Oneway Anova and t-test) (Figure 11).

### Albumin (ALB) secretion

MSC were exposed sequentially, at different time intervals during cell culture, to LSP cytokines (FGF-4, HGF, ITS and Dex). TSA was added from day 0 until addition of HGF. It was also tested whether hepatic secretion factors exert a positive effect on differentiation of MSC into hepatocyte-like cells (Figure 12). As expected, an upregulation of the albumin excretion was seen when conditioned medium, containing hepatic secretion factors, was introduced. A clear difference was seen, however, between cells cultivated with and without TSA. From day 15 on, ALB secreted by treated cells was significant hig her than non-treated cells (p<0.01, Oneway Anova). As such a 2.28 fold difference in ALB secretion could be seen between both culture conditions at day 15. This could indicate that, upon addition of hepatic secretion factors, the difference between the measured levels of ALB secretion in treated MSC (+ TSA) and control cultures (- TSA), is the effective level of ALB secreted by the MSC and not the ALB which was already present in the conditioned media. On days 15 (2.747 ± 0.118) pµ/ml and 17 (2.26 ± 0.092) ALB levels were even similar to those produced by monolayer cultures of primary rat hepatocytes on day 2. Previous research showed that rat hepatocytes in conventional monolayer cultures secrete constant levels of ALB between (3,6 ± 0,1) µg/ml and (2,5 ± 1,1) µg/ml from day 2 to 7 after isolation (unpublished data).

Seen the upregulation of ALB secretion obtained upon TSA introduction, the experiments were repeated in the absence of hepatic secretion factors and in the presence of 1µM TSA, added from day 6 of differentiation on. hMSC were cultivated on 1mg/ml collagen type I at 21.5 10³ cells/cm² in the presence of LSP cytokines (FGF-4, HGF, ITS and Dex), added either as a cocktail or separately (Figure 13). Addition of 1µM TSA to sequentially treated hMSC induced a significant upregulation of the ALB secretion from day 15 on (p<0.01, Oneway Anova) compared with control cultures (sequential without TSA). At day 17, the ALB production in the presence of TSA even reached similar levels as observed in monolayer cultures of primary rat hepatocytes at day 2. This observation was similar to MSC sequentially treated with LSP cytokines and hepatic secretion factors in the presence of 1µM TSA, but in a later stage of differentiation. An upregulation of the ALB secretion was also seen in the presence of a cocktail and 1µM TSA, although not significant and in less extent than the former treatment. As such, at day 15 and 17, (2.019 ± 0.144) µg/ml and (3.456 ± 0.08) µg/ml ALB, respectively, were secreted by sequentially treated hMSC which is 2.5 times higher than treatment with all cytokines simultaneously and TSA. MSC cultivated without TSA did not secrete albumin.

### Conclusions

Our results clearly indicate that in the presence of a cocktail of LSP cytokines, low-density MSC differentiate into mesodermal (adipocytes and osteoblasts) and progenitors of ectodermal (neuron-like cells) cells. Under these conditions, no cells with morphological characteristics of hepatocytes were seen. On the other hand, sequential treatment of hMSC with LSP cytokines induced differentiation into endodermal cells (hepatocyte-like cells). Expression of the mid-late hepatic marker CK18 was seen from day 7 on and PAS-staining showed an upregulation of glycogen storage from day 9 to 22. For further differentiation of hMSC towards endodermal lineage, hMSC needed a trigger. Our data show that addition of 1µM TSA to hMSC, treated with LSP cytokines in a sequential way, significantly increased the differentiation process of MSC into hepatocyte-like cells. At day 17 of differentiation, the ALB production even reached similar levels as found in monolayer cultures of primary rat hepatocytes at day 2. Microscopic analysis supported these observations. MSC treated with 1µM TSA in the presence of LSP cytokines, added sequentially, formed after 3-5 days epithelioid cells with a round clear nucleus.

### EXAMPLE 3: Some sequences of adding differentiation agents to produce hepatocyte-like cells from multipotent adult progenitor cells and/or mesenchymal stem cells

The below listed sequences of adding differentiation agents may be used to produce hepatocyte-like cells from multipotent adult progenitor cells and/or mesenchymal stem cells. In all cases, MAPC or MSC are expanded and plated at high density in a EGF/PDGF-containing growth medium or basal medium, respectively. When the cells reach approximately 80-100% confluence, the differentiation process can be started (denoted as Day 0). A histone deacetylase inhibitor, such as TSA (typically 1 µM) or TSA a nalogues, are typically added from day 6 of the differentiation treatment onward. Note that all media comprise sodium pyruvate to promote growth of gluconeogenic cells, and nicotin amide.

Some useful sequences of adding differentiation agents are listed here:

### Sequential I

1) Day 0-3: 10ng/ml FGF-4
2) Day 3-6: 20ng/ml HGF
3) From Day 6 on: 20ng/ml HGF, 0.05µM Dexamethasone, 1 x ITS

### Sequential II

1) Day 0-1: 10ng/ml FGF-4
2) Day 1-3: 10ng/ml FGF-4, 20ng/ml HGF
3) Day 3-6: 5ng/ml FGF-4, 30ng/ml HGF, 0.5 x ITS
4) Day 6-Day 9: 5ng/ml FGF-4, 30ng/ml HGF, 0.05µM Dexamethasone
5) From Day 9 on: 20ng/ml HGF, 0.05µM Dexamethasone

### Sequential III

1) Day 0-3: 10ng/ml FGF-4
2) Day 3-6: 5ng/ml FGF, 20ng/ml HGF, 1 x ITS
3) Day 6-Day 9: 20ng/ml HGF, 10ng/ml OSM, 0.05µm Dexamethasone
4) From Day 9 on: 10ng/ml OSM (oncostatin M), 0.05µM Dexamethasone

### Sequential IV

1) Day 0-3: 10ng/ml FGF-4, 10ng/ml BMP (bone morphogenetic protein) 2, 10ng/ml BMP4, 10ng/ml BMP7
2) Day 3-6: 20ng/ml HGF, 1 × ITS
3) From Day 6 on: 10ng/ml OSM, 0.05µM Dexamethasone

### Sequential V

1) Day 0-3: 10ng/ml FGF-4, 10ng/ml BMP2, 10ng/ml BMP4, 10ng/ml BMP7
2) Day 3-6: 20ng/ml HGF, 1 × ITS
3) Day-6-9: 20ng/ml HGF, 10ng/ml OSM, 0.05µM Dexamethasone
4) From day 9 on: 10ng/ml OSM, 0.05µM Dexamethasone

### EXAMPLE 4: Anti-proliferative and anti-apoptotic activity of the histone deacetylase inhibitor 5-(4-dimethylaminobenzoyl)-aminovaleric acid hydroxamid (4-Me₂N-BAVAH) in primary hepatocytes

We previously reported that trichostatin A (TSA) arrests primary rat hepatocytes in either the early G1 phase or at the G1/S transition, depending on time of onset of treatment, further, prevents spontaneous apoptosis of primary rat hepatocytes, and improves one aspect of hepatocyte function, i.e., albumin secretion (Papeleu et al. J Hepatol 39: 374-382, 2003; Vanhaecke et al. in HUG meeting 2003, 28-29 March 2003, Aberdeen, UK). However, TSA undergoes intensive phase I biotransformation in rat hepatocytes, which limits its metabolic stability *in vivo* and in hepatocyte cultures (Elaut et al. Drug Metabol Dispos 30: 1320-1328, 2002). We previously synthesized several amide analogues of TSA and reported their activity as HDAC inhibitors and their higher metabolic stability as compared to TSA (Van Ommeslaeghe et al. Bioorg Med Chem Lett 13: 1861-1864, 2003; Elaut et al. Tox Lett 144(S1): 78, 2003). Among these, at least the 5-(4-dimethylaminobenzoyl)-aminovaleric acid hydroxamide (4-Me₂N-BAVAH) has a significantly higher metabolic stability than TSA (see Example 6). Here, the effects of 4-Me₂N-BAVAH on cell proliferation and survival of primary rat hepatocytes are investigated.

### Materials and methods

### Chemicals

Minimal Essential Medium (MEM), Medium 199 (M199), bovine serum albumin (BSA)- bovine insulin, L-glutamine and crude collagenase type I came from Sigma-Aldrich (Bornem, Belgium). Fetal bovine serum (FBS) was purchased from Invitrogen Life Technologies (Belgium). [methyl-³H]-Thymidine (25 Ci/mmol) was purchased from Amersham Pharmacia Biotech (Buckinghamshire, UK). L-Leucine [4,5-³H] (50 Ci/mmol) came from ICN Biomedicals Inc (CA, USA). Recombinant human epidermal growth factor (EGF) was from Promega (Leiden, The Netherlands). Benzyl penicillin was from Continental Pharma (Brussels, Belgium), streptomycin sulphate, kanamycin monosulphate and sodium ampicillin came from Sigma-Aldrich. TSA (7-[4-dimethylamino)phenyl]-N-hydroxy-4,6-dimethyl-7-oxo-2,4-heptadienamide) (purity ≥ 98%) was from Sigma-Aldrich. 4-Me₂N-BAVAH (5-(4-dimethylaminobenzoyl)-aminovaleric acid hydroxamide) (purity ≥ 96%) was synthesized as described previously (Van Ommeslaeghe et al. Bioorg Med Chem Lett 13: 1861-1864, 2003). All other reagents were readily commercially available and of molecular biology grade.

### Antibodies

Rabbit polyclonal antibodies directed against cdk4 (sc-749), cyclin E (sc-481), cdk1 (sc-954), goat polyclonal anti-p21 (sc-397-G) and mouse monoclonal anti-cdk2 (sc-6248) and Bax (sc-7480) came from Santa Cruz Biotechnology (CA, USA). Rabbit polyclonal anti-phospho-p44/42 MAP kinase (Thr202/Tyr204), anti-phospho-S6 ribosomal protein (Ser235/236), anti-S6 ribosomal protein, anti-Pak1, anti-c-jun and anti-phospho-Rb (Ser780) antibodies were purchased from Cell Signaling Technology (Beverly, USA). Mouse monoclonal anti-cyclin D1 antibody (clone DCS-6) and anti-pan-ERK antibody came from Neomarkers (CA, USA) and Transduction Laboratories (Lexington, USA), respectively. Rabbit polyclonal anti-caspase-3 antibody came from Calbiochem-Novabiochem, USA. Rabbit polyclonal anti-acetylated histone H4 was purchased from CamproScientific (Veenendael, The Netherlands). Goat polyclonal anti-Bid came from R&D Systems (Abingdon, UK).

### Primary rat hepatocyte cultures

Male outbred Sprague-Dawley rats (200-300 g, Iffa Credo, Brussels, Belgium) were kept under controlled environmental conditions (12-hr light-dark cycle) and fed a standard diet (Animalabo A04, water *ad libitum).* Hepatocytes were isolated by a two-step collagenase perfusion as described (De Smet et al. In 'Methods in Molecular Biology, Vol. 107: Cytochrome P450 Protocols', Philips IR and Shephard EA, eds., pp 295-301, Humana Press, Totowa, 1998). Cell viability was >90% as determined by trypan blue exclusion. Cells (0.4 x 10⁵ cells/cm²) were plated in MEM/M199 (3:1), containing BSA (1 mg/ml), bovine insulin (10 µg/ml), L-glutamine (2 mM), FBS (10% v/v) and antibiotics (7.3 IU/ml benzyl penicillin, 50 µg/ml streptomycin sulphate, 50 µg/ml kanamycin monosulphate and 10 µg/ml sodium ampicillin). Four hours after plating, serum-containing medium was removed and fresh serum-free medium was added to the cultures. Culture medium was refreshed daily. EGF (50 ng/ml) was added 4 hours after plating. A 100 mM stock solution of 4-Me₂ N-BAVAH was prepared in ethanol. Final ethanol concentrations in the medium did not exceed 0.05% (v/v) and did not affect parameters investigated in this study.

### DNA synthesis

Hepatocytes were cultured in triplicate in 35 mm tissue culture dishes (Beckton Dickinson, UK). Cells were incubated with [methyl-³H]-thymidine (25 Ci/mmol (2 µCi/ml)) for 24 hours prior to harvesting. Its incorporation was measured by liquid scintillation counting (Wallac 1410; counting efficiency: 67.5%) after trichloroacetic acid precipitation of the DNA. Results are expressed as counts per minute (cpm) per µg of total cellular proteins. Protein concentration of the samples was determined according to the Bradford procedure (Bradford. Anal Biochem 72: 248-254, 1976), using BSA as a standard.

### Protein synthesis

*De novo* protein synthesis was determined by [³H]-leucine incorporation. [³H]-Leucine (2 µCi/ml final concentration) was added to the hepatocyte cultures 68 hours after plating. Four hours later, cells were washed twice with ice-cold PBS and lyzed in 40% cold trichloroacetic acid. After 2 additional washes in 10% cold trichloroacetic acid, the pellet was solubilized in NaOH (0.1N). The amount of [³H]-leucine was subsequently measured by liquid scintillation.

### Fluorescence-activated cell sorting (FACS)

Ploidy of hepatocyte nuclei was measured by FACS analysis. After hypotonic swelling of the cells with 0.1% sodium citrate, cellular DNA was stained with propidium iodide (50 µg/ml) and analyzed on a FACStar^{plus} (Beckton Dickinson Biosciences, San José, CA, USA).

### Western blotting

Total cell extracts were prepared as described previously (Papeleu et al. J Hepatol 39: 374-382, 2003) and proteins (25 or 50 µg per lane) were separated electrophoretically by SDS-PAGE and electroblotted onto nitrocellulose membranes (Amersham Pharmacia Biotech, Buckinghamshire, UK). Equal sample loading was verified by immersion of the membranes in Ponceau S. Unspecific binding sites were blocked with 5% skim mitk/Tris-buffered saline. Incubations with primary antibodies were performed overnight at 4°C under gentle shaking. Proteins were detected using the ECL detection system (Amersham Pharmacia Biotech, Buckinghamshire, UK) according to the manufacturer's recommendations.

### Histone H4 acetylation

Hepatocytes were treated for 48 hours with 4-Me₂N-BAVAH (10, 25 or 50 µM). Histones were acid-extracted as described previously (Coussens et al. J Biol Chem 254: 1716-1723, 1979). Extracted histones (20 µg per lane) were electrophoresed on a 15% SDS-PAGE as described (Waterborg J et al. Anal Biochem 162: 430-434, 1987) and blotted. Equal sample loading was confirmed with Ponceau S. Membranes were incubated at room temperature with primary antibody for 1 hour under gentle shaking and visualized by ECL. Band intensities were quantified by laser densitometry.

### Lactate dehydrogenase index

The % LDH leakage of the cells was calculated as the result of the ratio: [100 x LDH activity in supernatant]/[LDH activity in (supernatant + cells)]

### Albumin secretion and cell morphology

Medium samples were collected at 72 hours of culture and analyzed for their albumin content by an enzyme-linked immunosorbent assay (ELISA) as described (Dunn et al. Biotechnol Prog 7: 237-245, 1991). Cell morphology was analyzed by light microscopy (x100). Pictures were taken using an Optiphot Nikon Phase Contrast microscope (Cetec, Brussels, Belgium).

### Statistical analysis

Results are expressed as means ± SD. Statistical analyses were performed using a Student t-test test. The significance level was set as 0.05.

### Results

### In vitro HDAC inhibiting activity of 4-Me₂N-BAVAH

Hepatocytes were cultured for 48 hours (from time of plating on) in EGF-containing medium in absence (0 µM) or presence of 10, 25 and 50 µM 4-Me₂N-BAVAH, respectively. In untreated EGF-stimulated hepatocytes, only small amounts of acetylated histone H4 were detected (Figure 14). Exposure of cells to 4-Me₂N-BAVAH induced histone H4 acetylation in a concentration-dependent way. At concentrations as low as 10 and 25 µM, only slightly to moderately induced histone H4 acetylation was observed (2.35±0.05 and 3.56±0.23 fold induction, respectively). In contrast, a massive histone H4 hyperacetylation (6.91±0.63 fold induction) was found after exposure to 50 µM of the compound.

### 4-Me₂N-BAVAH inhibits EGF-induced proliferation and cyclin D1 expression in primary hepatocytes

Adult primary rat hepatocytes stimulated with EGF 4 hours after plating and constantly thereafter, started to incorporate ³H-thymidine between 36 and 48 hours of culture (Figure 15A). DNA synthesis further increased and reached a maximum at 72 hou rs of culture. In the absence of EGF, ³H-thymidine incorporation was very low. Treatment of EGF-stimulated hepatocytes with increasing concentrations of 4-Me₂N-BAVAH, revealed a clear dose-response relationship (Figure 15A). 50 µM 4-Me₂N-BAVAH totally suppressed EGF-induced DNA synthesis, correlating with the observation that a significantly higher proportion of cells remained in G0/G1 (p<0.001) (Figure 15B). The number of cells in G2/M was significantly lower (p<0,05) (Figure 15B).

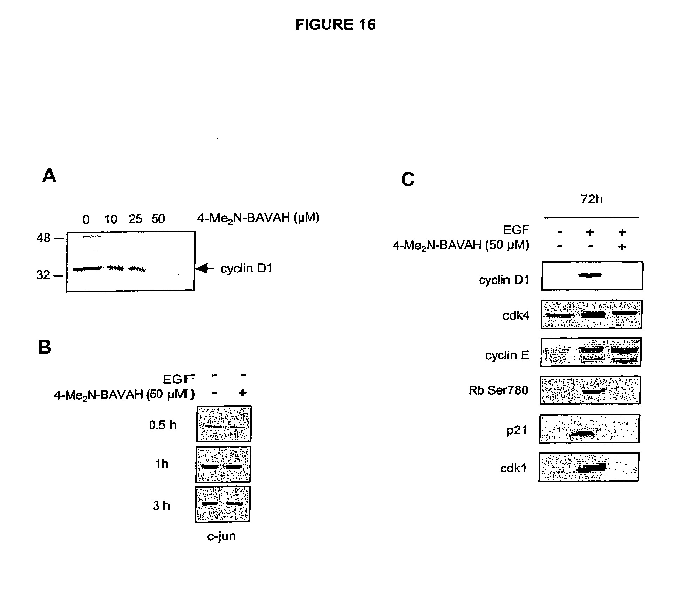

4-Me₂N-BAVAH dose-dependently downregulated cyclin D1 protein. In the presence of 50 µM ₄-Me₂N-BAVAH, hepatocytes completely failed to accumulate cyclin D1 protein upon growth factor stimulation (Figure 16A), even after a long time period of 72 hours (Figure 16A). Interestingly, cells did not return to the G0 phase as c-jun protein expression still could be detected (Figure 16B). The effect of 4-Me₂N-BAVAH (50 µM) on the expression of proteins acting downstream in the cell cycle was also investigated (Figure 16C). The increase in cyclin E and the absence of the S/G2/M marker cdk1 coincided well with the reduction of the number of cells in the S phase. In contrast to what is frequently observed following HDAC inhibitor treatment of cancer cells, the absence of phosphorylated Rb protein, appeared not to be mediated by p21, and was most likely the result of a deficiency in active cyclin D1/cdk4 (due to the absence of cyclin D1) and cyclin E/cdk2 (due to absence of cdk2; not shown) complexes. Indeed, p21 expression, known to be upregulated by mitogens and cyclin D1 expression in hepatocytes, was completely inhibited by treatment with 4-Me₂N-BAVAH (50 µM) (Figure 16C).

Measurement of LD H leakage into the culture medium (Figure 17A), together with morphological examination of the cells (Figure 17B), showed that the observed cell cycle arrest was not due to cytotoxicity. In contrast, hepatocytes cultured in the presence of 50 µM 4-Me₂N-BAVAH, retained the *in vivo-*like cuboidal shape and relatively more bile canaliculi were present (Figure 17B c) when compared to untreated control cultures (Figure 17B b).

### 4-Me₂N-BAVAH-induced G1 cell cycle arrest occurs in the absence of p21 induction

In general, HDAC inhibitors induce a G1 and/or a G2/M cell cycle arrest by upregulating the expression of the cdk inhibitors p21 and/or p27. The absence of p21 protein in 4-Me₂N-BAVAH-treated hepatocytes, therefore, was somewhat surprising. We could demonstrate a tight association between the expression of cyclin D1 and p21 and mitogenic stimulation in proliferating hepatocytes (Figure 18A). The absence of p21 could be easily explained by the fact that none of its functions (i.e. assembly of cyclin D1/cdk4 complexes and inhibition of cyclin D1/cdk4 and cyclin E/cdk2 kinase activities) were required.

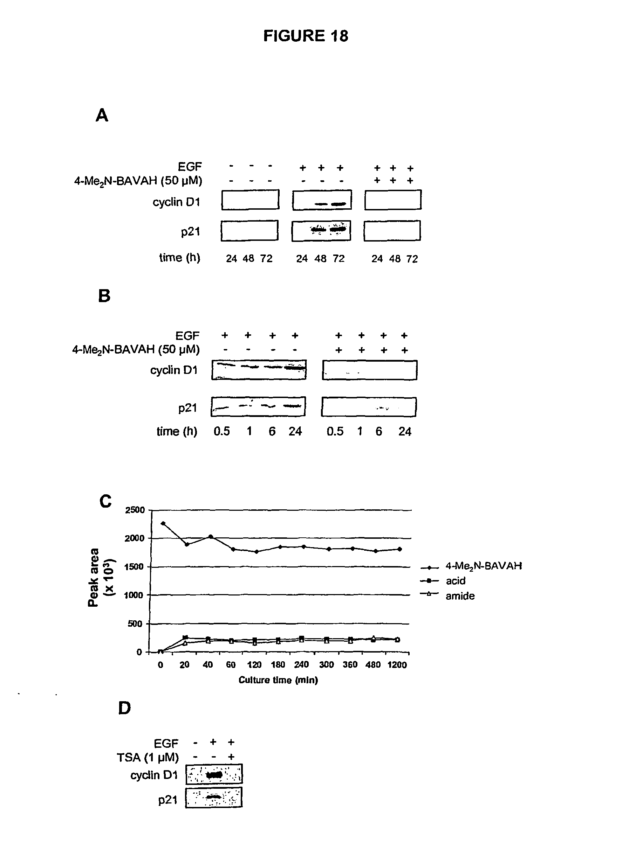

We confirmed this by verifying whether p21 might only be transiently ind uced by a combined treatment with EGF and 4-Me₂N-BAVAH (50 µM) due to rapid biotransformation or instability of the compound in the culture medium. Cells were cultured for 24 hours in the presence of EGF, alone or in combination with 4-Me₂N-BAVAH; culture media were renewed and the expression of p21 was subsequently analyzed at short time intervals (0.5, 1, 6 and 24 hours) (Figure 18B). In untreated EGF-stimulated hepatocytes, cyclin D1 and p21 proteins were induced 0.5 hour after medium renewal. A maximum was reached 24 hours later, namely after 48 hours of culture. As expected, no cyclin D1 and p21 proteins could be detected in 4-Me₂N-BAVAH-treated cells, not even after short exposure periods (Figure 18B).
In addition, we quantified the biotransformation rate of 4-Me₂N-BAVAH using high-performance liquid chromatography (HPLC) combined with simultaneous electrospray ionization mass spectrometric (ESI-MS)/UV detection. No metabolites of the compound could be detected after 20 hours (Figure 18C). We previously showed that the reference compound TSA, when administered during liver perfusion and continuously thereafter during culture (1 µM), induced a similar early G1 cell cycle arrest by inhibiting induction of cyclin D1 (Papeleu et al. J Hepatol 39: 374-382, 2003). Analysis of these samples showed a complete absence of p21 accumulation (Figure 18D). These findings clearly indicate that 4-Me₂N-BAVAH interferes with the mitogenic effects of EGF resulting in a failure to induce cyclin D1 and p21 expression.

### The anti-proliferative effects of 4-Me₂N-BAVAH depend on the length of exposure in G1 and the time of onset of treatment

When stimulated with the appropriate growth factors, hepatocytes progress through the G1 restriction point at approximately 40-44 hours after plating (Loyer Pet al. J Biol Chem 271: 11484-11492, 1996). This corresponds with induction of cyclin D1. In Figure 19A, we show that withdrawal of 4-Me₂N-BAVAH at 24 hours of culture (corresponding to mid-G1 phase), resulted in 50.9±1.6% reduction in ³H-thymidine incorporation compared to untreated control cultures. Concomitantly, EGF-induced cyclin D1 and p21 expression were not inhibited and the presence of cdk1 clearly indicates that, under these conditions, cells progressed through the S/G2/M phases (Figure 19B). Interestingly, when 4-Me₂N-BAVAH-treatment was maintained until 48 hours (i.e. after passage of the restriction point), no DNA synthesis occurred (Figure 19A) and the S/G2/M marker cdk1 could not be detected (Figure 19B). In addition, 4-Me₂N-BAVAH had less or no inhibitory effect on DNA replication when added either in mid-G1 phase (i.e., 24 hours) or after the restriction point (i.e., 48 hours) (Figure 19C), and only slightly reduced the expression of cyclin D1, p21 and cdk1 (Figure 19D) under these experimental conditions. This illustrates that 4-Me₂N-BAVAH interferes with signals that trigger early events in the cell cycle since cells that have progressed into mid- and mid-late G1 are no longer sensitive to its antiproliferative activity.

### Effect of 4-Me₂N-BAVAH on signal transduction pathways mediating EGF-induced hepatocyte, proliferation

Early cell cycle events can be triggered by distinct signalling pathways, activated by EGF, namely: the MEK/ERK, the mTOR/p70S6k, and the Pak1 pathways. Eventually, they all converge on cyclin D1 and play thus an essential role in the regulation of this critical cell cycle protein. As signal transduction kinases, including MEK/ERK and p70S6k, are often only transiently induced shortly after mitogenic stimulation, cells were first synchronized in G1. This was done by culturing the hepatocytes in the absence of EGF for 48 hours, consequently stimulating them with EGF and exposing them to 50 µM 4-Me₂N-BAVAH for 0.5, 1 and 3 hours before harvesting. Shortly after stimulation of synchronized pri mary hepatocytes with EGF, a rapid and sustained phosphorylation of ERK1/2 was observed (Figure 20A). Unexpectedly, an increase in ERK1/2 phosphorylation was seen during the initial 30 min, after concomitant treatment of hepatocytes with 4-Me₂N-BAVAH (50 µM), which decreased rapidly thereafter to control levels. This seems to exclude a role for the ERK1/2 pathway in the inhibitory effect of 4-Me₂N-BAVAH on cyclin D1 protein expression.

This finding also indicates that the compound does not negatively affect the anti-apoptotic function of EGF, which is known to be mainly mediated by ERK in primary rat hepatocyte cultures. Indeed, 4-Me₂N-BAVAH decreased the expression of the pro-apoptotic proteins Bid and Bax (Figure 20B). In addition, cleavage of the inactive pro-caspase-3 into the active smaller p18 fragments was significantly lower at 24 and 48 hours of culture when compared to untreated controls. It was even completely prevented after 72 hours of treatment (Figure 20B). These findings indicate that a transient increase in ERK activation seems to be sufficient to promote hepatocyte survival.

In synchronized hepatocytes, EGF had no substantial effect on the phosphorylation level of the ribosomal S6 subunit, at least not at Ser235/236 (Figure 21A). In the presence of 4-Me₂N-BAVAH (50 µM) only a minor increase in S6 phosphorylation was observed (Figure 21A). Upon EGF stimulation, the *de novo* protein synthesis, a hallmark of p70S6k activation, was dramatically increased but not further enhanced upon co-treatment with 4-Me₂N-BAVAH (Figure 21 B). Nonetheless, a 2.44±0.29-fold increase in albumin secretion, a general marker for hepatocyte differentiation, was observed in the presence of 4-Me₂N-BAVAH (Figure 21 C). Increased functionality is also reflected at the morphological level as hepatocytes cultured in the presence of 50 µM 4-Me₂N-BAVAH retained their *in vivo* cuboidal shape and relatively more bile canaliculi were present compared to untreated control cultures (Figure 17B).

Cyclin D1 expression can also be mediated by Pak1, which is activated by phosphorylation and activation of MEK/ERK or alternatively, through a NF-κB-dependent pathway. As shown in Figure 22, sustained treatment of EGF-stimulated primary hepatocytes with 50 µM 4-Me₂N-BAVAH, induced a decrease in the cellular levels of Pak1. Decreased Pak1 expression may coincide with inhibition of cell spreading as well, a condition that is non-permissive for cell proliferation (Royal et al. Mol Biol Cell 11: 1709-1725, 2000; Bhadriraju et al. Exp Cell Res 278: 92-100, 2002). Consequently, our data strongly suggest that the anti-proliferative activity of 4-Me₂N-BAVAH is mediated through a Pak1-dependent pathway, whereas ERK mediates its anti-apoptotic effects.

### Conclusions

Amide analogues of TSA have been synthesized (Van Ommeslaeghe et al. Bioorg Med Chem Lett 13: 1861-1864, 2003; Elaut et al. Tox Lett 144(S1): 78, 2003), including 4-Me₂N-BAVAH, which show a significantly higher metabolic stability than the reference compound TSA (see Example 6). This study investigated the effects of 4-Me₂N-BAVAH on hepatocyte proliferation and survival. We could show that 4-Me₂N-BAVAH strongly inhibits the mitogenic effects of EGF, but also positively affects the survival signals of EGF. Inhibition of EGF-mediated mitogenic signalling by 4-Me₂N-BAVAH results in an early G1 cell cycle arrest, prior to the restriction point, that is characterized by the absence of cyclin D1 accumulation and by the presence of active, hypophosphorylated Rb. In this study we provide further evidence for a protective role of the HDAC inhibitor 4-Me₂N-BAVAH against apoptosis by the findings that (i) activation of the executioner caspase, caspase-3, is strongly in hibited and (ii) expression levels of Bax and Bid, two pro-apoptotic proteins, are strongly decreased. 4-Me₂N-BAVAH also induces a massive increase in albumin secretion, indicating a significant improvement of the differentiated state of the hepatocytes. In conclusion, 4-Me₂N-BAVAH represents a novel hydroxamic acid-containing HDAC inhibitor with potent anti-proliferative and anti-apoptotic effects in primary hepatocytes.

### EXAMPLE 5: The effect of trichostatin A on dedifferentiation of cultivated rat hepatocytes

After isolation of primary hepatocytes, dedifferentiation occurs as a function of culture time. In this study, the ability of TSA to counteract dedifferentiation of rat hepatocytes in primary culture is analysed.

### Materials and methods

### Isolation of hepatocytes

Hepatocytes were isolated from male Sprague-Dawley rats by a two-step collagenase perfusion as described (De Smet et al. In 'Methods in Molecular Biology, Vol. 107: Cytochrome P450 Protocols', Philips IR and Shephard EA, eds., pp 295-301, Humana Press, Totowa, 1998). Hepatocytes were cultivated in monolayer cultures with Willam's E Medium (WE) for 7 days. TSA (25 µM) was added at time of plating (TSA 0h) or after 4 hours (TSA 4h). Negative controls were untreated; positive controls were treated with ethanol (the solvent of TSA), which was added after isolation (EtOH 0h) or after 4 hours (EtOH 4h). The maintenance of differentiation characteristics was studied after 2, 4, and 7 days (D2, D4, D7) of culture.

### Fluorimetry

Phase I biotransformation was assessed by means of activity measurements of the microsomal cytochrome P450 (CYP) 1A1 and 2B1/2 iso-enzymes, using standard techniques known in the art (i.e., measuring conversion of ethoxyresorufin into hydroxyresorufin for CYP 1A1 and conversion of penthoxyresorufin to hydroxyresorufin for CYP 2B1/2) (Burke et al. Drug Metab Dispos 2:583-588, 1974). Microsomes were prepared from freshly isolated and hepatocytes cultured for 7 days according to Hales et al. (Biochem Pharmacol 26:555-556, 1977).

### Spectrophotometry

In order to investigate Phase II biotransformation, total cytosolic glutathione S-transferase (GST) activity and activities of individual GSTA, GSTP, and GSTM iso-enzymes were measured using standard techniques known in the art. (i.e., measuring conjugation of reduced glutathione with 1-chloro-2,4-dinitrobenzene for total GST; measuring conjugation of reduced glutathione with 7-chloro-4-nitrobenzo-2-oxa-1,3-diazole for GSTA activity; measuring conjugation of reduced glutathione with ethacrynic acid for GSTP activity; and measuring conjugation of reduced glutathione with 1,2-dichloro-4-nireobenzene for GSTM activity).

### Western blotting

Western blotting was performed using standard techniques known in the art. Proteins (25 µg) were resolved on a 10% SDS-PAGE and blotted onto nitrocellulose membrane. The membranes were incubated with appropriate concentrations of primary and secondary antibodies. Proteins were detected using appropriate dilutions of primary and secondary antibodies. Detection was done using the ECL detection system (Amersham Pharmacia Biotech, UK) according to manufacturer's recommendations.

### Results

### Treatment with TSA preserves the activity of CYP 1A1 and CYP 2B1/2 activities in primary hepatocytes

Figure 23A, B shows that at day 4 and 7, the activity of CYP 1A1 and CYP 281/2 - the Phase I biotransformation enzymes - is significantly higher in hepatocytes treated with 1 µM TSA starting at 0h or at 4h than in negative (i.e., EtOH-treated) controls. See also Table 5.

**Table 5. Quantification of the CYP 1A1/2 and CYP 2B1 activities in 7-day conventionally cultured adult rat hepatocytes in the absence and presence of 1 µM TSA. Mean values ± SD of 3 separate experiments (n = 3) are shown. Statistical significance between control and TSA-treated hepatocyte cultures was tested by a paired Student's t-test (*P<0.05).**

| | Activity (pmol resorufin/minute mg microsomal protein) | |
|---|---|---|
| Culture medium | CYP 1A1/2 | CYP 2B1 |
| Control | 2.8 ± 0.5 | 3.4 ± 0.6 |
| TSA | 4.3 ± 0.6* | 4.1 ± 0.7 |

### Treatment with TSA preserves the GST activity in primary hepatocytes

Figure 24A shows that at day 4 and 7, the total GST activity - the Phase II biotransformation enzyme activity - is significantly higher in hepatocytes treated with TSA starting at 4hours than in negative (i.e., EtOH treated) controls. The activity of GSTA iso-enzyme is substantially unaffected by the TSA treatment (Figure 24B), the activity of GSTP iso-enzyme is stimulated at day 4 and 7 by the 1 µM TSA treatment started at 0h or at 4h (Figure 24C), and the activity of GSTM iso-enzyme is stimulated at day 4 and 7 by the TSA treatment started at 4h (Figure 24D).

### Treatment with TSA preserves the expression of the liver-enriched transcription factor C/EBPalpha in primary hepatocytes

Figure 25 shows that both 1 µM TSA and its solvent ethanol increase expression of the liver enriched transcription factor C/EBPalpha (upper band) in primary hepatocytes at day 4 and 7, when added at 0h or 4h.

### EXAMPLE 6: 5-(4-dimethylaminobenzoyl)-aminovaleric acid hydroxamid (4-Me2N-BAVAH) is more stable that trichostatine A in primary hepatocyte cultures and microsomal extracts

### Materials and methods

### Chemicals

TSA or 7-[4-(dimethylamino)phenyl]-4,6-dimethyl-7-oxo-hepta-2,4-dienoic acid hydroxamide (purity ≥ 98%), crude collagenase type I, bovine serum albumin fraction, 7-ethoxyresorufin, resorufin, N-2-hydroxyethylpiperazine-N'-2-ethanotsulfonic acid (HEPES), NADP⁺, glucose 6-phosphate and acetonitrile came from Sigma-Aldrich N.V./S.A. (Bornem, Belgium). Glucose 6-phosphate dehydrogenase (Grade I) was obtained from Boehringer Mannheim (Mannheim, Germany). Trifluoroacetic acid (TFA) of analytical reagent grade was purchased from Merck Eurolab (Leuven, Belgium). All other solvents and chemicals were obtained from various commercial sources and were HPLC or analytical grade. 4-Me₂N-BAVAH was synthesized according to Jung et al. (Journal of Medicinal Chemistry 42: 4669-4679, 1999) and Van Ommeslaeghe et al. (Bioorg Med Chem Lett 13: 1861-1864, 2003).

### Hepatocyte incubations

Male outbred Sprague Dawley rats (Iffa Credo, Brussels, Belgium) weighing 200-300g were used. The animals were kept under controlled environmental conditions (12h light-dark cycle) and had free access to food (Animalabo A 04) and water. After intraperitoneal injection of Nembutal^{R} (0.1ml/kg body weight), hepatocytes were isolated as described by De Smet *et al.* [30]. Cell integrity was evaluated by trypan blue exclusion, and was 88 ± 3% (n = 20). 5, 25 and 50µM of both compounds were incubated with 2×10⁶ hepatocytes/ml (final volume 3ml) in HEPES buffer (pH 7.65, 37°C) up to 6h. Control samples were obtained by boiling the hepatocyte suspension for 2min prior to the addition of substrate; cell-free controls contained the substrate in the incubation buffer without hepatocytes. At selected time intervals, 1.0ml aliquots were submersed in liquid nitrogen to stop the biotransformation reaction. Membrane damage, following exposure to TSA, 4-Me₂N-BAVAH and/or the solvent (0.0833% (v/v) methanol), was checked by measuring the LDH index (Merckotest, Merck, Darmstadt, Germany).

### Microsomal incubations

Human liver specimens (n=3) were acquired from organ donors at the Academic Hospital of the VUB in accordance with the regulations of the local Medical Ethical Committee and immediately frozen in liquid nitrogen. While human liver microsomes were prepared from frozen liver tissue, rat livers (n=3) were freshly isolated for immediate microsome preparation. Microsomal 7-ethoxyresorufin-O-deethylase (EROD) [31] was used as a marker enzyme to evaluate the microsomal biotransformation activity before and after storage. Incubations were carried out in a 100mM sodium potassium phosphate buffer pH 7.4 in the presence of a NADPH-generating system (0.5mM NADP⁺, 10mM glucose 6-phosphate, 2U/ml glucose 6-phosphate dehydrogenase and 0.6mM MgCl₂). Microsomes (0.0-2.0mg microsomal protein/ml) were incubated with 140µM substrate at 37°C for 150min. Boiled microsomes (2min) served as a control. Biotransformation reactions were initiated by addition of substrate and, at selected time intervals, terminated by dilution with ice-cold acetontrile (1:2.5).

### Metabolite separation and identification

After centrifugation (2000g, 45min, 4°C) of the diluted microsomal samples, the clear supernatant was injected onto a HPLC column. Samples from incubations with hepatocytes were thawed on ice and centrifugated (120g, 2min). The supernatants (extracellular) were subjected to solid phase extraction (Waters Oasis^{™} HLB cartridges, Waters Corporation, Milford, Massachusetts, USA), while cell pellets (intracellular) were ultrasonicated in 1ml methanol and centrifugated (2000g, 30min, 4°C). Separations (20µl samples) were performed by reversed-phase HPLC (Kontron chromatographic system, Milan, Italy) on a Discovery C18, 5µm, 250×4.6mm column (Supelco, Sigma-Aldrich, Bornem, Belgium). The mobile phase consisted of component A (0.1 % (v/v) aqueous trifluoroacetic acid) and component B (0.1% (v/v) trifluoroacetic acid in acetonitrile). A linear gradient from 3 to 80% (v/v) B was used over 30min at a flow rate of 1.0ml/min. The HPLC effluent was split (ACURATE by LC Packings, Amsterdam, the Netherlands) 9:1 to direct 100µl/min into a VG Quattro II triple quadropole mass spectrometer with electrospray ionization (ESI) interface (Micromass, Manchester, UK) and 900µl/min to a UV detector⁻(type 332, Kontron, Milan, Italy). ESI was performed in the positive mode, while UV-detection was done at 266nm (TSA) and 255nm (4-Me₂N- BAVAH). The full scan ESI-MS spectrum (mass range from m/z 110 to *m*/*z* 850) from the HPLC/MS analysis allowed us to determine the protonated molecular ions [M+H]⁺ of the metabolites. For structural identification of the metabolites, the mass spectrometer was used off-line in the MS/MS mode (product-ion scanning mode). The first quadrupole of the mass spectrometer, operated in the static mode, was set to monitor the selected molecular ions of the metabolites, and the third quadropole was used in the scan mode over a mass range of m/z 75 to m/z 350 and a scan rate of 1s/scan in order to detect all fragments obtained from the collision induced dissociation (CID) of the selected molecular ions. Structural assignments of the metabolites were based on the interpretation of the spectra obtained from HPLC/MS and CID MS/MS experiments, both with ESI. The degradation of TSA and 4-Me₂N-BAVAH and the formation of their respective metabolites were evaluated semi-quantitatively by comparing the peak areas obtained by UV detection.

### Statistical analysis

The results were analyzed using a paired Student's t-test (degradation of TSA and -4-Me₂N-BAVAH) or a two-way Anova followed by a Student-Newman-Keuls test (TSA and 4-Me₂N-BAVAH metabolites). P<0.05 was the set level of significance.

### Results

### Trichostatin A

### Microsomal incubations

Microsomes (protein range from 0.0 to 2.0mg/ml) were incubated with 140µM TSA during a total period of 150min. Control experiments with microsomes boiled for 2min were performed under identical incubation conditions in order to assess non-enzymatic degradation products ('metabonates'). TSA showed a slow and incomplete biotransformation in both rat (Figure 26) and human (not shown) microsome suspensions. Only two major metabolites could be detected: *N-*mono- (1) and *N*-didemethylated TSA (2) (Figure 28). As this was not within our expectations, higher microsomal protein concentrations and amounts of NADPH-generating system were used in a second set of experiments. However, no increase in the biotransformation rate nor the extent of metabolites formed, was seen (results not shown).

### Hepatocyte incubations

Concentrations of TSA up to 100µM did not affect cell viability (LDH leakage) when incubated with rat hepatocytes in HEPES buffer during 6h, as compared to controls without TSA and with only the solvent (results not shown). Therefore, 5, 25 and 50µM TSA were chosen for further biotransformation experiments. In contrast to microsomes, rat hepatocytes in suspension metabolized TSA rapidly, completely (within 40min; Figure 27A) and extensively. Seven phase I metabolites could now be chromatographically separated and identified; a scheme is provided in Figure 28. These five additional metabolites could also be identified in microsomal suspensions, although in trace amounts. Besides the TSA *N*-demethylation seen in liver microsomal suspensions, a reduction of the hydroxamic acid function to its corresponding amide (metabolites 5, 6 and 7) was now a major phase I biotransformation pathway. Hydrolysis with the formation of a carboxylic acid was a minor pathway (metabolites 3 and 4). While the *N*-demethylated amide 6 was a major metabolite formed in hepatocyte suspensions exposed to 25 and 50µM TSA, *N*-didemethylated TSA (2) took over its role in 5µM incubation experiments (results not shown). Thus, *N*-demethylation pathways are saturated in the presence of higher concentrations of TSA. While this pathway is probably catalyzed by cytochrome P450- or flavin monooxygenase-dependent enzymes, cytosolic or mitochondrial liver enzymes such as aldehyde oxidase are involved in the reduction of TSA. Although microsomes are often proposed as a suitable *in vitro* model to investigate the phase I metabolic stability and profile of NCE's, these results clearly illustrate the need for caution. Biotransformation experiments with microsomes should always be accompanied by confirming studies using a cellular model.

### 4-Me₂N-BAVAH

4-Me₂N-BAVAH inhibits HDAC in the micromolar range. We investigated the biotransformation of 4-Me₂N-BAVAH and compared it to TSA. Since microsomes have been shown to generate an incomplete picture of the phase I biotransformation of TSA, only suspensions of freshly isolated rat hepatocytes were used. Concentrations of 4-Me₂N-BAVAH up to 100µM were not toxic to hepatocytes in suspension (results not shown). Three concentrations were selected for further biotransformation experiments: 5, 25 and 50µM. The most striking difference between the two HDAC inhibitors lies in their metabolic stabilities. While 2×10⁶ hepatocytes/ml were able to fully degrade 50µM TSA within the first 40min of incubation, it took more than 3h in the case of 50µM 4-Me₂N-BAVAH. The most important phase I biotransformation pathways of 4-Me₂N-BAVAH were similar to TSA, including hydrolysis (*1*) and reduction (*2*) of the hydroxamic acid function, and N-demethylation of the dimethylaminobenzoyl moiety (*3*, *4*). In Figure 27B the metabolic degradation of 50µM 4-Me₂N-BAVAH and the formation of its identified phase I metabolites in function of the incubation time is shown. Figure 29 proposes a scheme of the major phase I biotransformation pathways of 4-Me₂N-BAVAH in rat hepatocytes. While hydrolysis was a minor metabolic route for TSA, the acid derivative *1* of 4-Me₂N-BAVAH was a major metabolite throughout the whole incubation period. Moreover, when lower concentrations of 4-Me₂N-BAVAH (5 and 25µM) were used, compound *1* was formed in relatively higher amounts, making it the only major metabolite in 5µM suspensions (results not shown). As for TSA, reduction of 4-Me₂N-BAVAH to its corresponding amide 2 was another major phase I biotransformation pathway. Unlike TSA, 4-Me₂N-BAVAH and its metabolites showed little affinity for *N*-demethylating enzymes. No *N*-demethylated products of 4-Me₂N-BAVAH, nor *N-*didemethylated metabolites could be found. This implies that cytochrome P450 enzymes are probably less involved in 4-Me₂N-BAVAH phase I biotransformation. In comparison to TSA, 4-Me₂N-BAVAH has a longer half-life, which may make it more interesting as a drug.

### EXAMPLE 7: Comparison of the effects of TSA and 5-(4-dimethylaminobenzoyl)-aminovaleric acid hydroxamid (4-Me2N-BAVAH) on dedifferentiation in rat primary hepatocytes

To compare the effects of trichostatin A and 5-(4-dimethylaminobenzoyl)-aminovaleric acid hydroxamid (4-Me2N-BAVAH) on dedifferentiation in primary hepatocytes, we here studied their effects on albumin secretion and on the expression of several hepatocyte-specific or hepatocyte-enriched genes.

### Materials and methods

### Chemicals

Minimal Essential Medium (MEM), Medium 199 (M199), bovine serum albumin (BSA), bovine insulin, L-glutamine and crude collagenase type I came from Sigma-Aldrich (Bornem, Belgium). Fetal bovine serum (FBS) was purchased from Invitrogen Life Technologies (Belgium). Recombinant human epidermal growth factor (EGF) was from Promega (Leiden, The Netherlands). Benzyl penicillin was from Continental Pharma (Brussels, Belgium), streptomycin sulphate, kanamycin monosulphate and sodium ampicillin came from Sigma-Aldrich. TSA (7-[4-dimethylamino)phenyl]-N-hydroxy-4,6-dimethyl-7-oxo-2,4-heptadienamide) (purity ≥ 98%) was from Sigma-Aldrich. 4-Me₂N-BAVAH (5-(4-dimethylaminobenzoyl)-aminovaleric acid hydroxamide) (purity ≥ 96%) was synthesized as described previously (Van Ommeslaeghe et al. Bioorg Med Chem Lett 13: 1861-1864, 2003). All other reagents were readily commercially available and of molecular biology grade.

### Primary rat hepatocyte cultures

Male outbred Sprague-Dawley rats (200-300 g, Iffa Credo, Brussels, Belgium) were kept under controlled environmental conditions (12-hr light-dark cycle) and fed a standard diet (Animalabo A04, water *ad libitum*)*.* Hepatocytes were isolated by a two-step collagenase perfusion as described (De Smet et al. In 'Methods in Molecular Biology, Vol. 107: Cytochrome P450 Protocols', Philips IR and Shephard EA, eds., pp 295-301, Humana Press, Totowa, 1998). Cell viability was >90% as determined by trypan blue exclusion. Cells (0.4 x 10⁵ cells/cm²) were plated in MEM/M199 (3:1), containing BSA (1 mg/ml), bovine insulin (10 µg/ml), L-glutamine (2 mM), FBS (10% v/v) and antibiotics (7.3 IU/ml benzyl penicillin, 50 µg/ml streptomycin sulphate, 50 µg/ml kanamycin monosulphate and 10 µg/ml sodium ampicillin). Four hours after plating, serum-conta ining medium was removed and fresh serum-free medium was added to the cultures. Culture medium was refreshed daily. EGF (50 ng/ml) was added 4 hours after plating. A 100 mM stock solution of 4-Me₂N-BAVAH was prepared in ethanol. Final ethanol concentrations in the medium did not exceed 0.05% (v/v) and did not affect parameters investigated in this study.

### Albumin secretion

Medium samples were collected at 72 hours of culture and analyzed for their albumin content by an enzyme-linked immunosorbent assay (ELISA) as described (Dunn et al. Biotechnol Prog 7: 237-245, 1991).

### Results

### Comparison of albumin secretion of primary hepatocyte cultures treated from day 0 with 1µ trichostatin A or 50 µM 4-Me₂N-BAVAH

Figure 30 shows that primary hepatocytes treated with 50 µM 4-Me₂N-BAVAH show significantly higher secretion of albumin into the culture medium than cells treated with 1 µM TSA.

### 50 µM 4-Me₂N-BAVAH also results in increased expression of CYP 2B as assayed by Western blot (not shown)

### EXAMPLE 8: The effect of TSA on dedifferentiation in rat primary hepatocytes when added to the collagenase perfusion buffer during isolation.

### Materials and methods

### Chemicals

Minimal Essential Medium (MEM), Medium 199 (M199), bovine serum albumin (BSA), bovine insulin, L-glutamine and crude collagenase type I came from Sigma-Aldrich (Bornem, Belgium). Fetal bovine serum (FBS) was purchased from Invitrogen Life Technologies (Belgium). Recombinant human epidermal growth factor (EGF) was from Promega (Leiden, The Netherlands). Benzyl penicillin was from Continental Pharma (Brussels, Belgium), streptomycin sulphate, kanamycin monosulphate and sodium ampicillin came from Sigma-Aldrich. TSA (7-[4-dimethylamino)phenyl]-N-hydroxy-4,6-dimethyl-7-oxo-2,4-heptadienamide) (purity ≥ 98%) was from Sigma-Aldrich. All other reagents were readily commercially available and of molecular biology grade.

### Primary rat hepatocyte cultures

Male outbred Sprague-Dawley rats (200-300 g, Iffa Credo, Brussels, Belgium) were kept under controlled environmental conditions (12-hr light-dark cycle) and fed a standard diet (Animalabo A04, water *ad libitum*)*.* Hepatocytes were isolated by a two-step collagenase perfusion as described (De Smet et al. In 'Methods in Molecular Biology, Vol. 107: Cytochrome P450 Protocols', Philips IR and Shephard EA, eds., pp 295-301, Humana Press, Totowa, 1998) in absence or in presence of 1µM TSA (Papeleu et al. J Hepatol 39: 374-382, 2003). Cell viability was >90% as determined by trypan blue exclusion. Cells (0.4 x 10⁵ cells/cm²) were plated in MEM/M199 (3:1), containing BSA (1 mg/ml), bovine insulin (10 µg/ml), L-glutamine (2 mM), FBS (10% v/v) and antibiotics (7.3 IU/ml benzyl penicillin, 50 µg/ml streptomycin sulphate, 50 µg/ml kanamycin monosulphate and 10 µg/ml sodium ampicillin). Four hours after plating, serum-containing medium was removed and fresh serum-free medium was added to the cultures. Culture medium was refreshed daily. EGF (50 ng/ml) was added 4 hours after plating.

### Albumin secretion

Medium samples were collected at 72 hours of culture and analyzed for their albumin content by an enzyme-linked immunosorbent assay (ELISA) as described (Dunn et al. Biotechnol Prog 7: 237-245, 1991).

### Results

### Comparison of albumin secretion of primary hepatocyte cultures isolated with or without inclusion of 1µM trichostatin A in the collagenase perfusion solution

Figure 31 shows that primary hepatocytes isolated in presence of 1µM TSA in the collagenase perfusion solution show significantly higher secretion of albumin into the culture medium than cells isolated in absence of 1µM TSA in the collagenase perfusion solution.

### EXAMPLE 9: The effect of trichostatin A on gap junctional intercellular communication in primary rat hepatocyte cultures

Trichostatin A (TSA), a histone deacetylase inhibitor, is known to exert major effects on the cellular homeostatic balance, including the induction of cell cycle arrest, differentiation and apoptosis. This equilibrium is controlled, at least in part by gap junctions (GJs)- GJs regulate the traffic of homeostasis mediators between cells, a process which is called the gap junctional intercellular communication (GJIC) and are composed of connexin (Cx) proteins. In liver, both Cx32 and Cx26 are expressed by hepatocytes, whereas Cx43 only becomes detectable upon dedifferentiation.

Considering (i) the involvement of GJIC in the control of homeostasis and (ii) the effects of TSA on this dynamic process, we raised the question whether TSA might affect the expression of Cx proteins in primary cultures of rat hepatocytes.

### Materials and methods

### Isolation of rat hepatocytes

Hepatocytes were isolated from adult male outbred Sprague-Dawley rats (Charles River Laboratories) as previously described (Papeleu et al. J Hepatol 39: 374-382, 2003) and cultivated in the absence (condition C0) or presence (condition C1) of 1 µM TSA. In another set of experiments, the isolated liver was perfused with 1 µM TSA, and drug treatment was continued during subsequent cultivation of the hepatocytes (condition C2). In all conditions, the hepatocytes were cultivated as a monolayer configuration, and samples were taken at days 1, 4 and 7 (D1, D4, D7) of the cultivation time.

### LDH-leakage

The time dependence of the cytotoxicity of TSA (1, 5, 25µM) was evaluated by means of measurement of lactate dehydrogenase leakage into the media. The assay was done with an LDH-kit (Merck 1 14869.0001; Ecoline 1S) according to the manufacturer's recommendations.

### Immunoblotting

Proteins (25 or 50µg) were resolved on SDS-PAGE (7.5, 10 or 12%) and blotted afterwards onto nitrocellulose membranes. After blocking with non-fatty milk, membranes were incubated with appropriate concentrations of primary and secondary antibodies (HRP labeled). Proteins were detected using the ECL detection system (Amersham Biosciences). Primary antibodies: Cx26 (Zymed, 1/250), Cx32 (Sigma, 1/500), Cx43 (Zymed, 1/100).

### Immunohistochemistry

Hepatocytes were fixed with cold ethanol, permeabilised with Triton X-100 (Sigma) and blocked with non-fatty milk. Following incubation with appropriate concentrations of primary and secondary antibodies (FITC or TRITC labeled), samples were mounted with DAPI-containing Vectashield (Vector Laboratories). Detection was performed by fluorescence microscopy (Leica DMR/XA).

### GJIC assay

Scrape loading/dye transfer assay was performed as described by el-Fouly et al., 1987: 422-430. In brief, hepatocytes were scraped in the presence of 0,05 % m/V Lucifer Yellow and incubated for 5 minutes at 37°C. The dye was then removed, cells were rinsed twice with PBS and fixed with 4 % m/V paraformaldehyde. Evaluation was performed by fluorescence microscopy. The distance travelled by Lucifer Yellow from the scrape was used as a parameter to evaluate GJIC functionality.

### Albumin ELISA

ALB concentrations secreted into the media were analysed by competitive enzym-linked immunosorbent assay (ELISA) as described (Koebe et al. Int J Artif Organs 17: 95-106, 1994). The ALB levels were normalized with respect to the media used.

### Results

### Effects of TSA on Cx protein expression and localization

(Figure 32) The expression of Cx26 and Cx32 increase, whereas the expression of C43 decreases in course of the cultivation time. 1 µM TSA induces the expressions of Cx32 and Cx43, but decreases the expression of Cx26. 1 µM TSA causes cytosolic localization of Cx43 (Figure 35) and perinuclear localization of Cx26 (Figure 33).
Early onset of TSA treatment (C2) results in more pronounced effects on the Cx protein expression (Figure 32-35).

### Effect of TSA on GJIC activity

(Figure 37) 1 µM TSA increases GJIC activity from D4 on. Early onset of TSA treatment (C2) results in a more pronounced effect on GJIC.

### Acetylation of histone H4

(Figure 36) Acetylation of histone H4 decreases in course of the cultivation time. 1 µM TSA causes hyperacetylation of histone H4. Early onset of TSA treatment (C2) results in a higher degree of H4 acetylation.

### Effect of TSA on albumin secretion

(Figure 38) 1 µM TSA improves albumin secretion from D4 (C2) or D7 (C1) on. Early onset of TSA treatment (C2) results in a more pronounced effect on the albumin secretion.

## Claims

1. A method for producing hepatocyte-like cells from adult stem cells *in vitro,* comprising the steps of:
(i) providing at least one adult stem cell;
(ii) exposing said at least one adult stem cell to a first differentiation agent or a combination of differentiation agents chosen from fibroblast growth factor 4 (FGF-4), hepatic growth factor (HGF), ITS (insulin, transferrin, selenious acid), dexamethasone, oncostatin M (OSM), bone morphogenetic protein 2 (BMP-2), BMP-4 and BMP-7;
(iii) subsequently exposing said at least one adult stem cell to one or more another differentiation agent or another combination of differentiation agents chosen from those defined in step (ii);
(iv) repeating step (iii), whereby said at least one adult stem cell is sequentially exposed to a series of differentiation agents or combinations of differentiation agents chosen from those defined in step (ii); and
(v) exposing said at least one adult stem cell to at least one inhibitor of histone deacetylases during and/or after exposing said at least one adult stem cell to the differentiation agents according to steps (ii) to (iv).

2. The method for producing hepatocyte-like cells from adult stem cells *in vitro* according to claim 1, wherein said at least one adult stem cell is a bone marrow stem cell.

3. The method for producing hepatocyte-like cells from adult stem cells *in vitro* according to claim 1, wherein said at least one adult stem cell is a mesenchymal stem cell.

4. The method for producing hepatocyte-like cells from adult stem cells *in vitro* according to claim 1, wherein said at least one adult stem cell is a multipotent adult progenitor cell.

5. The method for producing hepatocyte-like cells from adult stem cells *in vitro* according to any of claims 1 to 4, wherein said at least one adult stem cell is derived from a human.

6. The method for producing hepatocyte-like cells from adult stem cells *in vitro* according to any of claims 1 to 4, wherein said at least one adult stem cell is derived from an animal.

7. The method for producing hepatocyte-like cells from adult stem cells *in vitro* according to claim 6, wherein said animal is chosen from the group comprising mouse, rat, guinea pig, hamster, rabbit, chicken, cat, dog, a non-human primate, pig, sheep, cow, horse, fish, reptile, and amphibian.

8. The method for producing hepatocyte-like cells from adult stem cells *in vitro* according to any of claims 1 to 7, wherein the said first differentiation agent is, or the said first combination of differentiation agents comprises, FGF-4.

9. The method for producing hepatocyte-like cells from adult stem cells *in vitro* according to claim 8, wherein subsequently to FGF-4, the said cells are exposed to HGF, optionally in continuous presence of FGF-4.

10. The method according to any of claims 1 to 9, comprising exposing the adult stem cell to the following sequence of differentiation agents:
(i) FGF-4,
(ii) HGF,
(iii) HGF, ITS and dexamethasone.

11. The method according to any of claims 1 to 9, comprising exposing the adult stem cell to the following sequence of differentiation agents:
(i) FGF-4,
(ii) FGF-4 and HGF,
(iii) FGF-4, HGF and ITS,
(iv) FGF-4, HGF and dexamethasone,
(v) HGF and dexamethasone.

12. The method according to any of claims 1 to 9, comprising exposing the adult stem cell to the following sequence of differentiation agents:
(i) FGF-4,
(ii) FGF-4, HGF and ITS,
(iii) HGF, OSM and dexamethasone,
(iv) OSM and dexamethasone.

13. The method according to any of claims 1 to 9, comprising exposing the adult stem cell to the following sequence of differentiation agents:
(i) FGF-4, BMP-2, BMP-4 and BMP-7,
(ii) HGF and ITS,
(iii) OSM and dexamethasone.

14. The method according to any of claims 1 to 9, comprising exposing the adult stem cell to the following sequence of differentiation agents:
(i) FGF-4, BMP-2, BMP-4 and BMP-7,
(ii) HGF and ITS,
(iii) HGF, OSM and dexamethasone,
(iv) OSM and dexamethasone.

15. The method for producing hepatocyte-like cells from adult stem cells *in vitro* according to any of claims 1 to 14, wherein the first differentiation agent or combination of differentiation agents is added when the adult stem cells are 50-100% confluent, preferably 80-100% confluent and more preferably 90-100% confluent.

16. The method for producing hepatocyte-like cells from adult stem cells *in vitro* according to any of claims 1 to 15, wherein said at least one inhibitor of histone deacetylases is trichostatin A or a derivative thereof, or a semi-synthetic or a synthetic analogue of trichostatin A, or a derivative of such an analogue.

17. The method for producing hepatocyte-like cells from adult stem cells *in vitro* according to any of claims 1 to 15, wherein said at least one inhibitor of histone deacetylases is 5-(4-dimethylaminobenzoyl)-aminovaleric acid hydroxamid or a derivative thereof.

18. The method for producing hepatocyte-like cells from adult stem cells *in vitro* according to any of claims 1 to 17, wherein the cells are exposed to the said at least one inhibitor of histone deacetylases later than on 96h, and preferably on day 6 or later, after the start of the differentiation process.

19. The method for producing hepatocyte-like cells from adult stem cells *in vitro* according to any of claims 1 to 18, further comprising the use of the hepatocyte-like cells for the manufacture of a medicament for use in transplantation.

20. The method for producing hepatocyte-like cells from adult stem cells *in vitro* according to any of claims 1 to 18, further comprising the use of the hepatocyte-like cells for the manufacture of a medicament for use in gene therapy.

21. The method for producing hepatocyte-like cells from adult stem cells *in vitro* according to any of claims 1 to 18, further comprising the use of the hepatocyte-like cells in assays of toxicity.

22. The method for producing hepatocyte-like cells from adult stem cells *in vitro* according to any of claims 1 to 18, further comprising the use of the hepatocyte-like cells in assays of carcinogenicity.

23. The method for producing hepatocyte-like cells from adult stem celts *in vitro* according to any of claims 1 to 18, further comprising the use of the hepatocyte-like cells in assays of biotransformation.

24. A kit for use in the method for producing hepatocyte-like cells from adult stem cells *in vitro* according to claim 10, said kit comprising:
(i) optionally, at least one container comprising adult stem cells;
(ii) a container comprising FGF-4;
(iii) a container other than (ii) comprising HGF;
(iv) a container other than (ii) and (iii) comprising HGF, ITS and dexamethasone;
(v) at least one container comprising at least one histone deacetylase inhibitor; and
(vi) information for the user with at least one optimized manner of administering the components of said kit to the adult stem cells to produce the hepatocyte-like cell according to the method of claim 10.

25. A kit for use in the method for producing hepatocyte-like cells from adult stem cells *in vitro* according to claim 11, said kit comprising:
(i) optionally, at least one container comprising adult stem cells;
(ii) a container comprising FGF-4;
(iii) a container other than (ii) comprising FGF-4 and HGF;
(iv) a container other than (ii) and (iii) comprising FGF-4, HGF and ITS;
(v) a container other than (ii) to (iv) comprising FGF-4, HGF and dexamethasone;
(vi) a container other than (ii) to (v) comprising HGF and dexamethasone;
(vii) at least one container comprising at least one histone deacetylase inhibitor; and
(viii) information for the user with at least one optimized manner of administering the components of said kit to the adult stem cells to produce the hepatocyte-like cell according to the method of claim 11.

26. A kit for use in the method for producing hepatocyte-like cells from adult stem cells *in vitro* according to claim 12, said kit comprising:
(i) optionally, at least one container comprising adult stem cells;
(ii) a container comprising FGF-4;
(iii) a container other than (ii) comprising FGF-4, HGF and ITS;
(iv) a container other than (ii) and (iii) comprising HGF, OSM and dexamethasone;
(v) a container other than (ii) to (iv) comprising FGF-4, HGF and dexamethasone;
(vi) a container other than (ii) to (v) comprising OSM and dexamethasone;
(vii) at least one container comprising at least one histone deacetylase inhibitor; and
(viii) information for the user with at least one optimized manner of administering the components of said kit to the adult stem cells to produce the hepatocyte-like cell according to the method of claim 12.

27. A kit for use in the method for producing hepatocyte-like cells from adult stem cells *in vitro* according to claim 13, said kit comprising:
(i) optionally, at least one container comprising adult stem cells;
(ii) a container comprising FGF-4, BMP-2, BMP-4 and BMP-7;
(iii) a container other than (ii) comprising HGF and ITS;
(iv) a container other than (ii) and (iii) comprising OSM and dexamethasone;
(v) at least one container comprising at least one histone deacetylase inhibitor; and
(vi) information for the user with at least one optimized manner of administering the components of said kit to the adult stem cells to produce the hepatocyte-like cell according to the method of claim 13.

28. A kit for use in the method for producing hepatocyte-like cells from adult stem cells *in vitro* according to claim 14, said kit comprising:
(i) optionally, at least one container comprising adult stem cells;
(ii) a container comprising FGF-4, BMP-2, BMP-4 and BMP-7;
(iii) a container other than (ii) comprising HGF and ITS;
(iv) a container other than (ii) and (iii) comprising HGF, OSM and dexamethasone;
(v) a container other than (ii) to (iv) comprising OSM and dexamethasone;
(vi) at least one container comprising at least one histone deacetylase inhibitor; and
(vii) information for the user with at least one optimized manner of administering the components of said kit to the adult stem cells to produce the hepatocyte-like cell according to the method of claim 14.

## Patentansprüche

1. Verfahren zum Produzieren hepatozytenartiger Zellen aus adulten Stammzellen *in vitro,* welches die folgenden Schritte enthält:
(i) Vorsehen mindestens einer adulten Stammzelle;
(ii) Aussetzen der mindestens einen adulten Stammzelle einem ersten Differenzierungsmittel oder einer ersten Kombination von Differenzierungsmitteln, die aus Fibroblastenwachstumsfaktor 4 (Fibroblast Growth Factor 4, FGF-4), hepatischem Wachstumsfaktor (Hepatic Growth Factor, HGF), ITS (Insulin, Transferrin, Selensäure), Dexamethason, Oncostatin M (OSM), morphogenem Knochenprotein 2 (Bone Morphogenetic Protein 2, BMP-2), BMP-4 und BMP-7 ausgewählt sind;
(iii) anschließendes Aussetzen der mindestens einen adulten Stammzelle mindestens einem anderen Differenzierungsmittel oder einer anderen Kombination von Differenzierungsmitteln, die aus den in Schritt (ii) Definierten ausgewählt sind;
(iv) Wiederholen von Schritt (iii), wobei die mindestens eine adulte Stammzelle nacheinander einer Serie von Differenzierungsmitteln oder Kombinationen von Differenzierungsmitteln ausgesetzt wird, die aus den in Schritt (ii) Definierten ausgewählt sind; und
(v) Aussetzen der mindestens einen adulten Stammzelle mindestens einem Inhibitor von Histondeacetylasen während und/oder nach dem Aussetzen der mindestens einen adulten Stammzelle den Differenzierungsmitteln nach Schritt (ii) bis (iv).

2. Verfahren zum Produzieren hepatozytenartiger Zellen aus adulten Stammzellen *in vitro* nach Anspruch 1, wobei es sich bei der mindestens einen adulten Stammzelle um eine Knochenmarkstammzelle handelt.

3. Verfahren zum Produzieren hepatozytenartiger Zellen aus adulten Stammzellen *in vitro* nach Anspruch 1, wobei es sich bei der mindestens einen adulten Stammzelle um eine mesenchymale Stammzelle handelt.

4. Verfahren zum Produzieren hepatozytenartiger Zellen aus adulten Stammzellen *in vitro* nach Anspruch 1, wobei es sich bei der mindestens einen adulten Stammzelle um eine multipotente adulte Vorstufenzelle handelt.

5. Verfahren zum Produzieren hepatozytenartiger Zellen aus adulten Stammzellen *in vitro* nach einem der Ansprüche 1 bis 4, wobei die mindestens eine adulte Stammzelle von einem Menschen stammt.

6. Verfahren zum Produzieren hepatozytenartiger Zellen aus adulten Stammzellen *in vitro* nach einem der Ansprüche 1 bis 4, wobei die mindestens eine adulte Stammzelle von einem Tier stammt.

7. Verfahren zum Produzieren hepatozytenartiger Zellen aus adulten Stammzellen *in vitro* nach Anspruch 6, wobei das Tier aus der Gruppe ausgewählt ist, die Maus, Ratte, Meerschweinchen, Hamster, Kaninchen, Huhn, Katze, Hund, einen nicht-menschlichen Primaten, Schwein, Schaf, Kuh, Pferd, Fisch, Reptil und Amphib enthält.

8. Verfahren zum Produzieren hepatozytenartiger Zellen aus adulten Stammzellen *in vitro* nach einem der Ansprüche 1 bis 7, wobei es sich bei dem ersten Differenzierungsmittel um FGF-4 handelt oder wobei die erste Kombination von Differenzierungsmitteln FGF-4 enthält.

9. Verfahren zum Produzieren hepatozytenartiger Zellen aus adulten Stammzellen *in vitro* nach Anspruch 8, wobei die Zellen im Anschluss an FGF-4 HGF ausgesetzt werden, wahlweise unter anhaltendem Vorhandensein von FGF-4.

10. Verfahren nach einem der Ansprüche 1 bis 9, welches das Aussetzen der adulten Stammzelle der folgenden Abfolge von Differenzierungsmitteln enthält:
(i) FGF-4,
(ii) HGF,
(iii) HGF, ITS und Dexamethason.

11. Verfahren nach einem der Ansprüche 1 bis 9, welches das Aussetzen der adulten Stammzelle der folgenden Abfolge von Differenzierungsmitteln enthält:
(i) FGF-4,
(ii) FGF-4 und HGF,
(iii) FGF-4, HGF und ITS,
(iv) FGF-4, HGF und Dexamethason,
(v) HGF und Dexamethason.

12. Verfahren nach einem der Ansprüche 1 bis 9, welches das Aussetzen der adulten Stammzelle der folgenden Abfolge von Differenzierungsmitteln enthält:
(i) FGF-4,
(ii) FGF-4, HGF und ITS,
(iii) HGF, OSM und Dexamethason,
(iv) OSM und Dexamethason.

13. Verfahren nach einem der Ansprüche 1 bis 9, welches das Aussetzen der adulten Stammzelle der folgenden Abfolge von Differenzierungsmitteln enthält:
(i) FGF-4, BMP-2, BMP-4 und BMP-7,
(ii) HGF und ITS,
(iii) OSM und Dexamethason.

14. Verfahren nach einem der Ansprüche 1 bis 9, welches das Aussetzen der adulten Stammzelle der folgenden Abfolge von Differenzierungsmitteln enthält:
(i) FGF-4, BMP-2, BMP-4 und BMP-7,
(ii) HGF und ITS,
(iii) HGF, OSM und Dexamethason.
(iv) OSM und Dexamethason.

15. Verfahren zum Produzieren hepatozytenartiger Zellen aus adulten Stammzellen *in vitro* nach einem der Ansprüche 1 bis 14, wobei das erste Differenzierungsmittel bzw. die erste Kombination von Differenzierungsmitteln zugegeben wird, wenn die adulten Stammzellen 50-100 % konfluent, vorzugsweise 80-100 % konfluent und insbesondere 90-100 % konfluent sind.

16. Verfahren zum Produzieren hepatozytenartiger Zellen aus adulten Stammzellen *in vitro* nach einem der Ansprüche 1 bis 15, wobei es sich bei dem mindestens einen Inhibitor von Histondeacetylasen um Trichostatin A oder ein Derivat davon oder um ein halbsynthetisches oder ein synthetisches Analog von Trichostatin A oder um ein Derivat eines solchen Analogs handelt.

17. Verfahren zum Produzieren hepatozytenartiger Zellen aus adulten Stammzellen *in vitro* nach einem der Ansprüche 1 bis 15, wobei es sich bei dem mindestens einen Inhibitor von Histondeacetylasen um 5-(4-Dimethylaminobenzoyl)-aminovaleriansäurehydroxamid oder ein Derivat davon handelt.

18. Verfahren zum Produzieren hepatozytenartiger Zellen aus adulten Stammzellen *in vitro* nach einem der Ansprüche 1 bis 17, wobei die Zellen dem mindestens einen Inhibitor von Histondeacetylasen später als 96 Std. und vorzugsweise an Tag 6 oder später nach Beginn des Differenzierungsprozesses ausgesetzt werden.

19. Verfahren zum Produzieren hepatozytenartiger Zellen aus adulten Stammzellen *in vitro* nach einem der Ansprüche 1 bis 18, welches des Weiteren die Verwendung der hepatozytenartigen Zellen für die Herstellung eines Medikaments zur Verwendung bei einer Transplantation enthält.

20. Verfahren zum Produzieren hepatozytenartiger Zellen aus adulten Stammzellen *in vitro* nach einem der Ansprüche 1 bis 18, welches des Weiteren die Verwendung der hepatozytenartigen Zellen für die Herstellung eines Medikaments zur Verwendung bei einer Gentherapie enthält.

21. Verfahren zum Produzieren hepatozytenartiger Zellen aus adulten Stammzellen *in vitro* nach einem der Ansprüche 1 bis 18, welches des Weiteren die Verwendung der hepatozytenartigen Zellen in Toxizitätsassays enthält.

22. Verfahren zum Produzieren hepatozytenartiger Zellen aus adulten Stammzellen *in vitro* nach einem der Ansprüche 1 bis 18, welches des Weiteren die Verwendung der hepatozytenartigen Zellen in Karzinogenitätsassays enthält.

23. Verfahren zum Produzieren hepatozytenartiger Zellen aus adulten Stammzellen *in vitro* nach einem der Ansprüche 1 bis 18, welches des Weiteren die Verwendung der hepatozytenartigen Zellen in Biotransformationsassays enthält.

24. Kit für die Verwendung in dem Verfahren zum Produzieren hepatozytenartiger Zellen aus adulten Stammzellen *in vitro* nach Anspruch 10, wobei das Kit Folgendes enthält:
(i) wahlweise mindestens einen Behälter, der adulte Stammzellen enthält;
(ii) einen Behälter, der FGF-4 enthält;
(iii) einen anderen Behälter als (ii), der HGF enthält;
(iv) einen anderen Behälter als (ii) und (iii), der HGF, ITS und Dexamethason enthält;
(v) mindestens einen Behälter, der mindestens einen Histondeacetylaseinhibitor enthält; und
(vi) Informationen für den Anwender mit mindestens einer optimierten Art und Weise des Verabreichens der Bestandteile des Kits an die adulten Stammzellen, um die hepatozytenartigen Zellen nach dem Verfahren von Anspruch 10 zu produzieren.

25. Kit für die Verwendung in dem Verfahren zum Produzieren hepatozytenartiger Zellen aus adulten Stammzellen *in vitro* nach Anspruch 11, wobei das Kit Folgendes enthält:
(i) wahlweise mindestens einen Behälter, der adulte Stammzellen enthält;
(ii) einen Behälter, der FGF-4 enthält;
(iii) einen anderen Behälter als (ii), der FGF-4 und HGF enthält;
(iv) einen anderen Behälter als (ii) und (iii), der FGF-4, HGF und ITS enthält;
(v) einen anderen Behälter als (ii) bis (iv), der FGF-4, HGF und Dexamethason enthält;
(vi) einen anderen Behälter als (ii) bis (v), der HGF und Dexamethason enthält;
(vii) mindestens einen Behälter, der mindestens einen Histondeacetylaseinhibitor enthält; und
(viii) Informationen für den Anwender mit mindestens einer optimierten Art und Weise des Verabreichens der Bestandteile des Kits an die adulten Stammzellen, um die hepatozytenartigen Zellen nach dem Verfahren von Anspruch 11 zu produzieren.

26. Kit für die Verwendung in dem Verfahren zum Produzieren hepatozytenartiger Zellen aus adulten Stammzellen *in vitro* nach Anspruch 12, wobei das Kit Folgendes enthält:
(i) wahlweise mindestens einen Behälter, der adulte Stammzellen enthält;
(ii) einen Behälter, der FGF-4 enthält;
(iii) einen anderen Behälter als (ii), der FGF-4, HGF und ITS enthält;
(iv) einen anderen Behälter als (ii) und (iii), der HGF, OSM und Dexamethason enthält;
(v) einen anderen Behälter als (ii) bis (iv), der FGF-4, HGF und Dexamethason enthält;
(vi) einen anderen Behälter als (ii) bis (v), der OSM und Dexamethason enthält;
(vii) mindestens einen Behälter, der mindestens einen Histondeacetylaseinhibitor enthält; und
(viii) Informationen für den Anwender mit mindestens einer optimierten Art und Weise des Verabreichens der Bestandteile des Kits an die adulten Stammzellen, um die hepatozytenartigen Zellen nach dem Verfahren von Anspruch 12 zu produzieren.

27. Kit für die Verwendung in dem Verfahren zum Produzieren hepatozytenartiger Zellen aus adulten Stammzellen *in vitro* nach Anspruch 13, wobei das Kit Folgendes enthält:
(i) wahlweise mindestens einen Behälter, der adulte Stammzellen enthält;
(ii) einen Behälter, der FGF-4, BMP-2, BMP-4 und BMP-7 enthält;
(iii) einen anderen Behälter als (ii), der HGF und ITS enthält;
(iv) einen anderen Behälter als (ii) und (iii), der OSM und Dexamethason enthält;
(v) mindestens einen Behälter, der mindestens einen Histondeacetylaseinhibitor enthält; und
(vi) Informationen für den Anwender mit mindestens einer optimierten Art und Weise des Verabreichens der Bestandteile des Kits an die adulten Stammzellen, um die hepatozytenartigen Zellen nach dem Verfahren von Anspruch 13 zu produzieren.

28. Kit für die Verwendung in dem Verfahren zum Produzieren hepatozytenartiger Zellen aus adulten Stammzellen *in vitro* nach Anspruch 14, wobei das Kit Folgendes enthält:
(i) wahlweise mindestens einen Behälter, der adulte Stammzellen enthält;
(ii) einen Behälter, der FGF-4, BMP-2, BMP-4 und BMP-7 enthält;
(iii) einen anderen Behälter als (ii), der HGF und ITS enthält;
(iv) einen anderen Behälter als (ii) und (iii), der HGF, OSM und Dexamethason enthält;
(v) einen anderen Behälter als (ii) bis (iv), der OSM und Dexamethason enthält;
(vi) mindestens einen Behälter, der mindestens einen Histondeacetylaseinhibitor enthält; und
(vii) Informationen für den Anwender mit mindestens einer optimierten Art und Weise des Verabreichens der Bestandteile des Kits an die adulten Stammzellen, um die hepatozytenartigen Zellen nach dem Verfahren von Anspruch 14 zu produzieren.

## Revendications

1. Procédé de production de cellules de type hépatocyte à partir de cellules souches adultes *in vitro,* qui comprend les étapes consistant à :
(i) fournir au moins une cellule souche adulte ;
(ii) exposer ladite ou lesdites cellules souches adultes à un premier agent de différenciation ou à une combinaison d'agents de différenciation choisis parmi le facteur de croissance des fibroblastes 4 (FGF-4), le facteur de croissance des hépatocytes (HGF), l'ITS (insuline, transferrine, acide sélénieux), la dexaméthasone, l'oncostatine M (OSM), la protéine morphogénétique osseuse 2 (BMP-2), la BMP-4 et la BMP-7 ;
(iii) exposer ensuite ladite ou lesdites cellules souches adultes à un ou plusieurs autres agents de différenciation ou autres combinaisons d'agents de différenciation choisis parmi ceux définis à l'étape (ii) ;
(iv) répéter l'étape (iii), ladite ou lesdites cellules souches adultes se trouvant ainsi séquentiellement exposées à une série d'agents de différenciation ou de combinaisons d'agents de différenciation choisis parmi ceux définis à l'étape (ii) ; et
(v) exposer ladite ou lesdites cellules souches adultes à au moins un inhibiteur des histones désacétylases pendant et/ou après l'exposition de ladite ou desdites cellules souches adultes aux agents de différenciation selon les étapes (ii) à (iv).

2. Procédé de production de cellules de type hépatocyte à partir de cellules souches adultes *in vitro* selon la revendication 1, dans lequel ladite ou lesdites cellules souches adultes sont une cellule souche de moelle osseuse.

3. Procédé de production de cellules de type hépatocyte à partir de cellules souches adultes *in vitro* selon la revendication 1, dans lequel ladite ou lesdites cellules souches adultes sont une cellule souche mésenchymateuse.

4. Procédé de production de cellules de type hépatocyte à partir de cellules souches adultes *in vitro* selon la revendication 1, dans lequel ladite ou lesdites cellules souches adultes sont une cellule progénitrice adulte multipotente.

5. Procédé de production de cellules de type hépatocyte à partir de cellules souches adultes *in vitro* selon l'une quelconque des revendications 1 à 4, dans lequel ladite ou lesdites cellules souches adultes sont dérivées d'un être humain.

6. Procédé de production de cellules de type hépatocyte à partir de cellules souches adultes *in vitro* selon l'une quelconque des revendications 1 à 4, dans lequel ladite ou lesdites cellules souches adultes sont dérivées d'un animal.

7. Procédé de production de cellules de type hépatocyte à partir de cellules souches adultes *in vitro* selon la revendication 6, dans lequel ledit animal est choisi dans le groupe comprenant les souris, les rats, les cobayes, les hamsters, les lapins, les poulets, les chats, les chiens, les primates non humains, les porcs, les moutons, les vaches, les chevaux, les poissons, les reptiles, et les amphibies.

8. Procédé de production de cellules de type hépatocyte à partir de cellules souches adultes *in vitro* selon l'une quelconque des revendications 1 à 7, dans lequel ledit premier agent de différenciation est, ou ladite première combinaison d'agents de différenciation comprend, le FGF-4.

9. Procédé de production de cellules de type hépatocyte à partir de cellules souches adultes *in vitro* selon la revendication 8, dans lequel après le FGF-4, lesdites cellules sont exposées au HGF, du FGF-4 étant facultativement toujours présent.

10. Procédé selon l'une quelconque des revendications 1 à 9, comprenant l'exposition de la cellule souche adulte à la séquence suivante d'agents de différenciation :
(i) FGF-4,
(ii) HGF,
(iii) HGF, ITS et dexaméthasone.

11. Procédé selon l'une quelconque des revendications 1 à 9, comprenant l'exposition de la cellule souche adulte à la séquence suivante d'agents de différenciation :
(i) FGF-4,
(ii) FGF-4 et HGF,
(iii) FGF-4, HGF et ITS,
(iv) FGF-4, HGF et dexaméthasone,
(v) HGF et dexaméthasone.

12. Procédé selon l'une quelconque des revendications 1 à 9, comprenant l'exposition de la cellule souche adulte à la séquence suivante d'agents de différenciation :
(i) FGF-4,
(ii) FGF-4, HGF et ITS,
(iii) HGF, OSM et dexaméthasone,
(iv) OSM et dexaméthasone.

13. Procédé selon l'une quelconque des revendications 1 à 9, comprenant l'exposition de la cellule souche adulte à la séquence suivante d'agents de différenciation :
(i) FGF-4, BMP-2, BMP-4 et BMP-7,
(ii) HGF et ITS,
(iii) OSM et dexaméthasone.

14. Procédé selon l'une quelconque des revendications 1 à 9, comprenant l'exposition de la cellule souche adulte à la séquence suivante d'agents de différenciation :
(i) FGF-4, BMP-2, BMP-4 et BMP-7,
(ii) HGF et ITS,
(iii) HGF, OSM et dexaméthasone,
(iv) OSM et dexaméthasone.

15. Procédé de production de cellules de type hépatocyte à partir de cellules souches adultes *in vitro* selon l'une quelconque des revendications 1 à 14, dans lequel le premier agent de différenciation ou la première combinaison d'agents de différenciation est ajouté(e) lorsque les cellules souches adultes sont à une confluence de 50 à 100 %, de préférence à une confluence de 80 à 100 % et de manière davantage préférée à une confluence de 90 à 100%.

16. Procédé de production de cellules de type hépatocyte à partir de cellules souches adultes *in vitro* selon l'une quelconque des revendications 1 à 15, dans lequel ledit ou lesdits inhibiteurs des histone désacétylases sont la trichostatine A ou un dérivé de celle-ci, ou un analogue semi-synthétique ou synthétique de la trichostatine A, ou un dérivé d'un tel analogue.

17. Procédé de production de cellules de type hépatocyte à partir de cellules souches adultes *in vitro* selon l'une quelconque des revendications 1 à 15, dans lequel ledit ou lesdits inhibiteurs des histones désacétylases sont l'hydroxamide de l'acide 5-(4-diméthylaminobenzoyl)-aminovalérique ou un dérivé de celui-ci.

18. Procédé de production de cellules de type hépatocyte à partir de cellules souches adultes *in vitro* selon l'une quelconque des revendications 1 à 17, dans lequel les cellules sont exposées au dit ou auxdits inhibiteurs des histones désacétylases après 96 h, et de préférence le jour 6 ou ultérieurement, après le début du processus de différenciation.

19. Procédé de production de cellules de type hépatocyte à partir de cellules souches adultes *in vitro* selon l'une quelconque des revendications 1 à 18, comprenant en outre l'utilisation de cellules de type hépatocyte pour la fabrication d'un médicament destiné à être utilisé dans la transplantation.

20. Procédé de production de cellules de type hépatocyte à partir de cellules souches adultes *in vitro* selon l'une quelconque des revendications 1 à 18, comprenant en outre l'utilisation de cellules de type hépatocyte pour la fabrication d'un médicament destiné à être utilisé en thérapie génique.

21. Procédé de production de cellules de type hépatocyte à partir de cellules souches adultes *in vitro* selon l'une quelconque des revendications 1 à 18, comprenant en outre l'utilisation de cellules de type hépatocyte dans des dosages de toxicité.

22. Procédé de production de cellules de type hépatocyte à partir de cellules souches adultes *in vitro* selon l'une quelconque des revendications 1 à 18, comprenant en outre l'utilisation de cellules de type hépatocyte dans des dosages de cancérogénicité.

23. Procédé de production de cellules de type hépatocyte à partir de cellules souches adultes *in vitro* selon l'une quelconque des revendications 1 à 18, comprenant en outre l'utilisation de cellules de type hépatocyte dans des dosages de biotransformation.

24. Kit destiné à être utilisé dans le procédé de production de cellules de type hépatocyte à partir de cellules souches adultes *in vitro* selon la revendication 10, ledit kit comprenant :
(i) facultativement, au moins un récipient comprenant des cellules souches adultes ;
(ii) un récipient contenant le FGF-4 ;
(iii) un récipient autre que le récipient (ii) comprenant le HGF ;
(iv) un récipient autre que les récipients (ii) et (iii) comprenant le HGF, l'ITS et la dexaméthasone ;
(v) au moins un récipient comprenant au moins inhibiteur des histones désacétylases ; et
(iv) des informations destinées à l'utilisateur avec au moins une manière optimisée d'administrer les composants dudit kit aux cellules souches adultes pour produire la cellule de type hépatocyte selon le procédé selon la revendication 10.

25. Kit destiné à être utilisé dans le procédé de production de cellules de type hépatocyte à partir de cellules souches adultes *in vitro* selon la revendication 11, ledit kit comprenant :
(i) facultativement, au moins un récipient comprenant des cellules souches adultes ;
(ii) un récipient contenant le FGF-4 ;
(iii) un récipient autre que le récipient (ii) comprenant le FGF-4 et le HGF ;
(iv) un récipient autre que les récipients (ii) et (iii) comprenant le FGF, le HGF et l'ITS ;
(v) un récipient autre que les récipients (ii) à (iv) comprenant le FGF-4, le HGF et la dexaméthasone ;
(vi) un récipient autre que les récipients (ii) à (v) comprenant le HGF et la dexaméthasone ;
(vii) au moins un récipient comprenant au moins inhibiteur des histones désacétylases ; et
(viii)des informations destinées à l'utilisateur avec au moins une manière optimisée d'administrer les composants dudit kit aux cellules souches adultes pour produire la cellule de type hépatocyte selon le procédé selon la revendication 11.

26. Kit destiné à être utilisé dans le procédé de production de cellules de type hépatocyte à partir de cellules souches adultes *in vitro* selon la revendication 12, ledit kit comprenant :
(i) facultativement, au moins un récipient comprenant des cellules souches adultes ;
(ii) un récipient contenant le FGF-4 ;
(iii) un récipient autre que le récipient (ii) comprenant le FGF-4 ; le HGF et l'ITS ;
(iv) un récipient autre que les récipients (ii) et (iii) comprenant le HGF, l'OSM et la dexaméthasone ;
(v) un récipient autre que les récipients (ii) à (iv) comprenant le FGF-4, le HGF et la dexaméthasone ;
(vi) un récipient autre que les récipients (ii) à (v) comprenant l'OSM et la dexaméthasone ;
(vii) au moins un récipient comprenant au moins inhibiteur des histones désacétylases ; et
(viii)des informations destinées à l'utilisateur avec au moins une manière optimisée d'administrer les composants dudit kit aux cellules souches adultes pour produire la cellule de type hépatocyte selon le procédé selon la revendication 12.

27. Kit destiné à être utilisé dans le procédé de production de cellules de type hépatocyte à partir de cellules souches adultes *in vitro* selon la revendication 13, ledit kit comprenant :
(i) facultativement, au moins un récipient comprenant des cellules souches adultes ;
(ii) un récipient contenant le FGF-4, la BMP-2, la BMP-4 et la BMP-7 ;
(iii) un récipient autre que le récipient (ii) comprenant le HGF et l'ITS ;
(iv) un récipient autre que les récipients (ii) et (iii) comprenant l'OSM et la dexaméthasone ;
(v) au moins un récipient comprenant au moins inhibiteur des histones désacétylases ; et
(vi) des informations destinées à l'utilisateur avec au moins une manière optimisée d'administrer les composants dudit kit aux cellules souches adultes pour produire la cellule de type hépatocyte selon le procédé selon la revendication 13.

28. Kit destiné à être utilisé dans le procédé de production de cellules de type hépatocyte à partir de cellules souches adultes *in vitro* selon la revendication 14, ledit kit comprenant :
(i) facultativement, au moins un récipient comprenant des cellules souches adultes ;
(ii) un récipient contenant le FGF-4, la BMP-2, la BMP-4 et la BMP-7 ;
(iii) un récipient autre que le récipient (ii) comprenant le HGF et l'ITS ;
(iv) un récipient autre que les récipients (ii) et (iii) comprenant le HGF, l'OSM et la dexaméthasone ;
(v) un récipient autre que les récipients (ii) à (iv) comprenant l'OSM et la dexaméthasone ;
(vi) au moins un récipient comprenant au moins inhibiteur des histones désacétylases ; et
(vii) des informations destinées à l'utilisateur avec au moins une manière optimisée d'administrer les composants dudit kit aux cellules souches adultes pour produire la cellule de type hépatocyte selon le procédé selon la revendication 14.
